# EUROPEAN PATENT SPECIFICATION

(11) **EP 4 069 065 B1**
(45) Date of publication and mention of the grant of the patent: **07.01.2026**
(21) Application number: 20896161.5
(22) Date of filing: 04.12.2020
(51) Int. Cl.: A61B 5/00, A41D 20/00, A61B 5/01, A61B 5/11, A61B 5/291, A61F 5/56, A61H 23/00, A61B 5/251, A61H 23/02, A61H 9/00, A61M 16/06, A61M 21/00, A61M 16/00

(54) **WEARABLE DEVICE**
TRAGBARE VORRICHTUNG
DISPOSITIF PORTABLE

(30) Priority: 05.12.2019 US 201962943845 P
(43) Date of publication of application: 12.10.2022
(60) Divisional application: 25214427.4
(73) Proprietor: InteraXon Inc., Toronto, Ontario M5V 3B1 (CA)
(72) Inventor: FLEURY, Amanda, Toronto, Ontario M4V 2A2 (CA); AIMONE, Christopher Allen, Toronto, Ontario M6K 2M2 (CA); ZACHAROWSKA, Marta Joanna, Toronto, Ontario M6S 3A9 (CA); MACKENZIE, Samuel Thomas, Toronto, Ontario M6R 1Y3 (CA); MATUTE, Nathaly Arraiz, Toronto, Ontario M5V 3B1 (CA); KERR, Sam, Toronto, Ontario M5P 2Y3 (CA); GREENLEAF, Walter John, Portola Valley, California 94028 (US)
(74) Representative: Appleyard Lees IP LLP
(86) International application number: PCT/CA2020/051672
(87) International publication number: WO 2021/108922

(56) References cited:
- US-A1- 2015 057 511
- US-A1- 2019 200 925
- US-A1- 2019 200 925
- US-B1- 6 445 942

## Description

### CROSS-REFERENCE TO RELATED APPLICATION(S)

This application claims priority from U.S. Provisional Patent Application No. 62/943845 filed on December 5, 2019**.**

### FIELD

The present disclosure relates to wearable devices. More specifically, the present disclosure relates to wearable devices with brainwave sensing components and that can be worn on the head of a user.

### BACKGROUND

A user may interact with a computing device for example using a keyboard, mouse, track pad, touch screen, or motion-capture devices. As the ways in which humans interact with computing devices change, computers may become usable for new purposes, or more efficient in performing existing tasks. A user command to a computing device that may require several commands on a keyboard may be instead associated with a thought or gesture captured and processed by a sensory input device. As the human body has many parts which may be controlled through voluntary movement, there are opportunities for capturing and interpreting other movements for interacting with a computing device.

Bio-signals are signals that are generated by biological beings that can be measured and monitored. Electroencephalographs, galvanometers, and electrocardiographs are examples of devices that are used to measure and monitor bio-signals generated by humans.

A human brain generates bio-signals such as electrical patterns, which may be measured/monitored using an electroencephalogram ("EEG"). These electrical patterns, or brainwaves, are measurable by devices such as an EEG. Typically, an EEG will measure brainwaves in an analog form. Then, these brainwaves may be analyzed either in their original analog form or in a digital form after an analog to digital conversion.

Measuring and analyzing bio-signals such as brainwave patterns can have a variety of practical applications. For example, brain computer interfaces ("BCI") have been developed that allow users to control devices and computers using brainwave signals. In another example, analysis of brainwave patterns during sleep may allow users to understand their sleep patterns and/or improve their quality of sleep.

US 2019/0200925 A1 shows a wearable device as depicted in figure **31****.**

### SUMMARY

The object of the present invention is achieved with a device according to claim 1. Preferred embodiments are described in the dependent claims.

According to an aspect, there is provided a wearable device comprising: a flexible and extendable body configured to encircle a portion of a user; an electronics module having a surface defining a concave space between a first end and a second end opposite the first end, the first end attachable to the flexible and extendable body at a first connection point with a first flexible retention mount to allow rotation of the flexible and extendable body relative to the electronics module about a first axis and to transfer tension force from the flexible and extendable body to the electronics module radially from the first axis, the second end attachable to the flexible and extendable body at a second connection point with a second flexible retention mount to allow rotation of the flexible and extendable body relative to the electronics about a second axis and to transfer tension force from the flexible and extendable body to the electronics module radially from the second axis; and a bio-signal sensor disposed on the flexible and extendable body between the first connection point and the second connection point to contact at least part of the portion of the user and to receive bio-signals from the user, upon the flexible and extendable body extending to be worn by the user with the electronics module attached: a tension force is applied from the flexible and extendable body to the electronics module through the first flexible retention mount and the second flexible retention mount to draw the electronics module towards the user, a portion of the flexible and extendable body between the first connection point and the second connection point rotates towards the concave space, and the electronics module urges the bio-signal sensor on the flexible and extendable body against the portion of the user.

**In** some embodiments, the bio-signal sensor is configured to contact at least part of a frontal region of a head of the user.

**In** some embodiments, the bio-signal sensor is an electroencephalography (EEG) sensor.

**In** some embodiments, the bio-signal sensor is an electrode to measure electrical potential and generate electric potential.

**In** some embodiments, the wearable device further comprises an additional bio-signal sensor to contact at least part of an auricular region of a head of the user.

In some embodiments, the additional bio-signal sensor is an electroencephalography (EEG) sensor.

In some embodiments, the wearable device further comprises an electrical connection between the electronics module and the flexible and extendable body.

In some embodiments, the wearable device further comprises an electrical connection between the electronics module and the bio-signal sensor.

In some embodiments, the electronics module is curved to generally correspond to a head of the user.

In some embodiments, the electronics module is attachable to the flexible and extendable body by a magnetic force.

In some embodiments, the electronics module includes a first magnet at the first end to attach to the first flexible retention mount by magnetic force and a second magnet at the second end to attach to the second flexible retention mount by magnetic force.

In some embodiments, the wearable device further comprises an additional bio-signal sensor disposed on the electronics module.

In some embodiments, the additional bio-signal sensor is an optical sensor.

In some embodiments, the optical sensor is mounted on a flexible protrusion to the electronics module, the flexible protrusion to compress as the electronics module is drawn towards the body of the user.

In some embodiments, the optical sensor is to detect a compression of the body based at least in part on a detected reflection distance of light reflected into the optical sensor.

In some embodiments, the optical sensor detects additional bio-signals based at least in part on a detected reflection distance of light reflected into the optical sensor.

In some embodiments, the optical sensor detects additional bio-signals based at least in part on a measured colour and intensity of light reflected into the optical sensor.

In some embodiments, the flexible and extendable body comprises a compressible section adjacent to the bio-signal sensor to compress to conform the at least one bio-signal sensor to the body of the user.

In some embodiments, the compressible section is shaped to conform to the at least part of the body of the user.

In some embodiments, the compressible section comprises foam having variable density.

In some embodiments, the wearable device further comprises a light emitter.

In some embodiments, the wearable device further comprises a light receiver.

In some embodiments, the light receiver is disposed on the flexible and extendable body adjacent an eye of the user to detect light adjacent the eye of the user.

In some embodiments, the wearable device further comprises a vibrational transducer.

In some embodiments, the vibrational transducer is a speaker.

In some embodiments, the vibrational transducer generates physical vibrations.

In some embodiments, the vibrational transducer is a microphone.

In some embodiments, the vibrational transducer is disposed on the flexible and extendable body to be adjacent an ear of the user.

In some embodiments, the vibrational transducer is disposed on the flexible and extendable body to be adjacent a front of a head of the user.

In some embodiments, the vibrational transducer is disposed on the flexible and extendable body to be adjacent a bone of the user.

In some embodiments, the wearable device further comprises multiple vibrational transducers for beamforming.

In some embodiments, the multiple vibrational transducers are an array of microphones to localize sound from a direction.

In some embodiments, the wearable device further comprises an accelerometer to detect movement of the user.

In some embodiments, the wearable device further comprises a thermistor to detect a temperature.

In some embodiments, the thermistor is configured to detect a relative temperature change.

In some embodiments, the wearable device further comprises a communicator to transmit data to a computing device.

In some embodiments, the communicator communicates with the computing device on a Bluetooth communications protocol.

In some embodiments, the communicator communicates with the computing device on a Wi-Fi communications protocol.

According to another aspect, there is provided a wearable device comprising: a body that is flexible and extendable to encircle a portion of a user; an electronics module having a surface defining a concave space; the electronics module attachable to the flexible and extendable body at a first connection point by a first flexible retention mount for rotation about a first axis and at a second connection point by a second flexible retention mount for rotation about a second axis to generate a force radially from the first axis and the second axis to draw the electronics module towards the portion of the user; a bio-signal sensor disposed on the flexible and extendable body between the first connection point and the second connection point to contact at least part of the portion of the user to receive bio-signals from the user, wherein upon the flexible and extendable body extending to be worn by the user with the electronics module attached, a portion of the flexible and extendable body between the first connection point and the second connection point, for example, the portion with the bio-signal sensor disposed, rotates towards the concave space and the force draws the electronics module to urge the bio-signal sensor on the flexible and extendable body against the portion of the user.

Other features will become apparent from the drawings in conjunction with the following description.

In this respect, before explaining any embodiments described herein in detail, it is to be understood that the disclosure is not limited in its application to the details of construction and to the arrangements of the components set forth in the following description or illustrated in the drawings. The disclosure is capable of other embodiments and of being practiced and carried out in various ways. Also, it is to be understood that the phraseology and terminology employed herein are for the purpose of description and should not be regarded as limiting.

### BRIEF DESCRIPTION OF DRAWINGS

Embodiments will now be described, by way of example only, with reference to the attached figures.
FIG. 1A illustrates a perspective view of an embodiment of a wearable device.
FIG. 1B illustrates a side view of the embodiment of the wearable device of FIG. 1A when worn by a user.
FIG. 1C - 1L illustrates schematics of example stack ups to form flexible electrodes, in embodiments of a wearable device.
FIG. 1M - 1V illustrates various embodiments of attachment mechanisms for a wearable device.
FIG. 2A illustrates a perspective view of a wearable device, according to an embodiment.
FIG. 2B illustrates a top view of the wearable device of FIG. 2A.
FIG. 2C illustrates a front view of the wearable device of FIG. 2A.
FIG. 2D illustrates a bottom view of the wearable device of FIG. 2A.
FIG. 2E illustrates a rear view of the wearable device of FIG. 2A.
FIG. 2F illustrates a bottom perspective view of the wearable device of FIG. 2A.
FIG. 2G illustrates a top perspective view of the wearable device of FIG. 2A.
FIG. 2H illustrates a rear-bottom perspective view of the wearable device of FIG. 2A.
FIG. 2I is a perspective view of an adjuster and a clasp of the wearable device of FIG. 2A.
FIG. 2J illustrates a perspective view of the wearable device of FIG. 2A when worn by a user.
FIG. 2K - 2N illustrate various embodiments of a bowstring design between an electronics module and a body of a wearable device.
FIG. 3 illustrates a side view of an embodiment of a wearable device having a crown strap when worn by a user.
FIG. 4 illustrates a side view of an embodiment of a wearable device having a crown strap and a top strap when worn by a user.
FIG. 5 illustrates a side view of an embodiment of a wearable device having a top strap when worn by a user.
FIG. 6 illustrates a perspective view of an embodiment of a wearable device having a top strap and a hair-penetrating sensor.
FIG. 7 illustrates a perspective view of an embodiment of a wearable device having a top strap, a crown strap, and a hair-penetrating sensor.
FIG. 8 is a top schematic view of a bio-signal sensor integrated into a fabric substrate according to an embodiment.
FIG. 9 is a cross-section schematic view of the bio-signal sensor integrated into the fabric substrate of FIG. 8 along lines I-I.
FIG. 10A is a rear view of an outer layer and an inner layer of a wearable device having a flexible printed circuit board, according to an embodiment.
FIG. 10B is a perspective view of the outer layer of FIG. 10A.
FIG. 10C is a perspective view of the inner layer and the flexible printed circuit board of FIG. 10A.
FIG. 10D is a side view of a flexible printed circuit board configuration, according to an embodiment.
FIG. 11A is a fabric pattern having bio-signal sensors for forming a wearable device, according to an embodiment.
FIGS. 11B to 11D illustrate embodiments of contact configurations of bio-signal sensors.
FIG. 11E illustrates bio-signal sensor contacts and leads, as connected to a flexible printed circuit board in a wearable device, according to an embodiment.
FIGS. 12A and 12B are schematic views of multiple bio-signal sensors disposed in a body of a wearable device, according to an embodiment.
FIG. 13A illustrates a schematic side view of an embodiment of a wearable device having an ear electrode with an open 'bow string' design, and FIG. 13B is an expanded view thereof, according to an embodiment.
FIG. 13C illustrates an expanded schematic side view of an embodiment of a wearable device having an ear electrode a closed 'bow string' design, according to an embodiment.
FIG. 13D illustrates various configurations of a wearable device having an ear electrode, according to various embodiments.
FIGS. 13E-13F illustrate various configurations of bio-signal sensors on a body of a wearable device, according to various embodiments.
FIG. 14 illustrates a side schematic view of an embodiment of wearable device 100 having an ear electrode shaped to contact the upper and rear surface of an ear of a user.
FIG. 15A illustrates a side schematic view of an embodiment of wearable device having a movable ear electrode, and FIG. 15B is an expanded view thereof, according to an embodiment.
FIG. 16 is a schematic view of an inner earpiece conductive sensor, according to an embodiment.
FIG. 17 is a schematic view of a sound delivery module, according to an embodiment.
FIG. 18 is a schematic view of the sound delivery module of FIG. 17 connected to an electronics module of a wearable device, according to an embodiment.
FIGS. 19A is a schematic view of an inner earpiece with a conductive sensor backing frame, according to an embodiment.
FIGS. 19B is a schematic view of an inner earpiece with a conductive sensor backing frame, according to another embodiment.
FIG. 20 illustrates a partial cross-sectional view of a through-hair bio-signal sensor in an uncompressed state, according to an embodiment.
FIG. 21 illustrates a partial cross-sectional view of the bio-signal sensor of FIG. 20 in a compressed state.
FIG. 22 illustrates a partial cross-sectional view of a bio-signal sensor, according to an embodiment.
FIG. 23 illustrates a perspective view of the bio-signal sensor of FIG. 22.
FIG. 24 illustrates a schematic view of placement of bio-signal sensors on a user, according to an embodiment.
FIG. 25 illustrates a schematic view of placement of bio-signal sensors on a user, according to an embodiment.
FIG. 26 illustrates a perspective view of a bio-signal sensor, according to an embodiment.
FIG. 27 illustrates a top view of the bio-signal sensor of FIG. 26.
FIG. 28 illustrates a non-contact electrode, according to an embodiment.
FIG. 29A illustrates a side view of a user wearing a wearable device having a capacitive electrode, according to an embodiment.
FIG. 29B illustrates a partial top view of the wearable device of FIG. 29A.
FIG. 30A is a front-top perspective view of an electronics module which is releasable from a wearable device, according to an embodiment of the invention.
FIG. 30B is another perspective view of an electronics module releasable from a wearable device, according to an embodiment of the invention.
FIG. 30C is an enlarged perspective view of retention mounts for an electronics module of a wearable device, according to an embodiment of the invention.
FIG. 30D is an exploded view of retention mounts for an electronics module of a wearable device, according to an embodiment of the invention.
FIG. 30E is a side view of a retention mount for an electronics module, according to an embodiment of the invention.
FIG. 30F is another front perspective view of retention mounts for an electronics module of a wearable device, according to an embodiment of the invention.
FIG. 30G is an exploded view of an electronics module, according to an embodiment of the invention.
FIG. 30H is a schematic view of a printed circuit board and components of an electronics module, according to an embodiment.
FIG. 30I illustrates an example embodiment of an electronics module with a flexible protrusion for an optical sensor.
FIG. 31A is a top schematic cross-sectional view of an electronics module connected to wearable device, according to an embodiment.
FIG. 31B is a side schematic cross-sectional view of the electronics module connected to the wearable device of FIG. 31A.
FIG. 32 is a side schematic cross-sectional view of an electronics module connected to a wearable device, according to another embodiment.
FIG. 33 is a schematic view of a pocket in a body of a wearable device for retaining an electronics module, according to an embodiment.
FIGS. 34A-34C illustrate schematic views of an electronics module with extruding conductive pins for contact with conductive threads, according to an embodiment.
FIG. 35A and 35B illustrate schematic views of an electronics module with recesses for receiving molded contacts, according to an embodiment.
FIGS. 36 and 37 illustrate a side view of an embodiment of a wearable device having an extendable, stretchable forehead contacting portion, according to an embodiment.
FIG. 38 illustrates a side view of an embodiment of a wearable device having an extendable, stretchable forehead contacting portion with attachment locations for auxiliary sensors, according to an embodiment.
FIG. 39A is a schematic perspective view of a wearable device with a touchpad location, according to an embodiment.
FIG. 39B is a schematic top view of the wearable device with the touchpad location of FIG. 39A.
FIG. 40 illustrates a cross-sectional side view of the wearable device with the touchpad location of FIGS. 39A and 39B.
FIG. 41 is a schematic perspective view of a wearable device with an extendable, stretchable forehead contacting portion in which an OLED flexible array may be disposed, according to an embodiment.
FIG. 42 is a schematic perspective view of the wearable device and the OLED flexible array of FIG. 41, in a folded down configuration.
FIG. 43A illustrates a top view of bladders that may be integrated into a wearable device, according to an embodiment, and FIG. 43B illustrates the bladders in use with the wearable device, according to an embodiment.
FIG. 44 is a perspective view of a wearable device having an electronics module disposed underneath a cover in a closed position, according to an embodiment.
FIG. 45 is a perspective view of a wearable device having an electronics module disposed underneath a cover in an open position, according to an embodiment.
FIG. 46 is a perspective view of a wearable device having an electronics module detached from a cover in an open position, according to an embodiment.
FIG. 47 is a perspective view of a wearable device having an electronics module disposed in a pocket, according to an embodiment.
FIG. 48 is a perspective view of a wearable device having an electronics module detached from a pocket, according to an embodiment.
FIG. 49 is a perspective view of a wearable device having an electronics module detached from a pocket and having a fabric flap, according to an embodiment.
FIG. 50 is a high-level block diagram of a bio-signal analysis system, according to an embodiment.
FIG. 51 is a block diagram of example hardware components of a computing device for bio-signal analysis, according to an embodiment.

### DETAILED DESCRIPTION

As used herein, the terms "downward" or "inward" generally refer to a direction toward a user's skin. Similarly, "lower" indicates a component disposed downward relative to another component. In contrast "upward", "upper", or "outward" are generally in a direction opposite the "downward" or "lower" component.

Bio-signals are signals that are generated by biological beings that can be measured and monitored. Electroencephalographs, galvanometers, and electrocardiographs are examples of devices that are used to measure and monitor bio-signals generated by humans. As the human body has many parts which may be controlled through voluntary movement, there are opportunities for capturing and interpreting movements for interacting with a computing device.

A human brain generates bio-signals such as electrical patterns, which may be measured/monitored using an electroencephalogram ("EEG"). These electrical patterns, or brainwaves, are measurable by devices such as an EEG. Typically, an EEG will measure brainwaves in an analog form. Then, these brainwaves may be analyzed either in their original analog form or in a digital form after an analog to digital conversion.

Measuring and analyzing bio-signals such as brainwave patterns can have a variety of practical applications. For example, brain computer interfaces ("BCI") have been developed that allow users to control devices and computers using brainwave signals. In another example, analysis of brainwave patterns during sleep may allow users to understand their sleep patterns and/or improve their quality of sleep.

In order to obtain bio-signal data, it may be desirable for sensors to be in close or constant contact with the user, or a body part of the user. Accordingly, it may be desirable to provide comfortable wearable devices, especially if the device is worn for extended periods, such as overnight, in the case of sleep monitoring; or during periods of high activity or movement.

In an aspect, a computer system is provided that is implemented by one or more computing devices. Computing devices may include one or more client or server computers in communication with one another over a near-field, local, wireless, wired, or wide-area computer network, such as the Internet, and at least one of the computers is configured to receive signals from sensors worn by a user.

In an implementation, sensors include one more bio-signal sensors, such as electroencephalogram (EEG) sensors, electromyography (EMG) sensors, electrocardiogram (ECG or EKG) sensors, galvanometer sensors, electrocardiograph sensors, heart rate sensors such as photoplethysmography (PPG), eye-tracking sensors, blood pressure sensors, breathing sensors, pedometers, gyroscopes, and any other type of sensor. Sensors may be of various types, including: electrical bio-signal sensor in electrical contact with the user's skin; capacitive bio-signal sensor in capacitive contact with the user's skin; blood flow sensor measuring properties of the user's blood flow; and wireless communication sensor placed sub-dermally underneath the user's skin. Other sensor types may be possible, including but not limited to temperature sensors, movement sensors such as accelerometers or gyrometers, sound sensors, such as a listening device to record ambient noise and/or other noise, vibration sensors.

Sensors may be connected to a wearable device, which may be a wearable computing device or a wearable sensing device such as a wearable headset or headband computer worn by the user. Sensors may be connected to the headset by wires or wirelessly. The headset may further be in communication with another computing device, such as a laptop, tablet, or mobile phone such that data sensed by the headset through the sensors may be communicated to the other computing device for processing at the computing device, or at one or more computer servers, or as input to the other computing device or to another computing device. One or more computer servers may include local, remote, cloud based or software as a service platform (SAAS) servers.

Embodiments of the system may provide for the collection, analysis, and association of particular bio-signal and non-bio-signal data with specific mental states for both individual users and user groups. The collected data, analyzed data or functionality of the systems and methods may be shared with others, such as third party applications and other users. Connections between any of the computing devices, internal sensors (contained within the wearable device), external sensors (contained outside the wearable device), user effectors, and any servers may be encrypted. Collected and analyzed data may be used to build a user profile that is specific to a user. The user profile data may be analyzed, such as by machine learning processes, either individually or in the aggregate to function as a BCI, or to improve the algorithms used in the analysis. Optionally, the data, analyzed results, and functionality associated with the system can be shared with third party applications and other organizations through an API. One or more user effectors may also be provided at the wearable device or other local computing device for providing feedback to the user, for example, to vibrate or provide some audio or visual indication to assist the user in achieving a particular mental state, such as a meditative state.

The wearable device may include a camera, a display, and bio-signal measuring means to sample a user's environment as well as the user's bio-signals, determining the user's state and context through sensors and user input. The wearable device may include at least one user-facing camera to track eye movement and / or facial expressions. In an aspect, the wearable device may be in a form resembling eyeglasses wearable on the user's face. Optionally, at least one camera may be oriented to generally align with the user's field of view. Embodiments may also include a listening device, which may be integrated with the wearable device, or separate.

In another aspect, the wearable device may be in a form of at least one sensor adapted to being placed at or adhered to the user's head or face. Each sensor may optionally communicate with one another either through wires or wirelessly. Each sensor may optionally communicate with a controller device either through wires or wirelessly. The controller device may be mounted to the wearable device in order to reside at or near the user's head or face. Alternatively, the controller device may be located elsewhere on the user's body, such as in a bag or pocket of the user's clothing. The controller device may also be disposed somewhere outside the user's body. For example, the sensors may monitor the user, storing data in local storage mounted to the wearable device, and once moving into proximity with the controller device, the sensors, or a transmitter of the wearable device may transmit stored data to the controller device for processing. In this implementation, the wearable device would be predominantly usable by the user when located nearby the controller device.

The wearable device may include a camera, a display and bio-signal measuring means. At least one of the bio-signal measuring means may employ at least one sensor in order to measure brain activity. Brain activity may be measured through electroencephalography ("EEG") techniques electrically, or through functional near-infrared spectroscopy ("fNIR") techniques measuring relative changes in hemoglobin concentration through the use of near infrared light attenuation. A sensor employing pulse oximetry techniques may also be employed in the wearable device. Optionally, the wearable device may include at least one sensor measuring eye activity using electrooculography ("EOG") techniques. Other sensors tracking other types of eye movement may also be employed.

In various implementations, the wearable device may include a variety of other sensors and input means. For example, the wearable device may comprise at least one audio transducer such as a single microphone, a microphone array, a speaker, and headphones. The wearable device may comprise at least one inertial sensor for measuring movement of the wearable device. The wearable device may comprise at least one touch sensor for receiving touch input from the user.

The wearable device may be configured to accept user input through the user's touch or body movements using an accelerometer or through an EEG sensor (e.g., worn near the ear). The wearable device may be configured to accept user inputs from slide touch inputs along a capacitor. The wearable device may be configured to accept user inputs from user eye commands (e.g., movement or blinking) using sensors configured to track the user's eye movement. The wearable device may be configured to accept user input from changes in muscle tension by measuring variations in device tension or EEG signals. The wearable device may accept input from a user's auditory commands using a microphone or array of microphones. The wearable device may be configured to accept user input from another device that is wirelessly connected such as a mobile computing device that accepts touch or voice inputs, examples include a handheld computing device, a desktop or laptop computer, a home automation device, a device such as a Google home or Alexa enabled speaker, a wearable device such as a wrist watch form factor, a ring etc. The wearable device may be configured to accept user input from a remote controller that uses light, sound, or radio frequency to communicate to the device.

The wearable device may sample from both the user's environment and bio-signals simultaneously or generally contemporaneously to produce sampled data. The sampled data may be analyzed by the wearable device in real-time or at a future predetermined time when not being worn by the user.

The wearable device may comprise user input detection methods that are adaptive and improve with use over time. Where the user attempts to command the wearable device, and the wearable device responds in an unexpected way, the user may attempt to correct the previous input by indicating that the wearable device response was incorrect, and retrying the initial command again. Over time, the wearable device may refine its understanding of particular user inputs that are corrected. Some user inputs may be easier to successfully measure with a high degree of accuracy than others. It may be preferable to assign a high-accuracy input to command the wearable device that the previous input was incorrect. For example, tapping the wearable device in a particular spot may indicate that the previous input response was incorrect. Explicit training such as with voice recognition may also be used to configure and command the wearable device.

Optionally, the wearable device may itself only provide bio-signal sensors and a processor for processing measurements from the sensors. The wearable device may communicate these measurements or data derived from processing the measurements to one or more secondary devices, such as glasses with video cameras embedded therein. In any of the implementations, embodiments, or applications discussed herein, it should be understood that some actions may be carried out by a plurality of interconnected devices, or just one of the wearable devices as disclosed herein. For example, the wearable device may not include a display. In such an example, the wearable device may communicate visual information to the user through the use of a second device, such as glasses with video cameras embedded therein, which does include a display.

Sensors usable with the wearable device may come in various shapes and be made of various materials. For example, the sensors may be made of a conductive material, including a conductive composite like rubber or conductive metal. The sensors may also be made of metal plated or coated materials such as stainless steel, silver-silver chloride, and other materials.

In addition to or instead of processing bio-signal measurements on the wearable device, the wearable device may communicate with one or more computing devices in order to distribute, enhance, or offload the processing of the bio-signal measurements taken or received by the wearable device. In particular, the one or more computing devices may maintain or have access to one or more databases maintaining bio-signal processing data, instructions, algorithms, associations, or any other information which may be used or leveraged in the processing of the bio-signal measurements obtained by the wearable device. The computing devices may include one or more client or server computers in communication with one another over a near-field, local, wireless, wired, or wide-area computer network, such as the Internet, and at least one of the computers may be configured to receive signals from sensors of the wearable device.

The wearable device may further be in communication with another computing device, such as a laptop, tablet, or mobile phone such that data sensed by the headset through the sensors may be communicated to the other computing device for processing at the computing device, or at one or more computer servers, or as input to the other computing device or to another computing device. The one or more computer servers may include local, remote, cloud based or software as a service platform (SAAS) servers. Embodiments of the system may provide for the collection, analysis, and association of particular bio-signal and non-bio-signal data with specific mental states for both individual users and user groups. The collected data, analyzed data or functionality of the systems and methods may be shared with others, such as third-party applications and other users. Connections between any of the computing devices, internal sensors (contained within the wearable device), external sensors (contained outside the wearable device), user effectors (components used to trigger a user response), and any servers may be encrypted. Collected and analyzed data may be used to build a user profile that is specific to a user. The user profile data may be analyzed, such as by machine learning algorithms, either individually or in the aggregate to function as a BCI, or to improve the algorithms used in the analysis. Optionally, the data, analyzed results, and functionality associated with the system can be shared with third party applications and other organizations through an API. One or more user effectors may also be provided at the wearable device or other local computing device for providing feedback to the user, for example, to vibrate or provide some audio or visual indication to assist the user in achieving a particular mental state, such as a meditative state. In an example, a light emitter may be in close proximity to a user's eyes, and provide the user with feedback by way of visual stimulus, such as light colour, frequency, intensity.

A cloud-based implementation for processing and analyzing the sensor data may provide one or more advantages including: openness, flexibility, and extendibility; centrally manageable; reliability; scalability; being optimized for computing resources; the ability to aggregate information across a number of users; and the ability to connect across a number of users and find matching sub-groups of interest. While embodiments and implementations may be discussed in particular non-limiting examples with respect to use of the cloud to implement aspects of the system platform, a local server, a single remote server, a SAAS platform, or any other computing device may be used instead of the cloud.

In one implementation of the system, a Multi-modal EEG Data-Collection and Adaptive Signal Processing System (MED-CASP System) for enabling single or multi-user mobile brainwave applications may be provided for enabling BCI applications. This system platform may be implemented as a hardware and software solution that is comprised of an EEG headset such as a wearable device as disclosed herein, a client side application and a cloud service component. The client side application may be operating on a mobile or desktop computing device. The system may provide for: estimation of hemispheric asymmetries and thus facilitate measurements of emotional valence (e.g. positive vs. negative emotions); and better signal-to-noise ratio (SNR) for global measurements and thus improved access to high-beta and gamma bands, which may be particularly important for analyzing cognitive tasks such as memory, learning, and perception. It has also been found that gamma bands are an important neural correlate of meditation expertise.

In the same or another non-limiting exemplary implementation, possible MED-CASP system features may include: uploading brainwaves and associated sensor and application state data to the cloud from mobile application; downloading brainwave & associated data from the cloud; real-time brain-state classification to enable BCI in games or other applications; transmitting real-time brain-state data to other users when playing a game to enable multi-user games; sharing brainwave data with other users to enable asynchronous comparisons of results; sharing brainwave data to other organizations or third party applications and systems; and support of cloud based user profiles for storing personal information, settings and pipeline parameters that have been tuned to optimize a specific user's experience. In this way, usage of the system platform can be device independent.

Each time analysis or processing of user bio-signal data (such as brainwave data) is performed, an instance of aspects of the software implementing analysis functionality may be generated by the wearable device, initiated at either the device or the cloud, in order to analyze the user's private bio-signal data using particular analysis or processing parameters applied during the analysis or processing. For simplicity, such an instance may be referred to as an algorithm "pipeline". Each instance of the pipeline may have an associated pipeline identifier ("ID"). Each pipeline may be associated with a particular activity type, user, bio-signal type of a particular user, application, or any other system platform-related data. Each pipeline may maintain particular pipeline parameters determined to analyze the user's bio-signal data in a particular way, consistent either with previous analysis of the particular user's bio-signal data, consistent with previous analysis of one or more other user's bio-signal data, or consistent with updated data at the cloud server derived from new or updated scientific research pertaining to the analysis of bio-signal data. Pipelines and/or pipeline parameters may be saved for future use at the client computing device or at the cloud. When a new pipeline is created for the user, the wearable device or the cloud may provide a new algorithm pipeline ID to be associated with the new pipeline at the cloud and at the device.

Each person's brainwaves are different, therefore requiring slightly different tunings for each user. Each person's brain may also learn over time, requiring the system platform to change algorithm parameters over time in order to continue to analyze the person's brainwaves. New parameters may be calculated based on collected data, and may form part of a user's dynamic profile (which may be called a bio-signal interaction profile). This profile may be stored in the cloud, allowing each user to maintain a single profile across multiple computing devices. Other features of the same or another non-limiting exemplary implementation may include: improving algorithms through machine learning applied to collected data either on-board the client device or on the server; saving EEG data along with application state to allow a machine learning algorithm to optimize the methods that transform the user's brainwaves into usable control signals; sharing brainwave data with other applications on mobile device through a cloud services web interface; sharing brainwave data with other applications running on client devices or other devices in the trusted network to provide for the user's brainwave data to control or effect other devices; integration of data from other devices and synchronization of events with brainwave data aid in context aware analysis as well as storage and future analysis; performing time locked stimulation and analysis to support stimulus entrainment event-related potential ("ERP") analysis; and data prioritization that maximizes the amount of useful information obtainable from an incomplete data download (i.e. data is transmitted in order of information salience). The core functionality of the MED-CASP system may be wrapped as an externally-usable library and API so that another developer may use the platform's features in the developer's application(s). The library may be a static library and API for Unity3D, iOS, Android, OSX, Windows, or any other operating system platform. The system platform may also be configured to use a pre-compiled algorithm supplied by a third party within the library, including the ability for a third-party developer using the library, to use the developer's own algorithms with the library. The system platform may also support headsets from a variety of vendors; personal data security through encryption; and sharing of un-curated data (optionally using time-limited and fidelity-limited access) though the sharing of encryption keys.

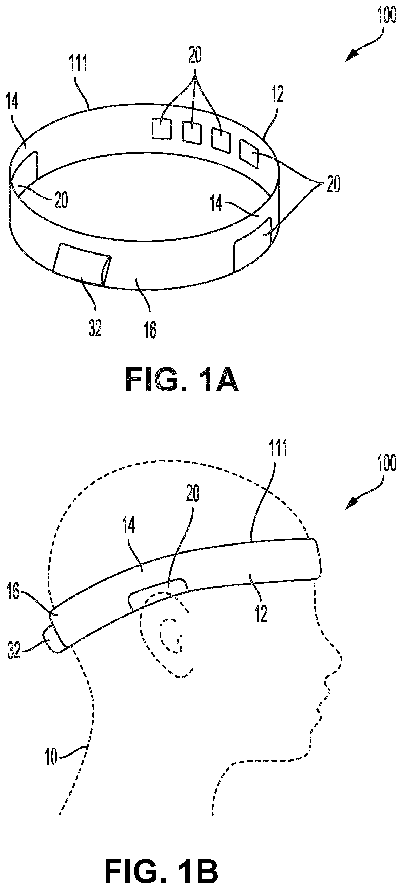

With reference to FIGS. 1A and 1B, in an aspect of the present disclosure, a wearable device 100 includes a front portion (in an example, a forehead contacting portion 12), a rear portion (in an example, an occipital contacting portion 16) and at least one side portion - for example, a right side portion and a left side portion - (in an example, two ear contacting portions 14) extending between the front portion and the rear portion to contact at least part of an auricular region of the head of user 10. Wearable device 100 may include one or more bio-signal sensors 20, such as electrodes, which may be carried and transmitted within wearable device 100 providing internal connectivity, and bio-signal sensors 20 may be connected to one or more electric modules 32.

FIG. 1B illustrates a side view of a user 10 wearing a wearable device 100, according to an embodiment. The forehead contacting portion 12, the two ear contacting portions 14, and the occipital contacting portion 16 are joined to form a body 111, as a flexible band generally shaped to correspond to the head of user 10.

**It** will be appreciated that wearable device 100 can be worn on various parts or portions of a user's body, including but not limited to a user's arm or wrist, a user's leg or ankle, and a user's chest or torso, for example, to measure heart and respiration data, and other data including temperature and temperature changes, as described herein.

**The** body 111 is extendible in length or diameter. By adjusting length or diameter of wearable device 100, it may be configured to be wearable at many different body parts. Furthermore, the modular aspects of various components of wearable device 100 may allow for certain components, such as electronics module 32 in various embodiments, to be used with a variety of other devices or form factors.

The flexible and extendable body 111 can extend in length or size to be longer or larger. The body can have an extendible portion in some embodiments. For example, body can have stretch and non-stretch portions and the stretch portion can extend the body. The body may include fabric and elasticized portions, for example, for extending in length or size. The flexible and extendable body can bend or be modified to adapt to different shapes and sizes of users and to encircle different portions of a user.

In some embodiments, wearable device 100 does not need to be secured directly to a user's body. For example, wearable device 100 can be mounted to a user's equipment, such as attached to a helmet used for bicycling or skiing while monitoring signals such as EKG.

Body 111 may form, for example, a loop configuration as shown in FIGS. 1A and 1B, and may have specific areas of stretch and non-stretch. For example, the body 111 can be made of stretch material or a combination of stretch material and non-stretch material.

Body 111 may be shaped such that, when worn, forehead contacting portion 12 contacts a forehead of a user, two ear contacting portions 14 contact the tops of the user's ears and occipital contacting portion 16 contacts the bottom of the user's occipital bone. At least one bio-signal sensor 20 may be located on the loop of body 111 on an inward facing side for receiving bio-signals from the user.

Body 111 may include material, textile, or fabric and elasticized portions. **In** some embodiments, some or all portions of body 111 are elastic or on an elastic substrate, while other portions or sections are relatively inelastic or rigid. Body 111 may be formed from a soft deformable fabric 121, for example, a woven, a knit, or a non-woven fabric. Fabric 121 may be formed, for example from a fabric that is cotton, synthetic, or any other suitable fabric. In an example, fabric 121 may be formed of 88% rayon, 9% nylon and 3% spandex. In some embodiments, fabric 121 of body 111 may be machine washable.

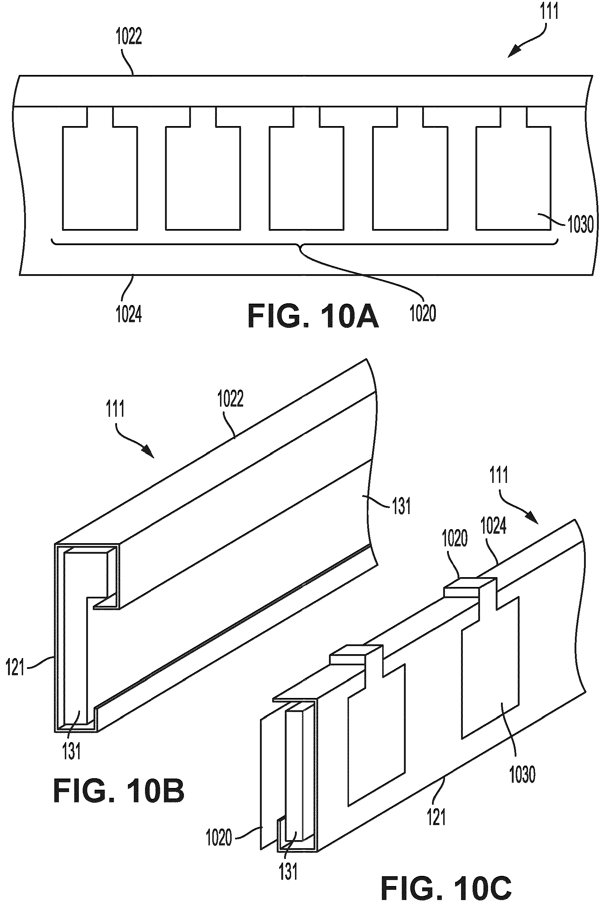

Body 111 may also include one or more reinforcing members 131 at various locations, for example to provide structural support to wearable device 100. Reinforcing members 131 may include a compressible foam, in an example, covered by a fabric 121, as shown by way of example in FIGS. 10B and 10C, which may conform to the shape of the head or other body part of user 10. In some embodiments, the compressible foam may be formed of an open cell foam, such as a suitable open cell foam material. In some embodiments, the compressible foam may be formed of a closed cell foam, such as a neoprene. Compressible foam may be compressible such that when the wearable device 100 is affixed to the head of user 10, the compressible foam conforms to the head of user 10. In use, the compressible foam may be compressed and conform to the head of user 10 by clinching of body 111 to size and secure wearable device 100 to user 10.

Parts of body 111 may be formed from foam that is molded to a specific shape of a user's head or other body part. For example, the circumference of body 111 may taper to correspond to a head shape. Foam used in body 111 may be shaped, for example, heat-formed, to mold to a user's particular shape of head. More generally, a compressible material such as foam used in body 111 may be heat-formed into the shape of a typical head, and not necessarily unique to an individual user. In some embodiments, compressible foam or foam may be shaped to conform a bio-signal sensor 20 to a section of the body of user 10 and prevent the sensor from moving, such as a user's ear to improve contact of a bio-signal sensor 20 to user's body, which may thereby improve the bio-signals received by a bio-signal sensor.

In some embodiments compressible foam or foam may be 3D printed to conform to a specific shape.

In some embodiments, a top portion of body 111 does not include foam or other compressible and/or reinforcing material, and a bottom portion includes foam, which may be reinforcing, and conveniently may provide slight flexibility to adjust body 111 around a user's ears more comfortably.

In some embodiments, side or ear contacting portions 14 of wearable device 100 may have foam stitched in along the bottom to provide structure and stability, but the top portion of the body (e.g., the top third) does not have the foam structure, this gives the top portion some elasticity (the material is slightly elastic, but the foam portion will not stretch well because of the foam) to bend the arm down over the ear to ensure proper contact.

Other reinforcing materials, such as interfacing in an example, may be used to provide rigidity, inelasticity, and/or inflexibility in certain areas of body 111, for example, where components such as bio-signal sensor 20 may be mounted.

In some embodiments, shielding may be incorporated into the fabric of body 111, for example, to shield conductive lines between bio-signal sensors 20 and electronics module 32.

In some embodiments, layering of wearable device 100 may include within the layers, memory foam, heat bonded tape, elastic, conductive thread, flexible printed circuit board, and other suitable structural components.

In some embodiments, wearable device 100 integrates flexible electrodes that touch the skin for use as bio-signal sensors 20. Electronics module 32 can optionally be configured to remove from body 111 or can be permanently affixed to body 111 in the manufacturing process.

Flexible electrodes can be manufactured for incorporation within the material of body 111. Flexible electrodes can be fabricated from the stacking of materials in a particular way (referred to as a 'stack up') which allows the sensor portion to detect a bio-signal from a user and communicate that signal to the processing center of the wearable device, such as electronics module 32.

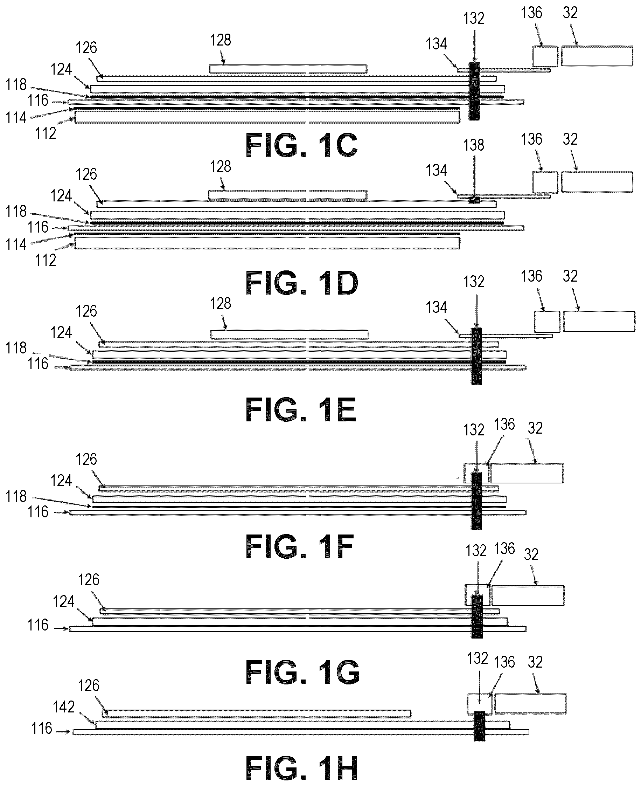

FIG. 1C illustrates an example of a stack-up that can be used to form a flexible electrode, such as bio-signal sensor 20, of wearable device 100. The stack up can comprise foam base 112, first adhesive layer 114, fabric layer 116, second adhesive layer 118, polyurethane layer 124, and conductive polymer layer 126. To block sensing in areas where receiving bio-signals is undesirable, conductive polymer layer 126 can optionally be blocked by dielectric coating 128. Conductive polymer layer 126 is configured to contact rivet 132. Rivet 132 can conduct a signal from conductive polymer layer 126 to electrical connection 136 through flexible printed circuit board 134. Electrical connection 136 can communicate the signal to electronics module 32.

Conductive polymer layer 126 can be a polymer composition that includes silver and / or carbon (e.g. PE874, and PE671 by Dupont). Conductive polymer layer 126 can be applied to both sides of polyurethane layer 124 and may have a different composition depending on the side. For example, the skin-exposed side of conductive polymer layer 126 may comprise a silver loaded material that is configured to touch the skin of a user, with a carbon loaded material beneath to adhere to the polyurethane layer 124. The side opposing the skin-exposed side may comprise a carbon layer that can provide shielding or some other electrical function. The top of conductive polymer layer 126 may feature a PEDOT surface coating to enhance conductivity with a user's skin.

Polyurethane layer 124 can comprise a thin flexible thermoplastic polyurethane (TPU), or other suitable polymer or rubber. The first and second adhesive layers 114 and 118 can comprise a heat activated adhesive that can be activated during a heat press process. Rivet 132 can extend through all layers of the stack up as illustrated in FIG. 1C. **In** some embodiments, electrical connection 136 and electronics module 32 can be permanently affixed to body 111 during manufacturing. Dielectric coating 128 can comprise PE773 encapsulant insulator.

FIG. 1D illustrates an example of an alternative stack up that can be used to form a flexible electrode, such as bio-signal sensor 20. An embodiment as illustrated FIG. 1D can include the same or similar elements as the embodiment illustrated in FIG. 1C, except there is a conductive adhesive 138 in place of rivet 132 to conduct the signal from the conductive polymer layer 126 to flexible printed circuit board 134. Embodiments of any stack up that conducts a signal through rivet 132 can alternatively use a conductive adhesive 138 or other suitable conductive connection (e.g., a crimp).

FIG. 1E illustrates a variant stack up that can be used to form a flexible electrode, such as bio-signal sensor 20. An embodiment as illustrated FIG. 1E can include the same or similar elements as the embodiment illustrated FIG. 1D, except that it lacks foam layer 112 and first adhesive layer 114 connecting foam layer 112 to fabric layer 116. Stack ups such as those shown in FIG. 1E can be used in embodiments that do not utilize foam within body 111. The omission of foam layer 112 may improve comfort by reducing thickness or stiffness of the stack up, such as where the electrode fits above the ears. The flexible electrode may be bent such as to increase contact area with the skin, and the exclusion of foam at or around the bent region may allow for greater conformability in the region of the deformation.

FIG. 1F illustrates an example of a stack up that can be used to form a flexible electrode, such as bio-signal sensor 20. An embodiment as illustrated FIG. 1F can include the same or similar elements as the embodiment illustrated in FIG. 1E except that it lacks the flexible printed circuit board 134 and it lacks optional dielectric coating 128. In these stack ups, rivet 132 (or, optionally, a conductive adhesive or crimp) conducts the signal from conductive polymer layer 124 directly to electrical connection 136. Embodiments that do not make use of flexible printed circuit board 134 can be suitable for a wearable device 100 where rivet 132 need not be spatially distant from electrical connection 136. The omission of flexible printed circuit board 134 may offer the benefit of reducing size of the overall assembly, or improving comfort and reliability due to the minimization of connections and material changes.

FIG. 1G illustrates an example of a stack up that can be used to form a flexible electrode, such as bio-signal sensor 20. An embodiment as illustrated in FIG. 1G can include the same or similar elements as the embodiment illustrated in FIG. 1F except it lacks second adhesive layer 118 that connects polyurethane layer 124 to fabric layer 116. In these embodiments, polyurethane layer 124 can be molded onto fabric layer 116 during the manufacturing process. This can remove the need for an adhesive layer between polyurethane 124 and fabric layer 116. Embodiments such as this can remove the need for some adhesives in the manufacturing process.

FIG. 1H illustrates an example of a stack up that can be used to form a flexible electrode, such as bio-signal sensor 20. An embodiment as illustrated in FIG. 1H can include the same or similar elements as the embodiment illustrated in FIG. 1G except that the conductive polymer layer 126 does not extend to rivet 132 (or, optionally, a conductive adhesive or a crimp) and polyurethane layer 124 has been replaced with conductive rubber layer 142. Conductive rubber layer 142 can be, for example, a carbon loaded TPU. Conductive rubber layer 142 can optionally be molded onto fabric layer 116 during the manufacturing process thereby eliminating the need for an adhesive layer to bond conductive rubber layer 142 to fabric layer 116 In these embodiments, conductive rubber layer 142 conducts the signal to rivet 132 (or optionally, a conductive adhesive or a crimp). Embodiments such as this provide an alternative means to transmit a signal from the sensors to a processing unit.

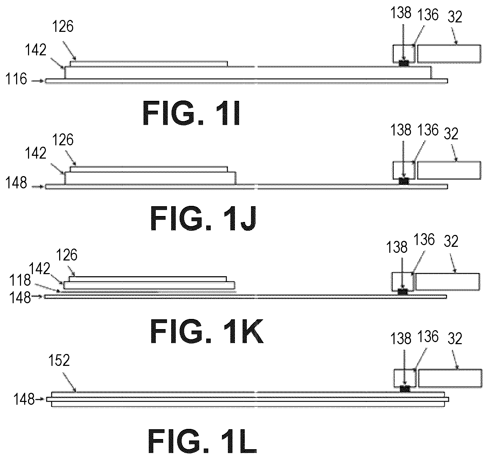

FIG. 1I illustrates an example of a stack up that can be used to form a flexible electrode, such as bio-signal sensor 20. An embodiment as illustrated in FIG 1I can include the same or similar elements as the embodiment illustrated in FIG. 1H except that rivet 132 has been replaced with conductive adhesive 138. Embodiments of a stack up that conducts a signal through rivet 132 can alternatively use a conductive adhesive 138 or other suitable conductive connection (e.g., a crimp).

FIG. 1J illustrates an example of a stack up that can be used to form a flexible electrode, such as bio-signal sensor 20. FIG. 1J illustrates conductive textile 148, affixed to conductive rubber layer 142. Conductive rubber layer 142 can be affixed to conductive polymer layer 126. Conductive polymer layer 126 can comprise a PEDOT coating (e.g., Techticoat by Heraeus). Conductive adhesive 138 connects conductive textile 148 to electrical connection 136. Electrical connection 136 is connected to electronics module 32. A bio-signal from a user can be conducted from conductive polymer layer 126 to conductive rubber layer 142, to conductive textile layer 148. Conductive textile layer 148 can conduct the signal to electrical connection 136 through conductive adhesive 138. Electronics module 32 can receive the signal from electrical connection 136. In some embodiments, conductive adhesive 138 can be replaced with a conductive crimp. Embodiments such as these provide yet another means of conducting a signal from the sensor through body 111 to electronics module 32.

FIG. 1K illustrates an example of a stack up that can be used to form a flexible electrode, such as bio-signal sensor 20. An embodiment as illustrated in FIG 1K can include the same or similar elements as the embodiment illustrated in FIG. 1J except that conductive textile 148 has been adhered to conductive rubber 142 using second adhesive layer 118. Embodiments such as these provide yet another means of conducting a signal from the sensor through body 111 to electronics module 32.

FIG. 1L illustrates an example of a stack up that can be used to form a flexible electrode, such as bio-signal sensor 20. Conductive textile 148 has a PEDOT coating 152 applied to fibres woven into the textile. Conductive adhesive 138 connects PEDOT coating 152 of conductive textile 148 to electrical connection 136. Electrical connection 136 transmits any signals received to electronics module 32. Embodiments such as these provide yet another means of conducting a signal from the sensor through body 111 to electronics module 32.

Stack ups such as those illustrated in FIG. 1C - FIG. 1L demonstrate how an electrode can be manufactured into wearable device 100. Such stack ups can provide flexible electrodes, such as bio-signal sensor 20, that can be more comfortable for a user to wear for long periods of time or during times when comfort is important. The stack ups can implemented in wearable device 100 alone or with other sensor implementations.

Returning to FIG. 1A, in some embodiments, forehead contacting portion 12 and occipital contacting portion 16 of body 111 are arcuate and are joined by two ear contacting portions 14. In some embodiments, the two arcuate portions are joined at each of the two ear contacting portions such that an angle forms between them, not as a straight line. In some embodiments, the angle is between about 90° and about 180°, between about 135° and about 180°, between about 155° and about 170°. In some embodiments, the angle is an oblique angle with the vertex located proximate the ear. A bend by the ear contacting portion 14 may allow a computing device to better conform to the head with less deformation of the wearable device 100 when worn and/or with better stability when worn. Further, a bend by the ear may follow the curvature of the ear, increasing the electrical contact area of a bio-signal sensor 20 located above the user's ear.

In some embodiments, body 111 may be formed as a single, integral piece, and thus wearable device 100 forms a general loop shape.

In some embodiments, wearable device 100 may include an attachment mechanism, such as a clasp or connector, for secure wearable device 100 to user 10.

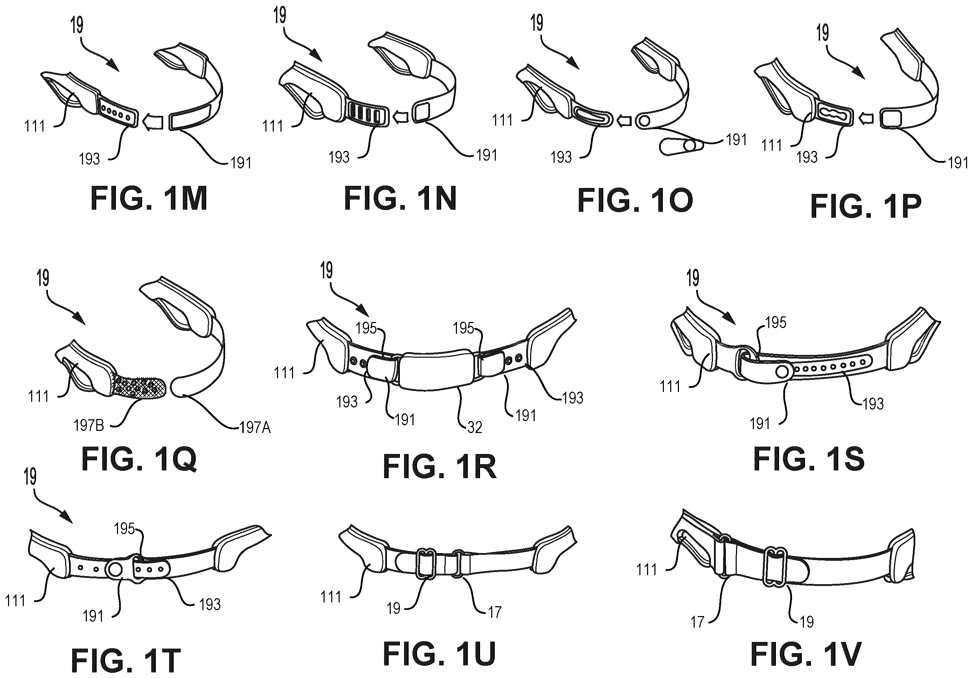

Wearable device 100 may be sized and secured to user 10, for example, using a cinch strap, fastened, for example, by hook and loop fasteners (such as Velcro^{™}), snap mechanism, buckle such as center post buckle, a magnet, or the like. The attachment mechanisms may be affixed to the wearable devices via a stretchable or non-stretchable material. A non-stretchable material may be used to increase comfort and ease of fit, and to create a consistent tension within the wearable device. FIGS. 1M-1V non-exhaustively illustrate various example embodiments of attachment mechanisms for wearable device 100. In these figures, the attachment mechanisms may attach to wearable device 100 via a stretchable material that protrudes within the wearable device to decrease variation of tensile force along the length of the wearable device, when it is lengthened. This can improve comfort and ease of use by reducing the need for accurate adjustment to the user's size.

FIG. 1M illustrates an example embodiment of fastener 19 using a button attachment mechanism for wearable device 100. A button, such as protrusion 191, can be received by any slot in plurality of apertures 193. Each aperture 193 may define a generally circular opening. The size of body 111 can be determined by the slot with which protrusion 191 is coupled.

FIG. 1N illustrates an example embodiment of fastener 19 using a hook attachment mechanism for wearable device 100. A hook, such as protrusion 191, can be received by any slot in plurality of apertures 193. Each aperture 193 may define a generally rectangular opening. The size of body 111 can be determined by the slot with which protrusion 191 is coupled.

FIG. 1O illustrates an example embodiment of fastener 19 using a button attachment mechanism for wearable device 100. A button such as protrusion 191 is received by aperture 193 to couple the ends of body 111 together. Aperture 193 may define a generally elongated ellipsoid opening.

FIG. 1P illustrates an example embodiment of fastener 19 using a button attachment mechanism for wearable device 100. A button such as protrusion 191 can be received by any slot in plurality of slots such as apertures 193. Aperture 193 may define a generally elongated and ribbed ellipsoid opening. The size of body 111 can be determined by the slot with which button 191 is coupled.

FIG. 1Q illustrates an example embodiment of fastener 19 using a hook and loop fastener, such as Velcro^{™}. The size of body 111 can be determined by the coupling position of first portion 197A and second portion 197B. First portion 191 can be either the hook component or loop component of Velcro^{™} and second portion 193 is the corresponding loop component or hook component respectively.

FIG. 1R illustrates an example embodiment of fastener 19 using dual belt slide button attachment mechanisms. Electronics module 32 can be affixed to loops 195. Both ends of body 111 comprise protrusion 191 and plurality of slots such as apertures 193. Body 111 is configured to extend through hook 195. Protrusion 191 can couple with a slot in plurality of apertures 193 on the same end of body 111 as protrusion 191.

FIG. 1S illustrates an example embodiment of fastener 19 using a slide button attachment mechanism. One end of body 111 comprises a button scuh as protrusion 191 and a plurality of slots such has apertures 193. The other end of body 111 comprises hook 195. The end of body 111 with button 191 is configured to extend through hook 195. Protrusion 191 can couple with a slot in plurality of slots such as apertures 193. Sizing of body 111 can be determined by the specific slot with which protrusion 191 couples.

FIG. 1T illustrates an example embodiment of fastener 19 using a slide button attachment mechanism. One end of body 111 comprises a button such as protrusion 191. The other end of body 111 comprises a plurality of slots such as apertures 193 and hook 195. The end of body 111 with protrusion 191 is configured to extend through hook 195. Button 191 can couple with a slot in plurality of apertures 193. Sizing of body 111 can be determined by the specific slot with which button 191 couples.

FIG. 1U illustrates an example embodiment of fastener 19. Adjustor 17 can adjust the length of body 111 when fastener 19 is fastened.

FIG. 1V illustrates an example embodiment of fastener 19. Adjustor 17 can adjust the length of body 111 when fastener 19 is fastened.

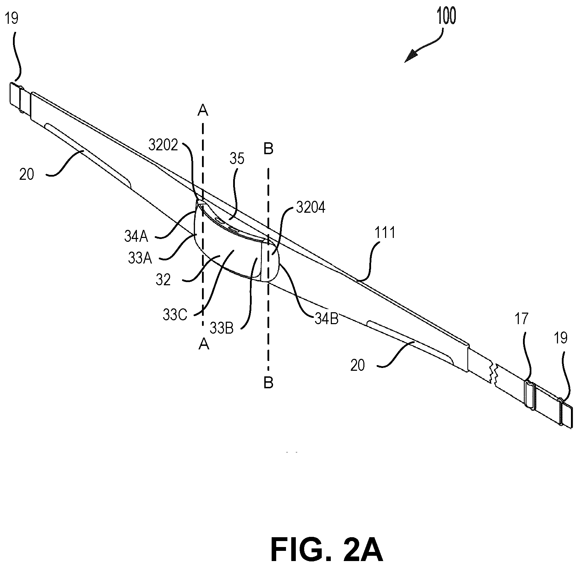

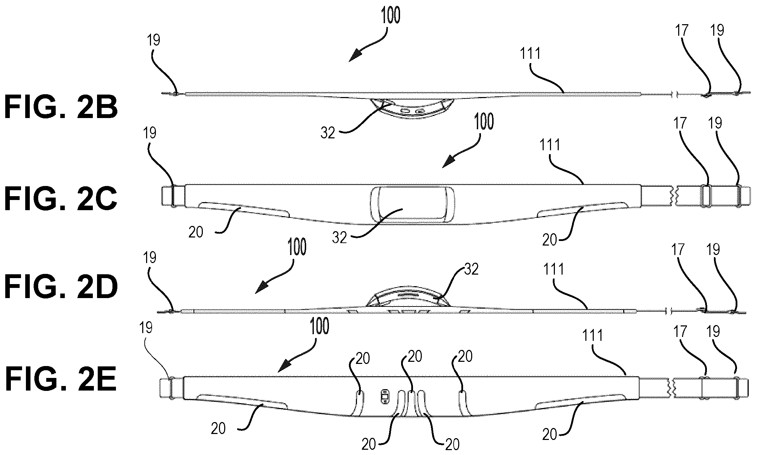

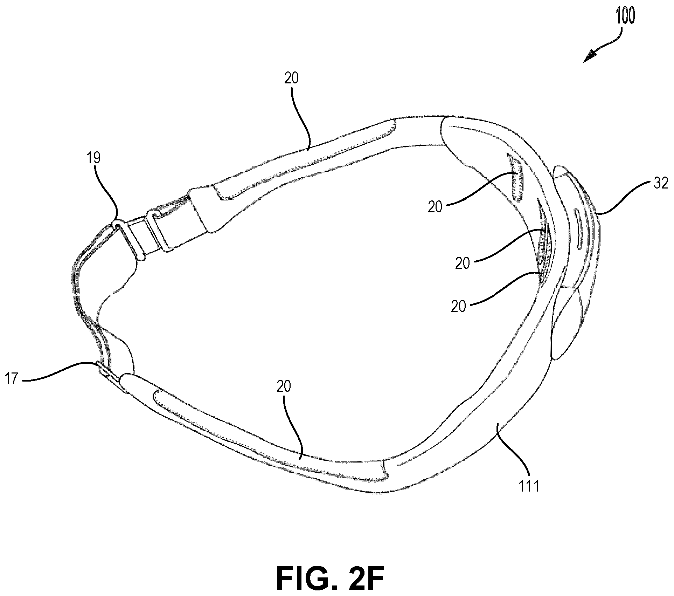

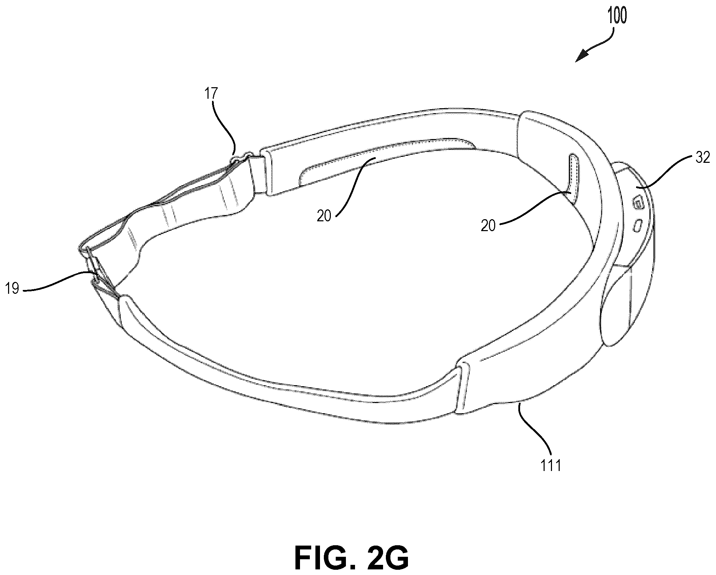

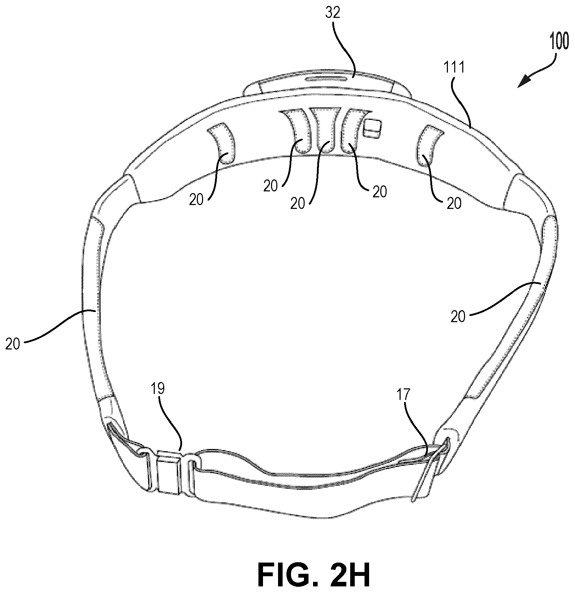

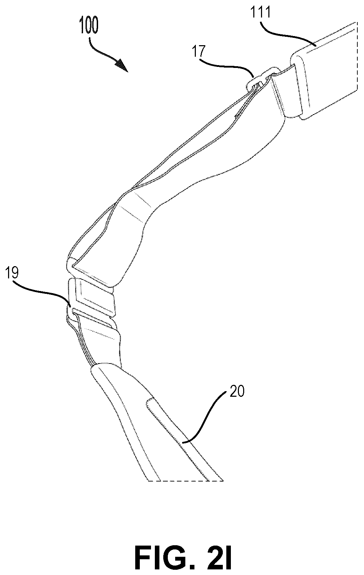

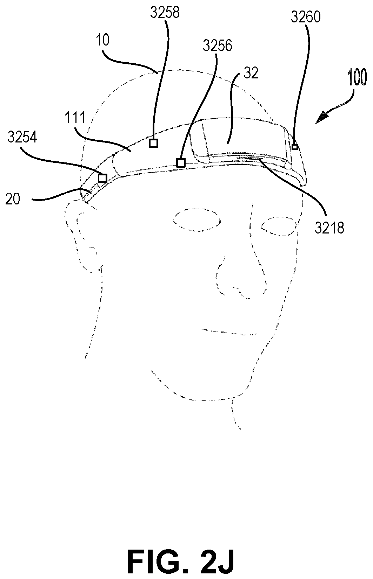

FIG. 2A illustrates a perspective view of a wearable device 100, according to an embodiment. FIG. 2B illustrates a top view of wearable device 100 of FIG. 2A; FIG. 2C illustrates a front view of wearable device 100 of FIG. 2A; FIG. 2D illustrates a bottom view of wearable device 100 of FIG. 2A; FIG. 2E illustrates a rear view of wearable device 100 of FIG. 2A; FIG. 2F illustrates a bottom perspective view of wearable device 100 of FIG. 2A; FIG. 2G illustrates a top perspective view of wearable device 100 of FIG. 2A; FIG. 2H illustrates a rear-bottom perspective view of wearable device 100 of FIG. 2A; FIG. 2I is a perspective view of an adjuster and a clasp of wearable device 100 of FIG. 2A; FIG. 2J illustrates a perspective view of wearable device 100 of FIG. 2A when worn by user 10.

As shown in FIGS. 2A-2J, in an embodiment, wearable device 100 includes body 111 to which electronics module 32 is disposed. Bio-signal sensors 20 are disposed on a rear face of body 111, and configured to contact, for example, a forehead of user 10 and disposed on a bottom portion of body 111 and configured to contact, for example, at least part of the auricular region of the user's head, for example, an ear or a mastoid bone region of user 10. Wearable device can also include an adjuster 17, such as a D-ring or a slide buckle as illustrated in FIG. 2I to adjust a length of wearable device 100 to fit a user, such as the user's head or chest, and a clasp 19, such as a snap as shown in FIG. 2I which may include a pair of polarized magnets that are magnetically attracted to each other, to secure wearable device 100 and generally form a loop.

Referring to FIG. 2A, in some embodiments, wearable device 100 can include a flexible and extendable body 111 configured to encircle a body part of user 10. Electronics module 32 can have a surface 33A defining concave space 35 between a first end 33A and a second end 33B opposite to first end 33A. First end 33A is attachable to body 111, for example, at a first connection point 34A, with first retention mount 3202 to allow rotation of body 111 relative to electronics module 32 about a first axis, such as a first axis A-A, and to transfer a force, such as tensile or tension force, from body 111 to electronics module 32 radially from the first axis. Second end 33B attachable to body 111, for example, at a second connection point 34B, with a second retention mount 3204 to allow rotation of body 111 relative to electronics module 32 about a second axis, such as a second axis B-B, and to transfer a force, such as tensile or tension force, from body 111 to electronics module 32 radially from the second axis.

One or more bio-signal sensors 20 can be disposed on body 111 between first connection point 34A and second connection point 34B to contact at least part of user 10's body and to receive bio-signals from user 10. When body 111 is extended to be worn by user 10 with electronics module 32 attached, a force, such as a tension force, retaining force, or other suitable force, is applied from body 111 to electronics module 32 and acts on electronics module 32 through first retention mount 3204 and/or second retention mount 3202 to draw electronics module 32 towards user 10's body, a portion of body 111 between first connection point 34A and second connection point 34B rotates towards the concave space, and electronics module 32 urges bio-signal sensor 20 on body 111 against user 10's body.

In some embodiments, a force, such as a retaining force, created by the electronics module 32 and the body acts on body 111 to urge the bio-signal sensor 20 on the body 111 against the user 10's body.

In some embodiments, drawing of electronics module 32 towards user 10's body creates tension in body 111 between first connection point 34A and second connection point 34B.

In some embodiments, the retaining force can push bio-signal sensors 20 against a variety of user body curvatures. The first retention mount 3202 and second retention mount 3204 can retain electronics module 32 against body 111 while maintaining the retaining force in the forehead region of wearable device 100 in a manner that is comfortable for users of different body curvatures.

In some embodiments, a stiffness provided by a reinforcing material in body 111 can result in a force pushing body 111 and electronics module 32 apart, such that body 111 is urged against user 10's body and subsequently bio-signal sensors 20 urged against user 10's body.

In some embodiments, a compressible material, such as foam, is disposed between body 111 and electronics module 32, for example, in some or all of concave space 35. Such foam may exert a spring force between body 111 and electronics module 32 upon compression (displacement of the compressible material as it compresses).

Various properties of body 111 may be modified to urge bio-signal sensors 20 against user 10's body.

Referring to FIG. 2J, in some embodiments, bio-signal sensor 20 is configured to contact at least part of a frontal region of user 10's head. **In** some embodiments, bio-signal sensor 20 is an electroencephalography (EEG) sensor.

In some embodiments, bio-signal sensor 20 is an electrode, such as disclosed herein, to measure electrical potential and generate electric potential, for example, for transcranial stimulation and galvanic skin response.

In some embodiments, wearable device 100 can also include electrical connection 3212 between electronics module 32 and body 111.

In some embodiments, wearable device 100 can also include an electrical connection between electronics module 32 and bio-signal sensor 20.

In some embodiments, electronics module 32 is curved to generally correspond to the user 10's head.

In some embodiments, flexible body 111 can include a compressible section adjacent to bio-signal sensor 20 to compress to conform the at least one bio-signal sensor 20 to user 10's body.

In some embodiments, the compressible section is shaped to conform to the at least part of the body of the user.

In some embodiments, the compressible section comprises foam having variable density.

In some embodiments, flexible body 111 comprises a reinforced section, such as interfacing, adjacent to bio-signal sensor 20.

In some embodiments, electronics module 32 can be permanently affixed to body 111. In some embodiments, electronics module 32 is detachable or otherwise removable from body 111. In some embodiments, electronics module 32 can be removed to permit the washing of body 111. In some embodiments, electronics module 32 can be swapped between bodies 111 and still offer the similar functionality. For example, a user can remove their electronics module 32 from one variant of body 111 and couple their electronics module 32 with another variant of body 111. The user may remove electronics module 32, to allow the sensing technologies within the module work on another part of the body, such as measuring blood flow at a fingertip, or tracking the movements of a hand, hip, chest or other body part.

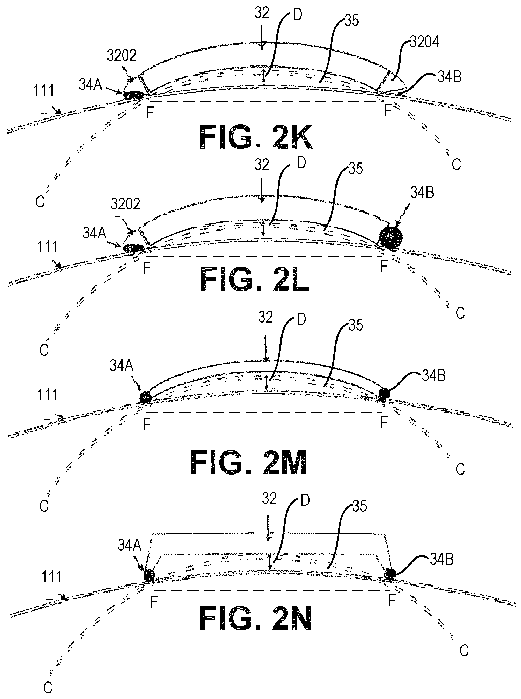

FIG. 2K illustrates one embodiment according to the invention of a bowstring design between electronics module 32 and body 111. Electronics module 32 can be mounted on retention mounts 3202 and 3204. Retention mounts 3202 and 3204 can be affixed to body 111 at connection points 34A and 34B respectively. Connection point 34A is configured to deform between body 111 and retention mount 3202 to accommodate the body shape of user 10. Connection point 34B and is configured to deform between body 111 and retention mount 3204 to accommodate the body shape of user 10. The flexibility of connection points 34A and 34B can allow the body 111 to rotate relative to electronics module 32 which can allow for a more comfortable fit. The flexibility of connection points 34A and 34B may have a flexibility that allows electronics module 32 to slide along the length of the body thereby increasing or decreasing the length of body 111 between points 34A and 34B.

FIG. 2L illustrates one embodiment not according to the invention of the bowstring design between electronics module 32 and body 111. In this embodiment, electronics module 32 can couple directly to body 111 at connection point 34B removing the need for retention mount 3204. Such asymmetric designs may be useful to fit certain body parts of user 10 or for certain designs of electronics module 32. In this embodiment, connection point 34B is configured to configured to deform between electronics module 32 and body 111 to accommodate a body of user 10. In analogous designs, retention mount 3202 can be removed instead of retention mount 3204. Such a design may have greater conformability or increased ease of use. Body parts that have high curvature such as the wrist or the ankle, or the head of a baby, may benefit from this increase conformability. Electronics module 32 would make an electrical connection to body 111 through connection point 34A and retention mount 3202 and it may be coupled at connection point 34B using a means that offers greater flexibility, such as a magnetic force, hook and loop such as Velcro^{™}, a fabric or elastomeric retention aspect that is part of body 111.

FIG. 2M illustrates one embodiment not according to the invention of the bowstring design between electronics module 32 and body 111. In this embodiment, electronics module 32 can couple directly to body 111 at connection points 34A and 34B removing the need for retention mounts 3202 and 3204. Such designs can be used to reduce the number of components making up wearable device 100.

FIG. 2N illustrates one embodiment not according to the invention of the bowstring design between electronics module 32 and body 111. FIG. 2N illustrates an alternative design for electronics module 32 than that of FIG. 2M. Electronics module 32 can have a more linear design that contains abrupt corners. Wearable device 100 can still accommodate curved body parts of user 10 in concave space 35.

In FIGS. 2K - 2N, connection points 34A and 34B are positioned distance F-F apart on body 111 such that when electronics module 32 is received by body 111 (either directly on connection point 34A or 34B, or through retention mount 3202 or 3204) connection points 34A and 34B generate tension in the portion of body 111 between connection points 34A and 34B (F-F). This tension can position body 111 adjacent to a user's body when worn if there is minimal body curvature (body 111 does not deform) and maintain concave space 35. This can enable sensors on body 111 between connection points 34A and 34B to maintain sufficient contact with the user to receive a signal. The tension will deform under a pressure, pushing into concave space 35 to a maximum distance of D and can accommodate curvature along line C-C. This can make the device more comfortable when worn on a body part with high curvature and still enable sensors on body 111 between connection points 34A and 34B to maintain sufficient contact with the user to receive a signal.

Contact with bio-signal sensors may be affected by barriers such as hair. Hair forms a physical barrier, lifting the bio-signal sensor away from the user's skin, especially if the hair is sufficiently dense that it forms a mat. As such, bio-signal sensors 20 may be placed on the device such that, when worn, the sensors are located on the head in an area with little hair. As such, in some embodiments, the at least one bio-signal sensor 20 is located on the forehead contacting portion 12, one or both of the two ear contacting portions 14, or any combination thereof. In some embodiments, the at least one bio-signal sensor 20 includes a bio-signal sensor located at each of the ear contacting portions. Bio-signal sensors 20 may be disposed in a fixed position on body 111. In some embodiments, bio-signal sensors 20 may be integrated into an aperture or track defined by body 111 that allows for lateral movement of bio-signal sensor 20 along body 111.

As seen in FIG. 2J, in particular, a bio-signal sensor 20 may be configured to contact at least part of the auricular region of the user's head, for example, an ear or a mastoid bone region of user 10. As user 10 is wearing wearable device 100, foam within wearable device 100 may allow a downward bend of body 111, and thus a bio-signal sensor 20, over the user's ear.

As such, a bio-signal sensor 20 may contact a top portion of an ear of user 10, and similarly configured on an opposing side. Conveniently, these sensors may have sufficient flex to be generally comfortable for a sleeping position, while the contacts maintain contact with the ear. The length of such a bio-signal sensor 20 may accommodate various sizes and shapes of ears, as well as movement of wearable device 100 with relation to a user's ear during movement or use.

Bio-signal sensor 20 may be an electrophysiological sensor of various types, including: electrical bio-signal sensor in electrical contact with the user's skin; capacitive bio-signal sensor in capacitive contact with the user's skin; blood flow sensor measuring properties of the user's blood flow.

The locations of bio-signal sensors 20 on body 111 may be reinforced, for example with reinforcing member 131, to reduce flexibility or elasticity and increase rigidity of locations on body 111 in which bio-signal sensors 20 are disposed. **In** some embodiments, body 111 locations may be reinforced by using interfacing to reduce stretch of fabric 121 of body 111.

In an example, forehead contacting portion 12 may be reinforced to structurally support bio-signal sensors 20, while ear contacting portions 14 of body 111 may remain elastic.

In some embodiments, bio-signal sensor 20 may be formed from material including silver-painted vinyl, flexible printed circuit board ("FPCB" or "PCB") with or without a conductive ink or precious metal plating such as silver or gold plating, conductive rubber such as heat-applied conductive rubber, conductive fabric (for example, silver ink on fabric, or conductive fibers that are woven in), a conductive fabric laminate, PEDOT-impregnated foam, and conductive carbon (for example, forming a conductive carbon layer). Other suitable conductive materials may also be used. In some embodiments, a conductive layer in the stack up that is separated with a dielectric can offer shielding to the signals from bio-signal sensor 20. For example, a TPU layer between a conductive polymer ink layer and a conductive fabric layer.

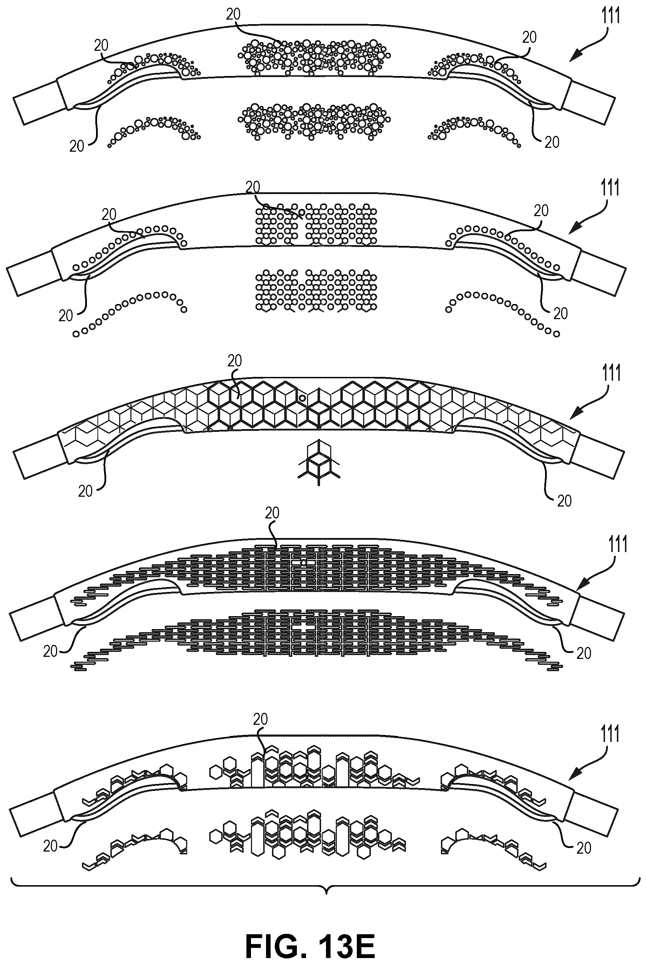

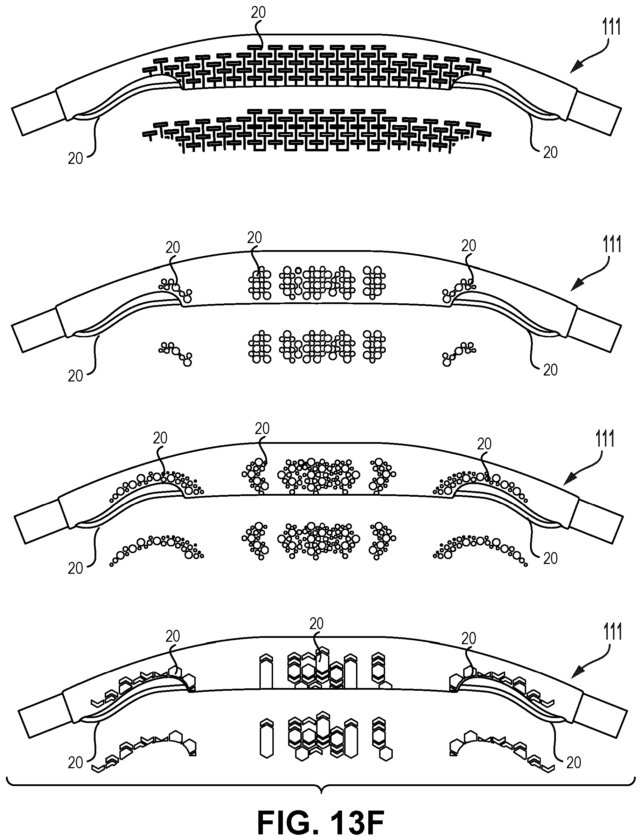

Bio-signal sensors 20 may be integrated into body 111 in a configuration so as to allow body 111 to flex and be breathable. For example, a vinyl or plastic substrate with silver ink on it may be cut into a pattern such as repeating shapes (for example, repeating squares or hexagons, and applied to body 111. FIGS. 13E-13F illustrate various configurations of bio-signal sensors on a body of a wearable device, such as using silver contacts and ear electrodes, as described in further detail below, according to various embodiments.

Body 111 may also be reinforced, and made less flexible and more rigid, in regions in which bio-signal sensors 20 are mounted, so that bio-signal sensors 20 may move around less, in use, for example, adjacent portions of a user's body such as a user's forehead or ear.

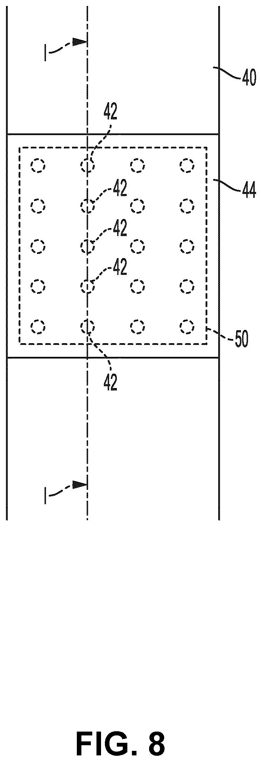

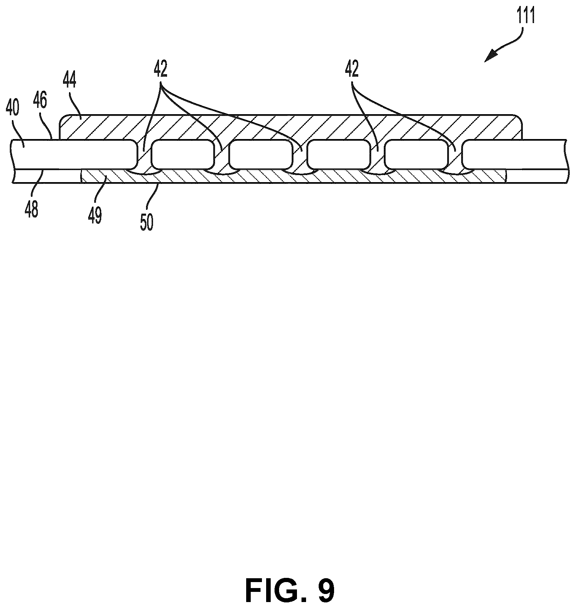

Having reference now to FIGS. 8 and 9, in some embodiments, body 111 or portions thereof include a substrate 40. FIG. 8 is a top schematic view of a bio-signal sensor integrated into a fabric substrate according to an embodiment. FIG. 9 is a cross-section schematic view of the bio-signal sensor integrated into the fabric substrate of FIG. 8 along lines I-I.

In some embodiments, substrate 40 is a woven or non-woven fabric substrate. In some embodiments, substrate 40 is an elastic material such as an elastic fabric. The elastic material may exhibit elastic deformation after being stretched to a length that is at least about 25%, 50%, 75%, 100%, 125% or 150% of the unstretched length. In an unstretched state, the loop may be slightly smaller than the circumference of the user's head. Once worn, the loop elongates to a stretched state around the user's head. In some embodiments, the loop is elongated from about 1% and about 50%, from about 5% to about 25%, or from about 5% to about 10% between the unstretched state and the stretched state. The tension exerted on the user's head and arising elastic forces due to the elongation of the loop tends to keep the device in place on the user's head.

A user may have an individual preference for levels of tension to keep the device in place on their head. As such, in some embodiments, the loop includes a tension adjuster. In some embodiments, the tension adjuster includes a buckle, for example, a sliding buckle near the back, a dial, hook and loop fasteners (such as Velcro^{™}), as described herein.

In some embodiments, a bio-signal sensor 20 is formed by applying a conductive layer 44 to the substrate 40. The conductive layer is applied to an inward face 46 of the substrate 40, the inward face 46 adapted to sit against the user's head when wearable device 10 is worn. In some embodiments, the conductive layer 44 is applied as a conductive ink. In some embodiments, the conductive ink includes silver, carbon, or combination thereof. In some embodiments, the conductive layer is applied by pad printing, silk screening, spraying, or painting. In some embodiments, a conductive layer is not applied directly to a material, and may instead be applied to a vinyl layer, which can then be applied to a fabric using heat and pressure, for example, ironed. The conductive layer 44, when in contact with the user's skin, is able to receive electrical bio-signals from the user at the point of contact.

In some embodiments, the substrate defines a plurality of apertures 42 such that upon application of the conductive ink to the inward face 46 of the substrate 40, the ink flows through and coats the apertures 42, eventually flowing to an outward face 48 of the substrate 40. The ink coating the aperture 42 acts as a through-substrate via, providing a path for signals collected at the interface between the user and the conductive layer 44 at the inward face to be transmitted and collected at the outward face 48.

At the outward face 48, a signal collector 50 is electrically connected to the conductive layer 44, providing electrical connection between the conductive layer 44 of the bio-signal sensor to the electronics module 32. The placement of elements not at the inward face 46 reduces the presence of potentially uncomfortable stress points pressing on the user's skin, when worn. In some embodiments, the signal collector 50 is connected to the conductive layer 44 by a bonding layer 49 such as an adhesive layer or second conductive ink layer. In some embodiments, the signal collector 50 attached to the substrate, such as by stitching or welded (such as by RF welding) onto the substrate 40.

In some embodiments, the signal collector 50 includes a flexible printed circuit board ("FPCB") or a film 50. In some embodiments, the FPCB includes a polyimide or similar film which is plated in copper and selectively removed (such as by etching) to create a circuit. The copper is optionally covered in another layer of polyimide or similar film or Liquid Solder mask. In some embodiments, the FPCB includes a plurality of copper layers. In some embodiments, the FPCB includes thicker polyimide or fiberglass or metal to provide stiffness to certain sections. In some embodiments, the film is a stretchable film, such as a thermoplastic elastomer, a thermoplastic urethane, or other plastic film. In some embodiments, the film may exhibit elastic deformation after having been stretched an elongation of at least 5%, 10%, 15%, 20%, 25%, 30%, 35%, 40%, 45%, or 50% as compared to its unstretched state.

In some embodiments, a covering layer is disposed over the substrate 40 and the signal collector 50. The covering layer may reduce protrusions that can catch on other surfaces, such as a pillow, helmet. In some embodiments, the covering layer is a fabric material, a rubber material, or any combination thereof.

As shown in FIGS. 10A to 10C, bio-signal sensor 20 may be formed from flexible printed circuit board ("FPCB") 1020. FPCB 1020 may have contacts 1030 on it formed from an appropriate conductive material such as silver ink. FPCB 1020 may be configured on body 111 of wearable device 100 such that in use, contacts 1030 contact at least part of the forehead of user 10. Such a FPCB may be hermetically sealed as long as the connections are appropriately sealed. PCBs and FPCBs as described herein, along with associated components, may be waterproofed by applying a coating, which may results in wearable device 100 and various components being washable.

In some embodiments, body 111 may be formed of an outer layer 1022 and an inner layer 1024. Each of outer layer 1022 and inner layer 1024 may be formed from materials such as fabric 121 and reinforcing members 131 as described herein.

FIG. 10A is a rear view of outer layer 1022 and inner layer 1024 of body 111. FIG. 10B is a perspective view of outer layer 1022 of body 111. FIG. 10C is a perspective view of inner layer 1024 of body 111. As shown in FIG. 10C, FPCB 1020 may fold over inner layer 1024.

Thus, as outer layer 1022 is affixed to inner layer 1024, the circuitry of FPCB 1020 may be sandwiched between outer layer 1022 and inner layer 1024, while contacts 1030 remain exposed on inner layer 1024 to contact the forehead of user 10.

The use of two layers, namely outer layer 1022 and inner layer 1024, for example, in the configuration described herein, may protect the edges of FPCB 1020 and protect circuitry of FPCB 1020 (as encased between outer layer 1022 and inner layer 1024) and may provide reduced visible seams.

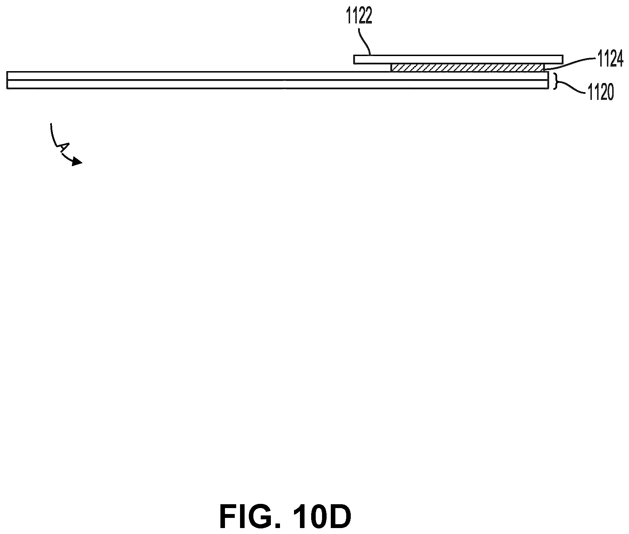

FIG. 10D is a side view of a flexible printed circuit board configuration, according to an embodiment, which may be used as a bio-signal sensor 20 in wearable device 100. A flexible printed circuit board ("FPCB") 1120 may be formed of copper and polyimide ("PI") arranged in PI-copper-PI layers.

A PI coverlay 1122 may be attached to FPCB 1120 by way of adhesive layer 1124. In use, PI coverlay 1122 may be disposed in wearable device 100 to contact, for example, a forehead of user 10. FPCB 1120 may thus be able to fold over in the direction shown by arrow A.

The configuration illustrated in FIG. 10D may allow for reduction of sharp edges and exposure of surfaces where tears may start in FPCB 1120.

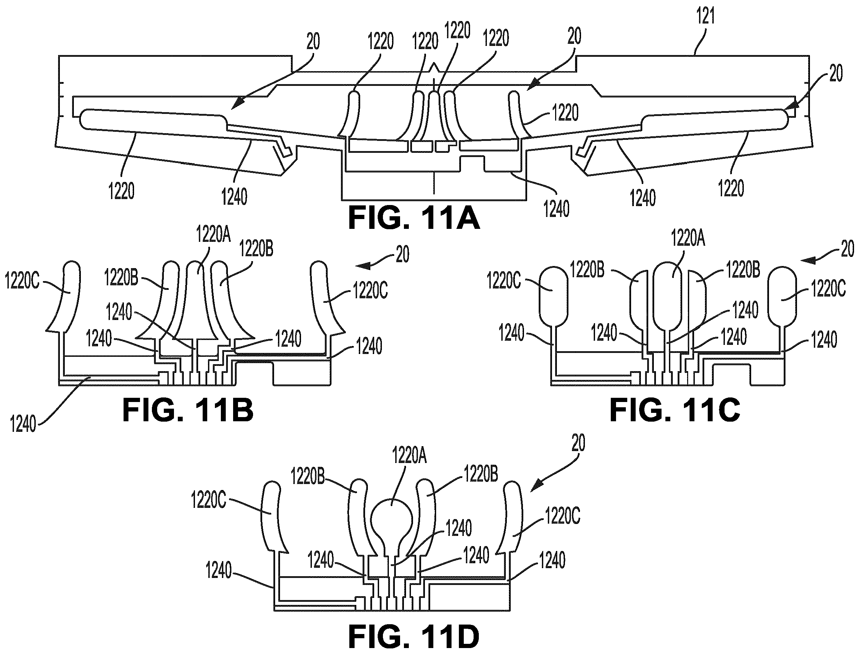

FIG. 11A illustrates an embodiment of a layer of fabric 121 upon which contacts 1220 of bio-signal sensors 20 are formed, for example, from one or more silver electrodes formed of silver paint (soft silver or silver chloride) on a thermoplastic polyurethane (TPU). Contacts 1220 may be connected (for example, riveted) to an FPCB by way of leads 1240, in an example, folded behind fabric 121, and can fold under or fold over between layers of fabric and/or foam forming wearable device 100.

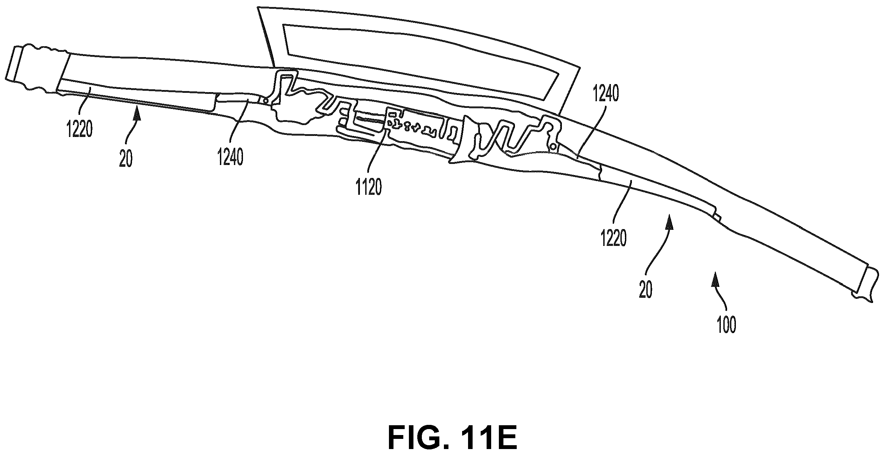

FIG. 11E illustrates bio-signal sensor contacts 1220 and leads 1240, as connected to a flexible printed circuit board 1120 in wearable device 100, according to an embodiment.

In some embodiments, bio-signal sensors 20 may be formed by a silver paste on a thermoplastic polyurethane (TPU), forming contacts such as contacts 1220. In some embodiments, a silver ink such as a DuPont^{™} textile ink, silver fabric or silver thread may be used to form contacts 1220. Silver contacts may be exposed where in contact with a user's body, for example, for sensing EEG.

In some embodiments, contacts 1220 as illustrated in FIG. 11A widen at a bottom portion of wearable device 100. When worn on a user's forehead, a position lower on a user's forehead may be less likely to be inhibited by hair, and thus a larger surface area of contacts 1220 is present, to increase surface area contact with a user's forehead and which may improve signal quality and reliability of EEG signals received by bio-signal sensors 20. Contacts 1220 can be positioned below the top of wearable device 100 and can extend to the bottom of wearable device 100. Hair can get trapped between the user and wearable device 100 so contacts 1220 can be positioned below the top of wearable device 100, where hair might likely interfere with contact 1220. Hair can be less likely to inhibit signals towards the bottom of wearable device 1220 and as such contacts 1220 can be configured to extend to the bottom of wearable device 100 and can receive bio-signals without being inhibited by hair.

FIG. 11B illustrates a zoomed-in view of the central contacts in FIG. 11A. The middle contact 1220A (a subset of contact 1220) has a symmetric design that is positioned below the top of wearable device 100 and widens to the bottom of wearable device 100. Peripheral contacts 1220B (a subset of contact 1220) are positioned adjacent to either side of middle contact 1220A. Peripheral contacts 1220B have a design that is positioned below the top of wearable device 100 and widens to the bottom of wearable device 100. Peripheral contacts 1220B also taper away from the middle contact 1220A at the bottom of their design. Distant contacts 1220C (a subset of contact 1220) have similar profiles to that of peripheral contacts 1220B, but are positioned on either side of middle contact 1220A at a distance away from peripheral contacts 1220B.

FIG. 11C illustrates an example alternative to the contact designs for the central contacts illustrated in FIG. 11B. The middle contact 1220A (a subset of contact 1220) has a symmetric design that is positioned below the top and above the bottom of wearable device 100 and has an elliptical shape. Peripheral contacts 1220B (a subset of contact 1220) are positioned adjacent to either side of middle contact 1220A. The shape of peripheral contacts 1220B matches that of middle contact 1220A except that it is shorter than middle contact 1220A, positioned further below the top of wearable device 100 than that of middle contact 1220A, and their shapes are cut laterally on the side of peripheral contact 1220B that is adjacent to middle contact 1220A. Distant contacts 1220C (a subset of contact 1220) have similar profiles to that of middle contact 1220A, except that they are shorter than middle contact 1220A and positioned further below the top of wearable device 100 than is middle contact 1220A. Distant contacts 1220C are positioned on either side of middle contact 1220A and at a distance away from peripheral contacts 1220B.

FIG. 11D illustrates an example alternative to the contact designs for the central contacts illustrated in FIG. 11B. The middle contact 1220A (a subset of contact 1220) has a generally circular shape and is positioned in the approximate center of wearable device 100. Peripheral contacts 1220B (a subset of contact 1220) are positioned adjacent to either side of middle contact 1220A. The shape of peripheral contacts 1220B have a narrower width at the top that widens slightly towards the bottom and their shape tapers around middle contact 1220A. Distant contacts 1220C (a subset of contact 1220) have similar profiles to that of peripheral contacts 1220B. Distant contacts 1220C are positioned on either side of middle contact 1220A and at a distance away from peripheral contacts 1220B.

As illustrated in FIG. 11E, contacts 1220 may be covered in carbon where not intended to be exposed, such as disposed within body 111 or between layers of wearable device 100, for example, forming leads 1240. In some embodiments, leads 1240 are reinforced by carbon, which may provide additional or redundant conductive properties. Carbon may allow for some conductivity and connectivity, which can help in the event that the silver substrate's connection becomes compromised or slightly compromised.

Signals may be carried by way of such leads to a rivet that connects the signal to a flexible PCB, such as flexible printed circuit board ("FPCB") 1120 formed of copper and polyimide ("PI") arranged in PI-copper-PI layers, within wearable device 100. The FPCB may then carry the signal to a connection in fabric 121 and connects it to electronics module 32.

In some embodiments, an FPCB may be riveted to a layer of fabric and the soft silver/silver chloride TPU contacts. Conveniently, riveting may enable mass production of wearable device 100, for example, at the factory level.

In some embodiments, layers such as FPCB and the contacts may be stitched together.

Additional configurations of contacts 1220 and leads 1240 of bio-signal sensors 20 are illustrated in FIGS. 11B-11D. Positioning of contacts 1220 may be selected such that the contacts are not in such close proximity to interfere with each other, for example, their signals, and receive discrete and useful bio-signal data.

Other suitable sensor configurations are also contemplated.

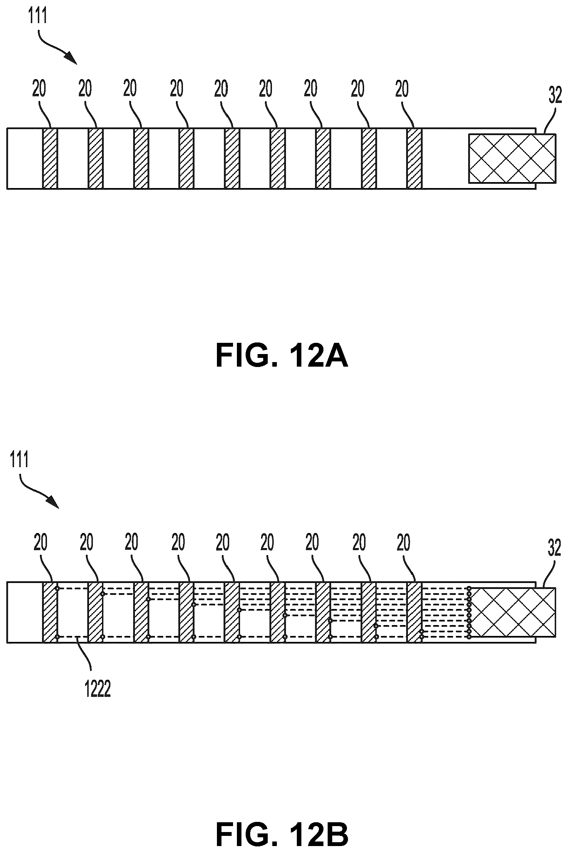

As shown in FIGS. 12A and 12B, multiple bio-signal sensors 20, such as electrodes, may be disposed in body 111 of wearable device 100.

FIGS. 12A and 12B illustrate an embodiment of wearable device 100 in which there is a redundant array of bio-signal sensor 20 electrodes connected to a single electronics module 32 by traces 1222. In an example, traces 1222 may be conductive thread. Electronics module 32 may use a signal quality indicator to assess which bio-signal sensor(s) 20 to use. For example, depending on where the received bio-signal is cleanest or strongest.

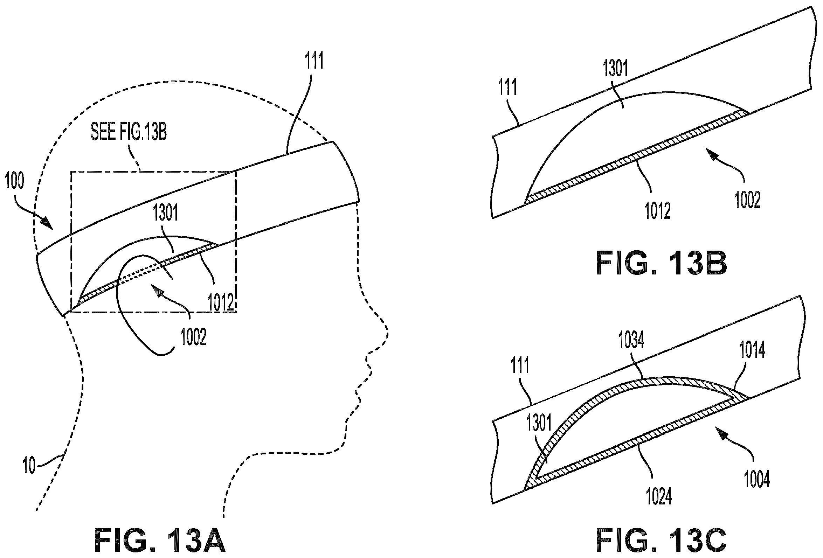

A bio-signal sensor 20 located and connected to an ear contacting portion 14 of wearable device 100 may be referred to as an "ear electrode", as described herein. It will be appreciated that such electrodes may also be used to detect signals above, on or below the ear of a user. Such an "ear electrode" may be a deformable earpiece that takes the form of an open bow-string ear electrode 1002, a closed bow-string ear electrode 1004, a shaped ear electrode 1102, and a moveable ear electrode 1202 as described below with reference to FIGS. 13A-13C, 14 and 15A-15B. Such ear electrodes may be configured to contact at least part of the auricular region of the user's head, for example, an ear or a mastoid bone region of user 10. The ear electrode may include a depressible area with a thin rubber cushion, air cushion, or gel cushion that can depress against an ear.

FIG. 13A illustrates a schematic side view of an embodiment of wearable device 100 having an ear electrode 1002 with an open 'bow string' design and an ear electrode 1004 having a closed 'bow string' design, and FIG. 13B is an expanded view thereof. FIG. 13C illustrates a schematic side view of an embodiment of wearable device 100 having an ear electrode 1004 with a closed 'bow string' design.

Ear electrode 1002 may include a strip of flexible conductive material 1012, connected at each end to body 111 of wearable device 100. Body 111 of wearable device 100 may have an area cut out above ear electrode 1002 which may allow conductive material 1012 to move freely. When wearable device 100 is placed on the head of user 10, downward pressure may be distributed along the length of conductive material 1012, which may increase the contact area and signal quality, and may provide a comfortable fit for user 10.

Variations of shape of the conductive material 1012 that contacts the user's ear are possible. For example, as shown in FIG. 13A, body 111 may define an aperture, illustrated as an inner region 1301 of body 111, and may be open and may be semicircular in shape, allowing conductive material 1012 to collapse toward body 111 when worn. Conductive material 1012 may be curved to form to user's 10 ear. Comfortable conductive rubber ear contacts may be provided to provide fit to keep the wearable device 100 on the head, comfort, as well as contact for conductivity from conductive material 1012. The conductive rubber 1012 may rest on top of the user's ear (for example, in the region between the top ear tip and the head). The ear of user 10 generally does not extend through inner region 1301, but body 111 would be between the ear and the head, with the ear sitting outside of inner region 1301 during regular wear.

Conductive material 1012 may be, for example, conductive rubber. In an example, conductive material 1012 may be formed from silicon rubber infused and/or coated with carbon. In some embodiments, conductive material 1014 may be formed from thermoplastic elastomer infused with carbon, and coated with a PEDOT conductive polymer layer. Other suitable conductive materials may also be used.

Similarly, ear electrode 1004, as shown in FIG. 13C, may include flexible conductive material 1014 (for example, formed from conductive rubber) and may be shaped with a bottom portion 1024 and a top portion 1034 to define an open aperture with a perimeter generally semicircular in shape, allowing bottom portion 1024 to collapse towards top portion 1034 and body 111. Conductive material 1014 may be curved on a bottom portion 1024 to form to user's 10 ear. Comfortable conductive rubber ear contacts may be provided to provide fit to keep the headband or wearable apparatus on the head, comfort, as well as contact for conductivity from conductive material 1014.

In use, upper portion 1034 may contact the top of an ear of user 10, for example, as wearable device 100 shifts during sleep. The conductive rubber 1014 may rest on top of the user's ear (for example, in the region between the top ear tip and the head). The ear of user 10 generally does not extend through inner region 1301, but body 111 would be between the ear and the head, with the ear sitting outside of inner region 1301 during regular wear, and bottom portion 1024 and top portion 1034 in contact with the region in and around the top ear tip and the head of user 10.

Conductive material 1014 may be formed of the same or similar materials to that of conductive material 1012.

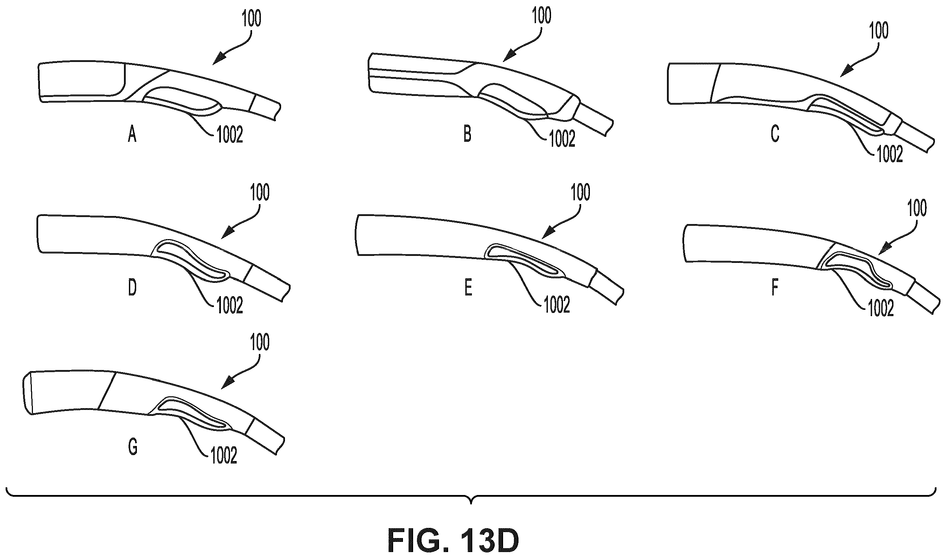

FIG. 13D illustrates various configurations of wearable device 100 having an ear electrode 1002, according to various embodiments.

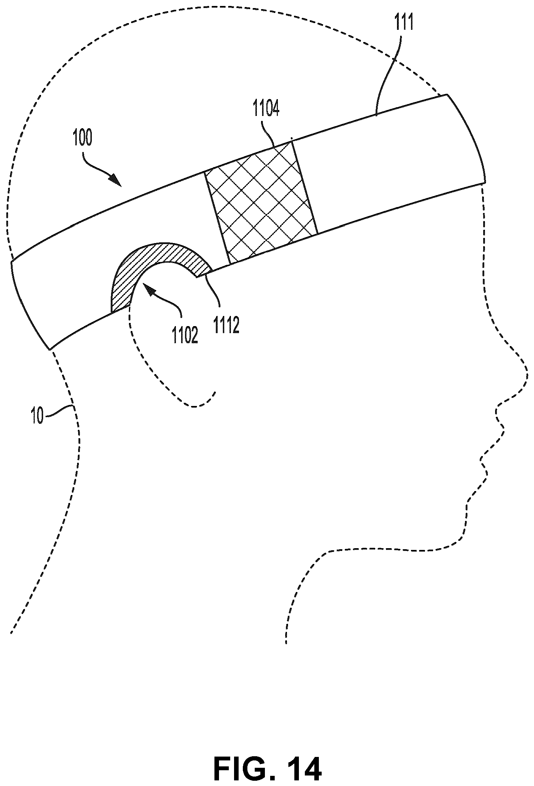

FIG. 14 illustrates a side schematic view of an embodiment of wearable device 100 having an ear electrode 1102 shaped to contact the upper and rear surface of an ear of user 10.

Shaped ear electrode 1102 may comprise a strip of flexible conductive material 1112 (e.g. rubber), connected to body 111 of wearable device 100. The electrode material 1112 may be shaped to contour around the upper and rear surface of the ear which may increase the skin contact area and aid in fitting.

Conductive material 1112 may be formed of the same or similar materials to that of conductive material 1012.

A stretchable or elastic portion 1104 of body 111 of wearable device 100 may allow fitting a multitude of head sizes while maintaining proper positioning of the electrodes above and behind the ears.

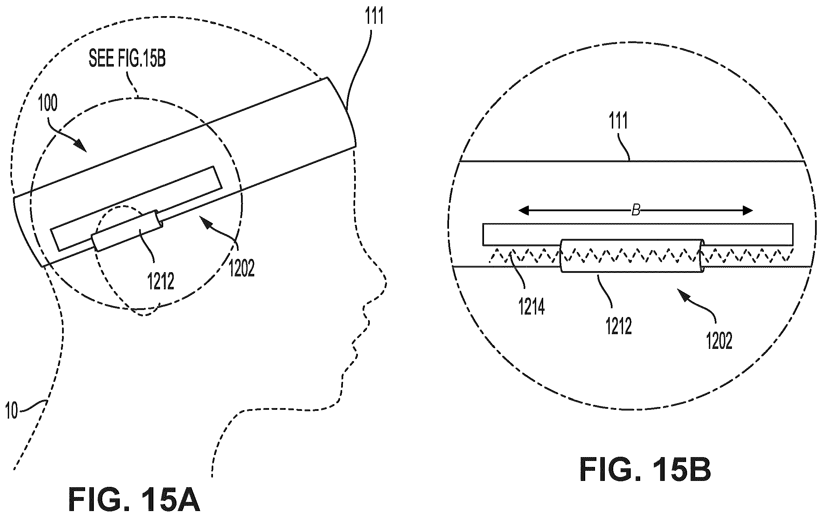

FIG. 15A illustrates a side schematic view of an embodiment of wearable device 100 having an ear electrode 1202 which may be movable to provide for skin contact on a variety of head shapes, and FIG. 15B is an expanded view thereof.

FIGS. 15A and 15B illustrate movable ear electrode 1202, with a conductive tube 1212 contacting an exposed conductive wire or thread 1214. The conductive tube 1212 contacts the wire 1214, independent of where it is placed along the open area. The wire 1214 then conveys the sensed bio-signals to the electronics module 32.

Conductive tube 1212 may be hollow and generally cylindrical in shape, or other suitable shape to allow movement in direction illustrated by arrow B.

Conductive tube 1212 may be formed of the same or similar materials to that of conductive material 1012.

Due to signal quality requirements, it may be desirable to place electrode(s) in areas where there is little hair, such as above or behind the ear. Because the bitragion frontal arc (distance between the ears, across the forehead) varies considerably between individuals, portions of wearable device 100 may be required to stretch or extend (for example, as shown in FIG. 14, where 1104 illustrates an extendable portion in an otherwise non-stretchable device), or alternatively, movable electrodes such as movable ear electrode 1202, may provide for placement of the electrode in contact with an ear of user 10.

In some embodiments, bio-signal sensors 20 may be integrated in body 111 at ear contacting portion 14, to cover an ear of user 10, for example, with a generally rectangular or generally circular contact or conductive sensor formed from silver ink or other material incorporated with body 111.

It will be appreciated that in various implementations, the shape and configuration of ear electrode 1002, 1004, shaped ear electrode 1102, and movable ear electrode 1202 may be coordinated with the shape and configuration of body 111 to complement each other. For example, body 111 may be configured to stretch in forehead contacting portion 12 and adjacent to ear contacting portion 14 for use with shaped ear electrode 1102 so as to allow more variance in body 111 while the ear electrode remains more fitted or is less deformable. This may allow for better conformance to a head of user 10. Similarly, a longer ear electrode, such as ear electrode 1004, may be used with a less flexible body 111, as a longer ear electrode may accommodate different ear sizes.

Some embodiments can reversibly couple the attachment portions of headphones (e.g., headphone wires) to wearable device 100. The reversible coupling can be used to loosely affix external headphones to an area proximate to the ears of a user. Coupling can be achieved in many ways such as with Velcro attachments or a clasp. This can militate against external headphone migration during sleep.

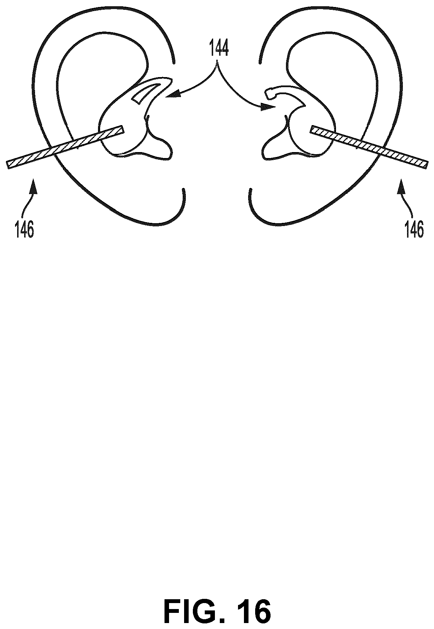

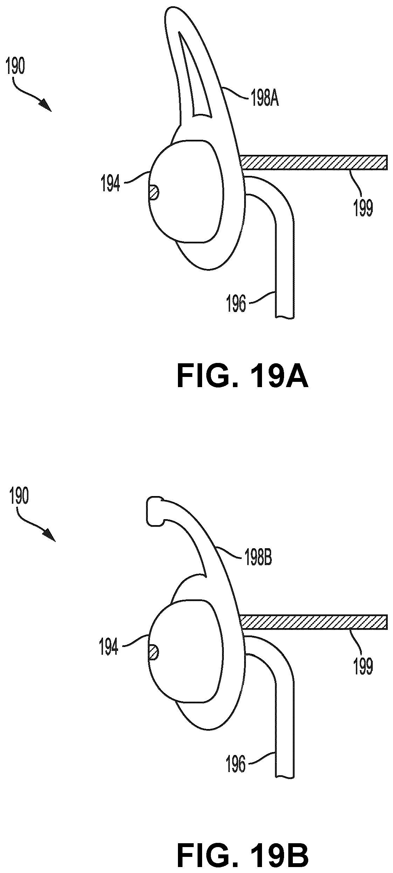

Having reference to FIGS. 16 to 19A and 19B, in some embodiments, the wearable device 100 includes an inner earpiece 144 having a conductive sensor.

FIG. 13A, 13B, 13C, 13D, 14, 15A, and 15B illustrate that in some embodiments, wearable device 100 can also include additional bio-signal sensor 20, 1002, 1004, 1102, or 1202 to contact at least part of an auricular region of a user 10's head. In some embodiments, additional bio-signal sensor 20, 1002, 1004, 1102, or 1202 is an electroencephalography (EEG) sensor.

As shown in FIG. 16, inner earpiece 144 may be shaped to form to the auricle of an ear of user 10. Inner earpiece 144 may be a conductive sensor and connect to signal transmission line 146. In some embodiments, inner earpiece 144 may have conductive material similar or the same to conductive material 1012 described herein. Inner earpiece 144 may thus form an inner-ear sensor to contact an ear canal, for example, outer ear canal, of user 10.

Signal transmission line 146 may be a wire or other similar conductive material, and connect to electronics module 32.

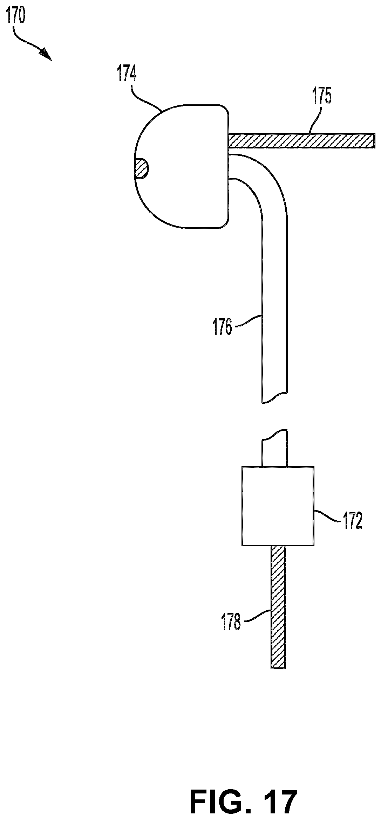

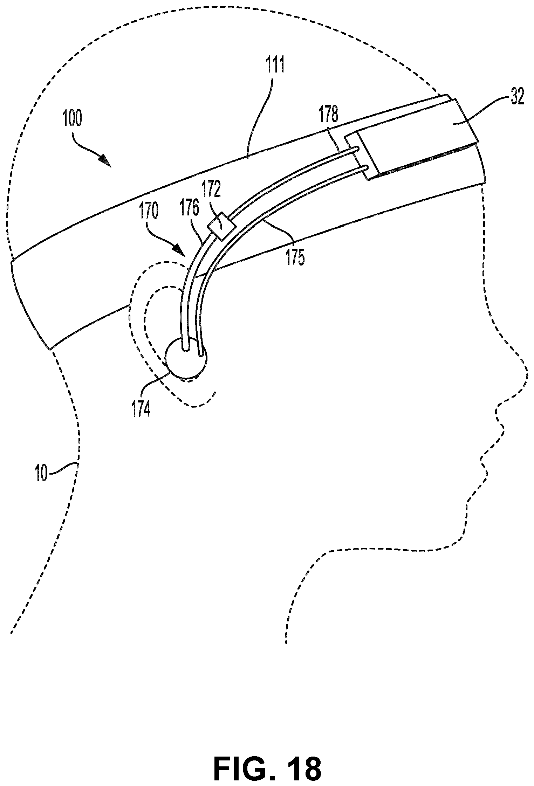

As shown in FIGS. 17 and 18, the wearable device 100 may further include a sound delivery module 170 including a sound generator 172 connected to an inner earpiece 174 by way of a hollow tube 176. Sound generator 172 receives an audio signal from audio transmission line 178.

As shown in FIG. 17, inner earpiece 174 may be shaped to form to the auricle of an ear of user 10. In some embodiments, inner earpiece 174 may be molded, for example, heat-molded to the shape of a specific ear of user 10. Inner earpiece 174 may be a conductive sensor and connects to signal transmission line 175. In some embodiments, inner earpiece 174 may have conductive material similar or the same to conductive material 1012 described herein. In some embodiments, inner earpiece 174 may be insulative, and signal transmission line may be disposed through inner earpiece 174 to provide a conductive surface to contact an ear of user 10.

Sound generator 172 may be located at a distance, for example between 2 cm and 30 cm, from inner earpiece 174. Sound generator 172 may be a speaker or a driver to generate sound waves for travel to inner earpiece 174 by way of hollow tube 176.

In an example, hollow tube 176 is a hollow plastic tube, for example, 2-3 mm in diameter. Hollow tube 176 may be generally rigid, so as to not fold over in a manner that would interfere with the travelling sound waves.

Conveniently, distancing the sound generator from sensing (for e.g., of bio-signals of user 10 from the conductive sensor portions of inner earpiece 174) may allow for sound to be generated with less interference with sensor readings.

FIG. 18 is a schematic view of sound delivery module 170 connected to electronics module 32 of wearable device 100, in which signal transmission line 175 and audio transmission line 178 are connected to electronics module 32.

FIGS. 19A and 19B illustrate a sound delivery module 190 including a sound generator (not shown, for example, sound generator 172) connected to an inner earpiece 194 by way of a hollow tube 196.

As shown in FIGS. 19A, 19B, inner earpiece 194 may be shaped to form to the auricle of an ear of user 10. Inner earpiece 194 may be insulative and may not be a conductive sensor.

Inner earpiece 194 may be backed by a conductive sensor such as a closed loop frame 198A, or an open loop frame 198B. Closed loop frame 198A, and similarly open loop frame 198B, may be shaped to form to the auricle of an ear of user 10.

Closed loop frame 198A connects to signal transmission line 199, which may connect to electronics module 32 to transmit bio-signals from the conductive sensor.

Conveniently use of a conductive sensor away from an inner ear canal of user 10, for example, as with closed loop frame 198A or open loop frame 198B, may avoid a build-up of ear wax, which may act as an insulator and reduce signal quality, on the conductive sensor. Such a conductive sensor may also allow for a larger surface area for the sensor to contact the user, beyond the inner ear canal.

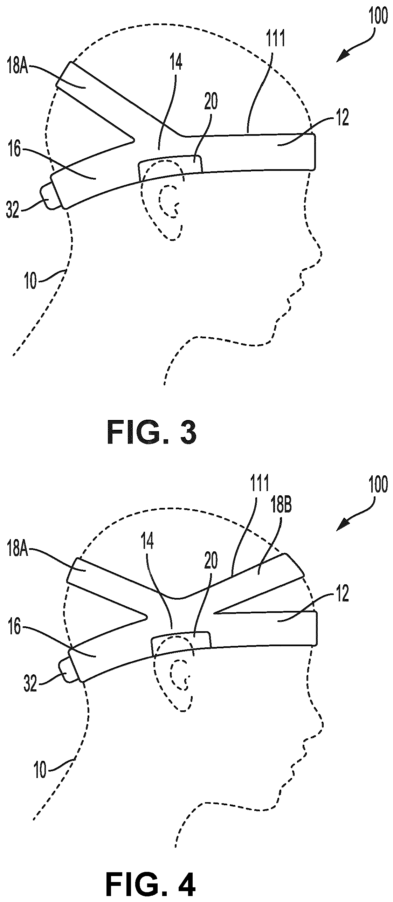

As shown in FIGS. 3 to 5, in some embodiments, body 111 of wearable device 100 may include at least one overhead support strap. The at least one support strap may provide additional support above the head and distributes forces on the head over a greater area. In some embodiments, the overhead support straps are placed in a front-to-back orientation, a side-to-side orientation, or a diagonal orientation. In some embodiments, the overhead support straps are placed in a side-to-side orientation. In some embodiments, the at least one overhead support strap are joined to the loop at the ear contacting portions 14. In some embodiments, the at least one overhead support strap includes a crown strap 18A, a top strap 18B or a combination thereof. The side-to-side orientation provides forces that may be partially opposed by a strap elsewhere in the device. For example, at least some of the forces acting on the user's head from the top strap 18B may be opposed by the occipital contacting portion 16 of the loop. Similarly, at least some of the forces acting on the user's head from the crown strap 18A is opposed by the forehead contacting portion 12 of the loop. In contrast, for a front-to-back strap to have an opposing force, the device may require a chin strap or other strap exerting forces on a lower surface of the skill.

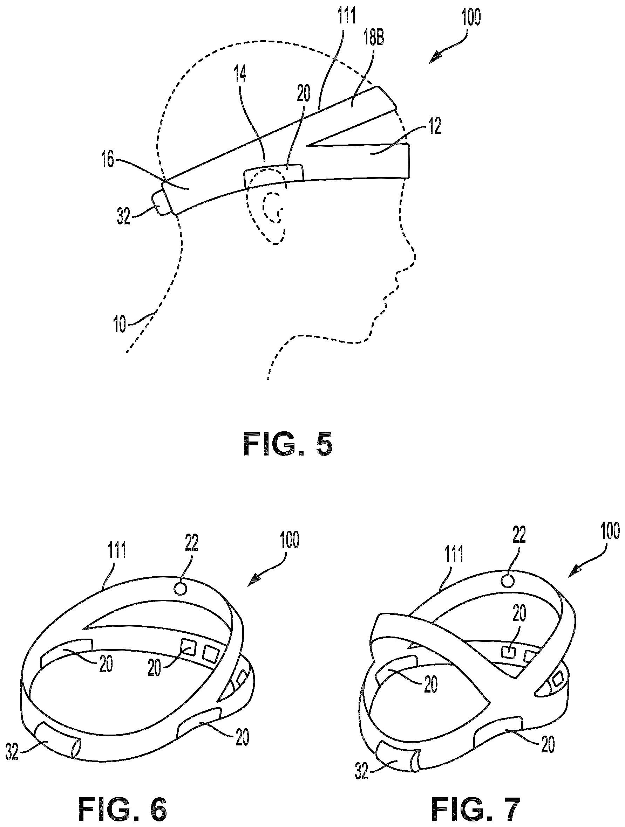

Bio-signal sensors located where there is hair may be selected for their ability to obtain a signal despite an impedance that may be created by presence of hair. With reference to FIGS. 6 and 7, in some embodiments, wearable device 100 includes bio-signal sensors 20 such as at least one hair-penetrating bio-signal sensor 22 located on the at least one support strap, such as crown strap 18A, top strap 18B, the occipital contacting portion 16, or both. In some embodiments, hair-penetrating bio-signal sensor 22 may be disposed at other locations on body 111, including forehead contacting portion 12 and ear contacting portions 14. Hair-penetrating bio-signal sensor 22 may be a pin sensor, or a sensor with prongs (similar for example to prongs 3536 discussed in further detail below) to extend through a user's hair to contact skin 11.

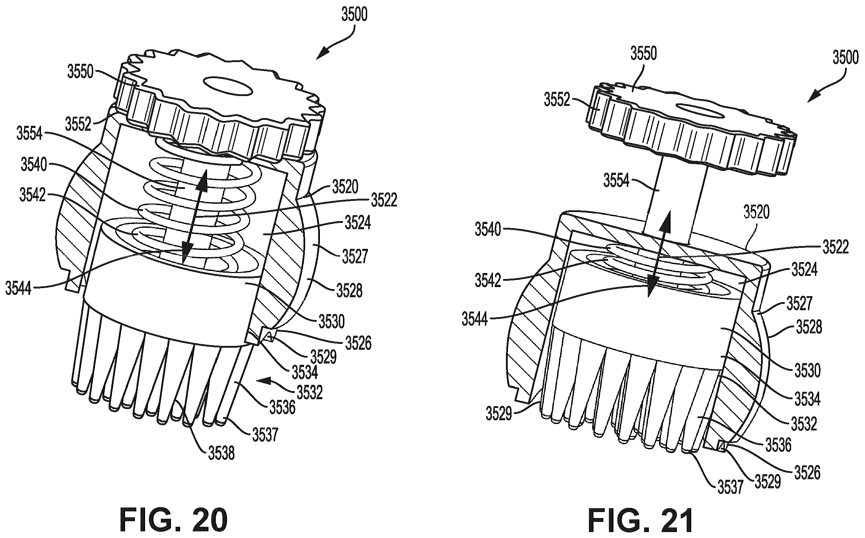

Example embodiments of a hair-penetrating bio-signal sensor 22 are described below with reference to FIGS. 20 to 27. A hair-penetrating bio-signal sensor 22 may be integrated in a section of body 111 of wearable device 100 that is rigid or reinforced, for example, with reinforcing member 131. Hair penetrating bio-signal sensor 22 may also be integrated into an aperture or track defined by body 111 that allows for lateral movement of hair penetrating bio-signal sensor along body 111. Hair penetrating bio-signal sensor 22 may thus be affixed in a position, for example, by way of a corresponding threads on hair penetrating bio-signal sensor 22 rotated to capture part of body 111 and provide a friction fit.

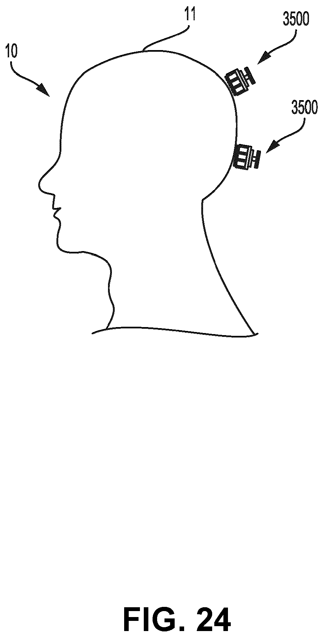

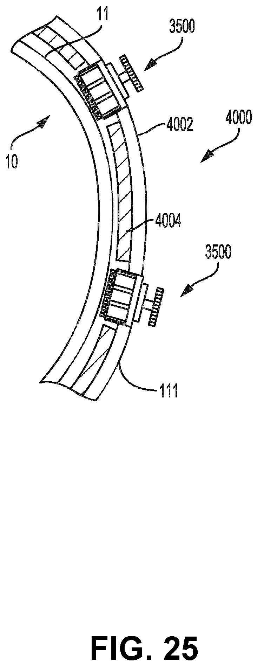

In accordance with an aspect of the embodiments described herein, body 111 may include sensors such as bio-signal sensors 3500 for obtaining bio-signals from the scalp or skin 11 of user 10. With reference to FIG. 24, there is provided a bio-signal sensor 3500. The sensor 3500 is configured to receive a bio-signal from a user 10, preferably, from the user's head or through the skin 11 of user 10. With reference to FIG. 25, the bio-signal sensor 3500 can be included on an apparatus 4000, for example on a support portion 4002 such as body 111 of wearable device 100. The apparatus 4000 optionally includes at least one deformable portion 4004, for example, made from foam, connected to the support portion 4002 to provide comfort and/or support when the apparatus 4000 is worn by the user 10.

With reference to FIGS. 20 and 21, the bio-signal sensor 3500 includes a body 3520, having a spherical portion 3528; an electrode 3530 extendable into the body 3520, the electrode 3530 having a contact end 3532 configured to receive an electrical bio-signal from a user's 10 skin 11, wherein in response to a downward force acting on the bio-signal sensor 3500 to urge the bio-signal sensor 3500 against the user's skin 11 and upon contact with the skin 11 of user 10, the electrode 3530 is configured for movement into the body 3520 along a movement axis 3522; an actuator 3540 operatively connected to the electrode 3530 for urging the electrode 3530 out of the body 3520 along the movement axis 3522 toward an extended position, wherein in the absence of the downward force, the electrode 3530 is disposed in the extended position; and a contact adjuster 3550 connected to the electrode 3530, the contact adjuster 3550 includes a handle 3552 manipulatable by the user to reduce noise the electrical bio-signal caused by impedance of the user's hair.

In use, a force having a downward component is applied to urge the bio-signal sensor 3500 against the skin 11 of user 10 to receive an electrical signal from the user 10. The electrode 3530 moves along the movement axis 3522 into an electrode receiving space 3524 of body 3520 from an extended position toward a retracted position (see, for example, FIG. 21). However, the user's hair may impede the ability of the bio-signal sensor 3500 to receive an electrical signal from the skin 11 of user 10. For example, the user's hair may form a barrier (or "mat") that acts as an insulation layer between the contact end and the user's skin. The insulation layer impedes or prevents the receiving of the electrical signal. As such, in some embodiments, the bio-signal sensor 3500 is configured to reduce the impedance effects of the user's hair.

In some embodiments, the contact end 3532 of the electrode 3530 includes a collection plate 3534 and a plurality of prongs 3536 extending from the collection plate 3534. Each prong includes a distal tip 3537 for contacting the skin 11 of user 10. Whereas with an electrode having a single contact surface, the user's hair may form a mat under the single contact surface, an interstitial volume 3538 defined by the prongs 3536, the collection plate 3534, and the skin 11 of user 10 may receive the user's hair and reduce or prevent the formation of a mat under the distal tips 3537 of the prongs. In some embodiments, the extension of the electrode 3530 from the body 3520 in the extended position is adjustable using the contact adjuster 3550. In some embodiments, contact adjuster 3550 includes a compression fitting, or threading that mates with the electrode or the body for adjusting the extension of the electrode 3530 in the extended position. The extension of the electrode 3530 from the body 3520 accommodates users with different volumes of hair. For example, a user with thick, long hair, may have a relatively greater volume of hair, which may create an electrical barrier if a mat is formed. For such users, the extended position may be adjusted such that the electrode 3530 extends further from the body 3520 than for users with shorter or no hair.

In some embodiments, the contact adjuster 3550 is configured to move the electrode along the movement axis 3522. In some embodiments, the handle is configured for lifting the electrode 3530 when urged against the skin 11 of user 10 and repositioning the electrode for placement against the skin 11 of user 10. In some embodiments, the movement of the contact adjuster 3550 moves the plurality of the prongs 3536 collectively. For example, in some embodiments, the contact adjuster 3550 is connected to the collection plate 3534 and is configured to move the collection plate. The movement of the collection plate 3534 causes the plurality of prongs 3536, which extend from the collection plate 3534, to move.

On the application of a downward force, the electrode 3530 moves along the movement axis 3522 into the body 3520 (see FIG. 21). Where there is significant retraction of the electrode 3530 into the body 3520, the body 3520 may become proximal to the skin 11 of user 10. This may cause, for instance, the user's hair disposed under the body 3520 of the sensor 3500 may form a barrier layer preventing good contact between the electrode 3530 and the skin 11 of user 10. Thus, in some embodiments, the body 3520 includes a contact end 3526 including at least one groove 3529 for receiving at least a portion of the user's hair therein.

In order to provide better comfort for a user, the pressure of the electrode 3530 against the skin 11 of user 10 may not be excessive. In some embodiments, the distal tips 3537 of the plurality of prongs 3536 are rounded. In contrast to a pointed tip, a rounded tip distributes the force applied to the skin over a greater area. In some embodiments, the radius of the distal tip is between about 0.25 mm and about 1 mm. In some embodiments, the radius of the distal tip is about 0.5 mm. The number and spacing of the prongs 3536 are selected such that the pressure applied to the skin 11 of user 10 is not excessive and has sufficient contact area to receive good adequate signal from the user's skin while maintaining sufficient void volume between prongs 3536 to receive the user's hair. In some embodiments, the electrode 3530 has a prong density of about 15 to 40 prongs per square centimeter. In some embodiments, the electrode 3530 has a prong density of about 25 prongs or pins per square centimeter.

A greater area of the contact end of the electrode 3530 may provide better electrical readings. However, when the area is too large, it may not conform well to the skin. One reason for this is that the skin is, typically, not perfectly flat. Increased area of the contact end of the electrode also increases the likelihood that the skin's curvature bends away, resulting in a loss of contact for the electrode. Thus, in some embodiments, the area of the contact end of the electrode 3530 comprising the prongs 3536, including the interstitial area between prongs, is between about 1 cm² and about 3 cm². In some embodiments, the area of the contact end of the electrode 3530 comprising the prongs, including the interstitial space between prongs, is about 1.5 cm². In some embodiments, the shape of the contact end 3532 of the electrode is round or polyhedral. The shape of the contact end 3532 may help move the user's hair to reduce or prevent the impedance effects of the user's hair.

In some embodiments, the contact adjuster 3550 is configured to rotate the electrode along a plane that is substantially perpendicular to the movement axis. The rotational movement may move the hair disposed under the sensor 3500. In some embodiments where the sensor includes a plurality of prongs 3536, the rotational movement may move the hair into the interstitial volume 3538. In some embodiments, the rotational movement of the contact adjuster 3550 is unrestricted. In some embodiments, the rotational movement of the contact adjuster 3550 is limited.

In some embodiments, the actuator 3540 includes a spring, a piston, a compressible material, or combination thereof. In some embodiments, the actuator 3540 includes a spring 3542. In some embodiments, the spring 3542 is a coil spring. The spring 3542 is disposed within the electrode receiving space 3524 such that one end is biased against an upper end 3526 of the body against the electrode 3530 such that the electrode 3530 is urged away from the electrode receiving space 3524 toward the extended position. In some embodiments, the spring 3542 biases against an upper end of the collection plate 3532 of the electrode 3530. When a downward force is applied to the sensor 3500 and when the electrode 3530 is against the skin 11 of user 10, the spring 3542 resists the movement of the electrode 3530 into the body 3520 such that a force is translated to the electrode 3530 urging it against the skin 11 of user 10.

In some embodiments, the spring 3542 is fixed on one end to the body 3520 and biased against the electrode 3530 on the other end, and wherein the contact adjuster 3550 includes a shaft 3554 extending through a compressive axis 3544 of the spring 3542 for translating rotational forces perpendicular to the movement direction from the handle 3552 to the electrode 3530, translational forces along the movement direction from the handle to the electrode, for both. In some embodiments, the compressive axis is co-axial or substantially co-axial with the movement axis 3522. In some embodiments where the spring 3542 is a coil spring, the coils of the coil spring are coiled around the shaft 3554 of the contact adjuster 3550.

In some embodiments, the actuator 3540 includes a plurality of actuators (not shown) corresponding to the plurality of prongs 3536. In some embodiments, the plurality of actuators individually bias the prongs against the skin 11 of user 10. This may allow, for instance, better conformity of the sensor against the skin 11 of user 10 as the skin may not be perfectly flat.

The electrical bio-signal received by the electrode 3530 may be transmitted to a signal receiver, such as a processor or other computing device (not shown). In some embodiments, the signal receiver receives the electrical bio-signal from the body 3520 of the sensor. In some embodiments, the body includes a conductive portion 3527 for receiving the electrical bio-signal from the electrode. The conductive portion 3527 may be a conductive coating, a conductive material integrated into the body, or both. In some embodiments, the conductive coating is a conductive paint, such as a metallic paint, or a carbon paint. In some embodiments, the metallic paint includes silver, gold, silver-silver chloride, or a combination thereof. In some embodiments, the conductive material is a carbon-loaded plastic, or a conductive metal. In some embodiments, the body is 3D printed with a conductive material incorporated therein. In some embodiments, impedance between the electrode and a connection on the sensor for a wire from the signal receiver is less than about 1 kΩ. In some embodiments, the impedance between the electrode and the connection on the sensor is from about 1 Ω to about 500 Ω. In some embodiments, the connection is on the body 3520 or on a housing 3760 of a sensor 3700 shown in FIG. 19

In some embodiments, the actuator 3540 electrically connects the electrode 3530 to the body 3520. For example, an electrical bio-signal may be transmitted from the electrode 3530 to the body 3520 via the actuator 3540. In some embodiments where the actuator 3540 includes a spring 3542, the spring 3542 is conductive. For example, a spring 3542 biased on one end against a collection plate 3534 and on the other end against the body 3520, the spring may act as a conductor.

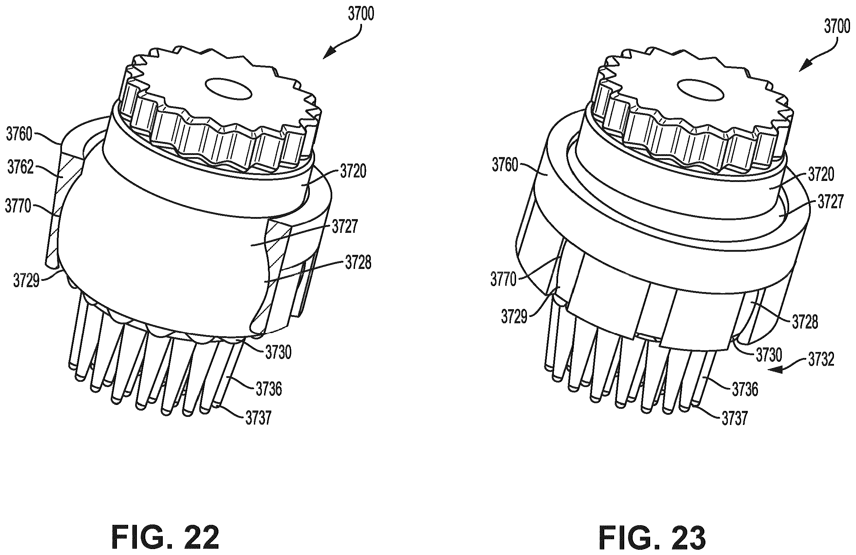

In accordance with an aspect of the embodiments described herein, body 111 may include sensors such as bio-signal sensors 3700 for obtaining bio-signals from the scalp or skin 11 of user 10. Having reference to FIGS. 22 and 23, in some embodiments, a sensor 3700 includes a gimbal 3770 configured to orient the electrode 3730 normal or substantially normal to the skin 11 of user 10. A normally oriented electrode 3730 may have better contact with the user's skin. For example, where prongs 3736 are the same length, a normal orientation prevents the angular contact with the user's skin where certain prongs are not lifted off from the user's skin. Further, where the electrode 3730 contacts the skin at an angle, one or more of the prongs 3736 may be pushed up by the hair. In some embodiments, body 3720 includes a spherical portion 3728, wherein the sensor further includes a housing 3760 defining a joint portion 3762 configured to receive the spherical portion 3728 of the body 3720 such that the gimbal 3770 includes the spherical portion 3728 and the joint portion 3762. In some embodiments, the spherical portion 3728 is removably receivable by the joint portion 3762. In some embodiments, the interface between the joint portion 3762 and the spherical portion 3728 includes a friction reducing agent. In some embodiments, the friction reducing agent is a carbonaceous material. In some embodiments, the carbonaceous material is integral to at least a portion the body 3720, the housing 3760, or both. In some embodiments, the housing 3760 includes an electrical connection portion for establishing an electrical connection between the sensor 3700 and a signal receiver.

In some embodiments, body 3720 includes at least one groove 3729 for receiving at least a portion of the user's hair therein.

In some embodiments, at least a portion of the conductive portion 3727 is disposed in or on the spherical portion 3728. In some embodiments, the electrical bio-signal received from the electrode 3720 is transmitted to the housing 3760 from the body 3720. In these embodiments, the signal received may connect to the housing 3760. In some embodiments where a friction reducing agent is included, the friction reducing agent includes or is a conductivity modifier to improve impedance. In some embodiments, the conductivity modifier is a metal powder, graphite, carbon nanotubes, metal-coated glass or plastic beads. For example, where the friction reducing agent is a carbonaceous material integral to the body 3720, the carbonaceous material may provide both friction reduction and conductivity. In some embodiments, a wire on a support portion 4002 of a head-mounted apparatus 4000 is connected at one end to the sensor 3700.

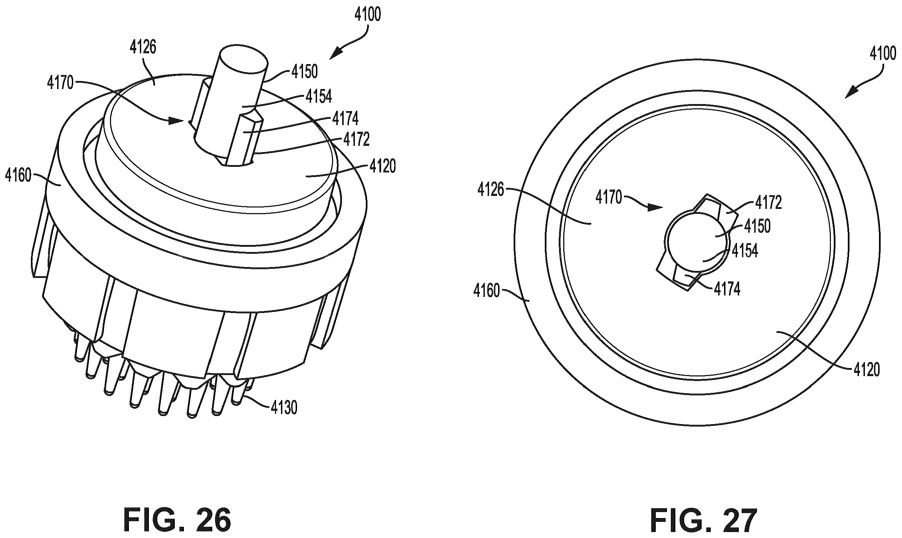

Having reference now, to FIGS. 26 and 27, in some of the embodiments where the rotational movement is limited, the sensor 4100 includes a rotational limiter 4170 for limiting the rotational movement of the electrode 4130. If the hair is rotated excessively in a single direction, the hair may become wrapped or tangled. In some embodiments, the rotational limiter allows an oscillatory movement along a rotational axis for the electrode to get between the user's hairs. In some embodiments, the rotational limiter limits the rotational movement to at least about 0.25 radians. In some embodiments, the rotational limiter 4170 includes a slot 4172 and a key 4174 configured to rotate restrictively within the slot 4172. The movement of the electrode 4130 with respect to the body 4120 are limited by the slot 4172 and the key 4174. In some embodiments, the upper end 4126 of the body 4120 defines the slot 4172 and the shaft 4154 of the contact adjuster 4150 includes the key 4174. In some embodiments, the rotational limiter includes a stop disposed in the body, the electrode, the shaft, or any combination thereof. In some embodiments, a housing 4160 is configured to receive body 4120.

In some embodiments, a light connected to the processor indicates a brain state at the sensor 3500 or sensor 3700. In some embodiments, the brightness or color of the light is modified according to an event in the brain, such as an event related potential, a continuous EEG, a cognitive potential, a steady state evoked potential, or combination thereof. In some embodiments, the light is integral with the sensor or mounted proximate the sensor on a support portion of a head-mounted apparatus.

In some embodiments, body 111 may include other bio-signal sensors 20 such as non-contact electrodes 180.

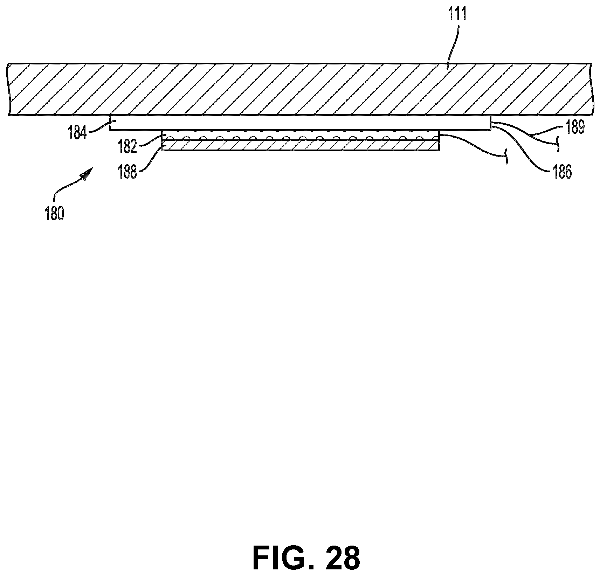

Having reference to FIG. 28, in some embodiments, non-contact electrodes 180 include a conductive layer 182 and a conductive noise layer 184 with a dielectric layer 186 disposed therebetween. The conductive noise layer 184 reduces the noise in the signal obtained by the electrode 180. The conductive noise layer 184 may be an active guard or a ground plane. In some embodiments, a dielectric layer 188 is applied to a user facing side of the conductive layer 182. The conductive layer 182 connects to electronics module 32 or sensor electronics via a wire 189.

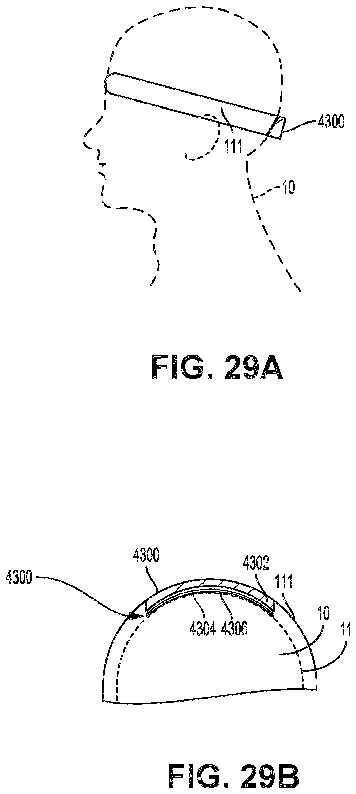

In some embodiments, a non-contact electrode may take the form of capacitive electrode 4300, as shown in FIG. 29A, or other suitable capacitive electrode. FIG. 29A illustrates a side view of user 10 wearing a wearable device 100 having a bio-signal sensor in the form of a capacitive electrode 4300, according to an embodiment. FIG. 29B illustrates a partial top view of wearable device 100 of FIG. 29A.

In some embodiments, body 111 includes one or more capacitive electrodes 4300, for example, positioned adjacent a top of the head of user 10 and the back of the head of user 10, as shown in FIG. 29A. Electrodes 4300 may be disposed in body 111 of wearable device 100 to receive bio-signal data of user 10. In some embodiments, received bio-signal data may include brainwave data of user 10. In some embodiments, capacitive electrode 4300 may be a noncontact electrode that does not come into direct contact with skin 11 of user 10.

Body 111 may include a compressible foam 4302 which may conform to the shape of the head of user 10. In some embodiments, compressible foam 4302 may be formed of an open cell foam, such as open cell foam material known to a user skilled in the art. Compressible foam 4302 may be compressible such that when the wearable device 100 is affixed to the head of user 10, compressible foam 4302 conforms to the head of user 10. In use, the compressible foam 4302 may be compressed and conform to the head of user 10 by clinching of body 111 to secure wearable device 100 to user 10.

In some embodiments, on a surface of compressible foam 4302 adjacent user's 10 head, a conductive layer 4304 of capacitive electrode 4300 is secured to compressible foam 4302.

Conductive layer 4304 may have a thickness between 1 and 100 µm, in an example 20 µm. Conductive layer 4304 may be formed of a conductive material such as a polymer substrate with conductive ink, a conductive polymer, conductive fabric or a flexible PCB.

Conductive layer 4304 may be insulated adjacent to the head of user 10 with an insulating layer 4306. Insulating layer 4306 forms a dielectric medium, creating a capacitive coupling between conductive layer 4304 and skin 11 of user 10. In some embodiments, hair or other body tissue of user 10 may further contribute to the dielectric formed by insulating layer 4306 and the capacitive coupling may form across hair or other body tissue of user 10. Hair of user 10 may be compressed and held in place by the pressure exerted by compressible 4302.

Insulating layer 4306 may have a thickness between 1 and 100 µm, in an example 50 µm. Insulating layer 4306 may be formed of a polymer, for example, polyester.

Insulating layer 4306, by providing a minimal insulating layer between conductive layer 4304 and skin 11 of user 10, may moderate variability in the capacitive coupling between conductive layer 4304 and skin 11 of user 10 caused by variances in the properties of user's 10 hair. Insulating layer 4306 may also minimize salt bridging effects that may arise, for example, due to user 10 sweat creating a salt bridge forming an electrical connection between electrodes leading to improper readings being obtained by the electrodes.

In some embodiments, conductive layer 4304 may be connected to the HMD 110 or sensor electronics, for example, a signal conditioning and amplification circuit, via a wire (not shown).

In some embodiments, wearable device 100 includes an electronics module 32 including a computing device or a processor 30 for receiving the bio-signals from the at least one bio-signal sensors 20 and/or hair-penetrating bio-signal sensors 22 located on the loop. Electronics module 32 may connect to any bio-signal sensors 20, 22 or other sensors described herein. Electronics module 32 may also contain a power source such as one or more batteries, for powering electronics module 32. In some embodiments, the electronics module 32 is located on the forehead portion 12, the ear contacting portion 14, the occipital portion 16, or the support straps 18 such as crown strap 18A, and top strap 18B. Electronics module 32 may be mounted on a portion of body 111 that is reinforced and inflexible, so as to structurally support electronics module 32. Electronics module 32 may be selectively mountable and selectively removable on body 111 of wearable device 100.

Conveniently, electronics module 32 may be removable, as described herein, and in combination with a machine washable fabric 121 used for body 111, may allow for wearable device 100 to be machine washable upon removal of the electronics module 32.

Due to the modular capabilities of electronics module 32, it will be appreciated that electronics module 32 may be used with various body configurations or designs, and with different compatibilities.

Since PCBs or FPCBs and electrical components within the body are all passive (unless electricity is passing through them), other components of wearable device 100, such as body 111 may be washable or hand washable.

In some embodiments, electronics module 32 may be integral with body 111, and not releasable. In such embodiments, wearable device 100 may be machine washable.

In some embodiments, electronics module may include "stick-on" electrodes or bio-signal sensors as described herein that may be applied directly to a user's body, or other configuration, using a suitable adhesive. As such, an electronics module in certain embodiments can wrap direct around a user's arm, be stuck on their chest, or other suitable body part.

In an embodiment, the electronics module 32 may be configured to be located on the occipital contacting portion when worn, such that the electronics module 32 is located at an indent in the skull under the occipital bone. Placement of the electronics module 32 at the occipital portion reduces protrusion and may provide better aerodynamics and weight distribution if the device is worn while performing activities requiring movement, and provide a sleeker appearance as compared to placement at the forehead contacting portion 12 or the support strap 18. When the device is designed for a user who is lying down, such as when sleeping, the electronics module 32 may be placed at a point on the device that minimizes the formation of stress points, such as that created by the user's head against a pillow, and minimizes the possibility that the device will snag or catch on a pillow, blanket, etc., for example, if the user moves in their sleep. In some embodiments, electronics module 32 may be located at or adjacent forehead contacting portion 12, which may reduce interference of electronics module 32 with a user's sleep, regardless of whether the user sleeps on their back or side, with or without any sort of pillow.

In an embodiment, the electronics module 32 is located adjacent the forehead contacting portion such that when worn, the electronics module 32 is located proximal the user's forehead.

Centering electronics module 32 on a user's head may improve the quality and reliability of bio-signals received, as the module may thus be closer to various sensing areas of interest.

In some embodiments, the electronics module 32 includes electronics components such as at least one of an analog front end to amplify and filter the bio-signal data, an analog-to-digital converter, a memory to store the bio-signal data received from the bio-signal sensors 20, a wireless radio for communication with a remote processor, a battery, a charging circuit, and a connector for charging the battery. In some embodiments, the electronics package is fixedly or removably mounted on the device. In some embodiments where the electronics package is removably mounted on the device, the loop includes a pouch for containing the electronics module 32.

In some embodiments, one or more electronics components, for example, a pre-amp, may be disposed outside of electronics module 32, and integrated into body 111 of wearable device 100.

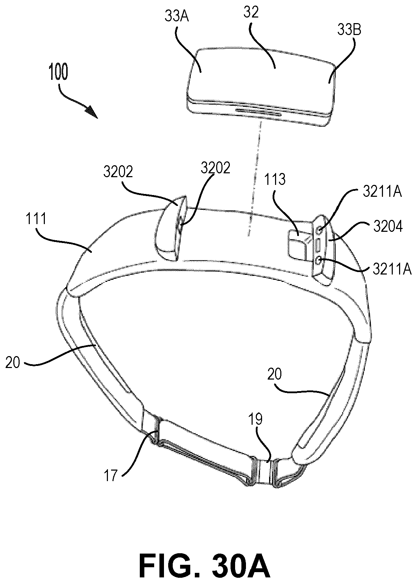

FIG. 30A is a perspective view of an electronics module 32 which is releasable from wearable device 100 by way of a first retention mount 3202 and a second retention mount 3204, mounted on body 111 of wearable device 100.

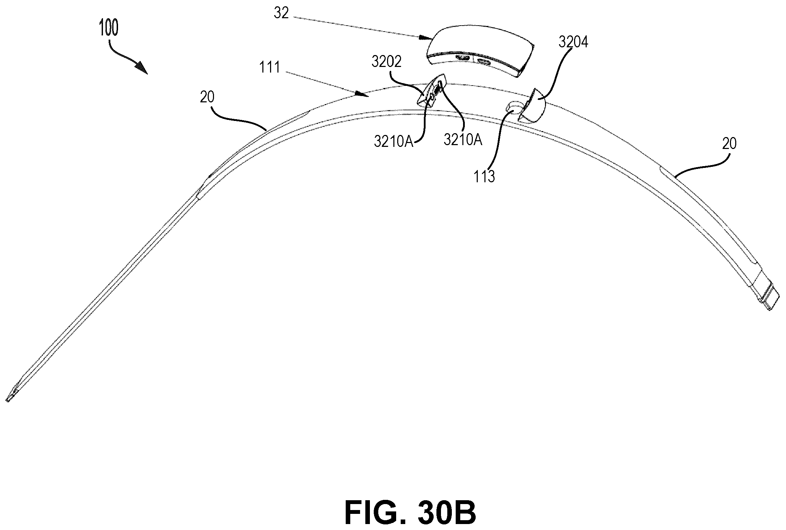

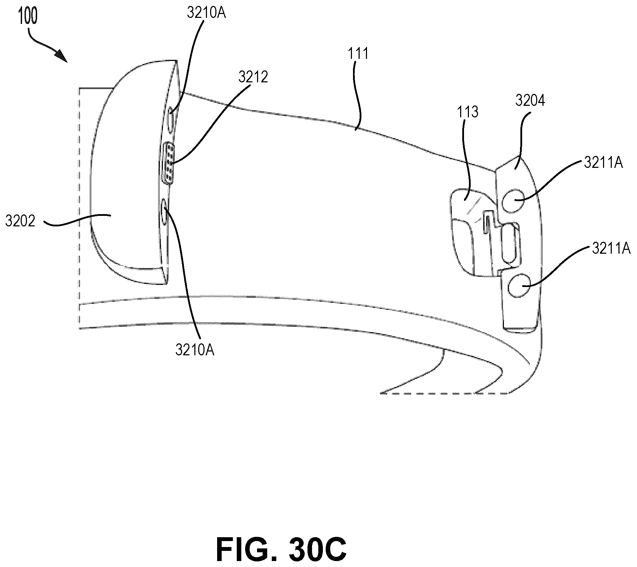

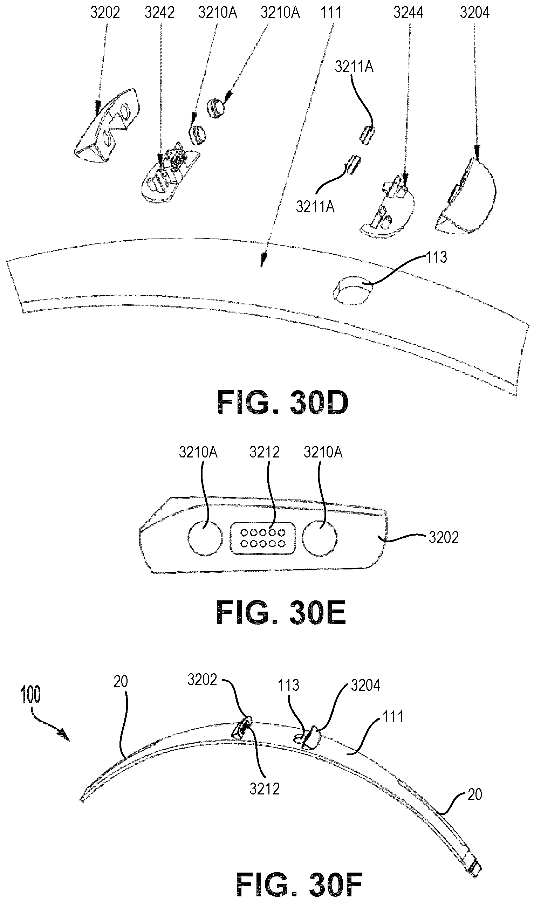

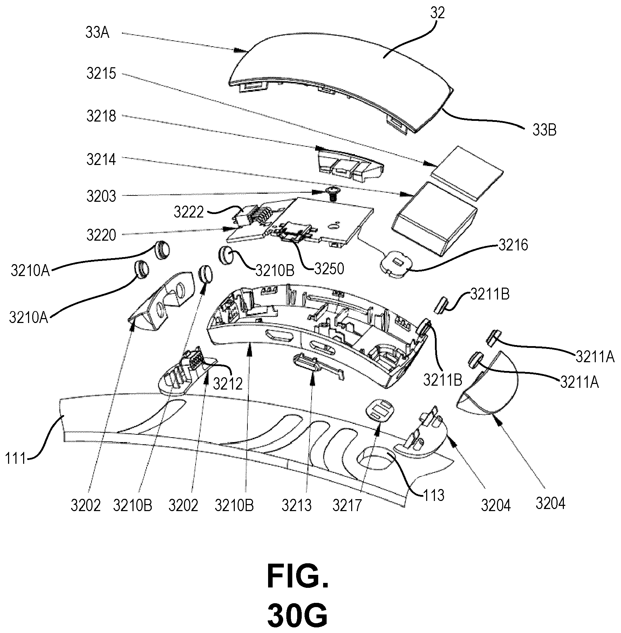

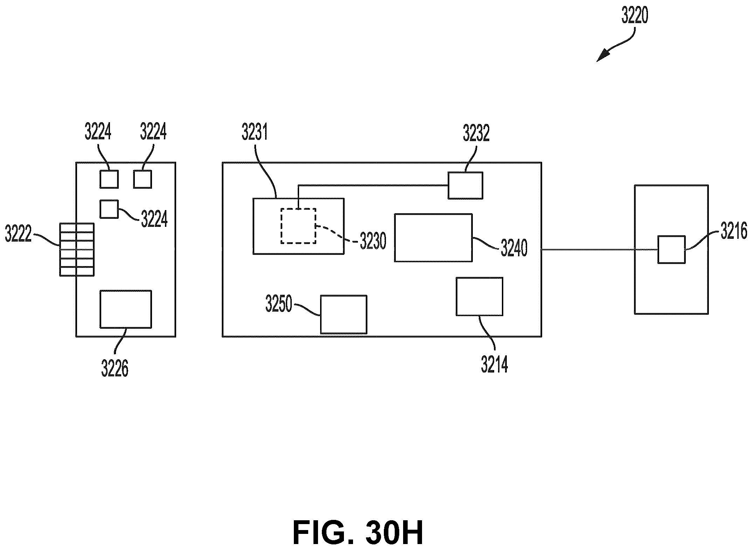

FIG. 30B is another perspective view of an electronics module 32 releasable from wearable device 100. FIG. 30C is an enlarged perspective view of retention mounts for an electronics module 32 of wearable device 100. FIG. 30D is an exploded view of retention mounts for an electronics module 32 of wearable device 100. FIG. 30E is a side view of a retention mount for an electronics module 32. FIG. 30F is another front perspective view of retention mounts for an electronics module 32 of wearable device 100. FIG. 30G is an exploded view of an electronics module 32. FIG. 30H is a schematic view of a printed circuit board and components of an electronics module 32.

First retention mount 3202 and second retention mount 3204 may be configured to provide connectivity between electronics module 32 and other components of wearable device 100, for example, a flexible PCB such as FPCB 1020 or FPCB 1120 and bio-signal sensors 20.

As shown in FIG. 30D, retention mounts 3202, 3204 can include a base 3242, 3244, respectively. Bases 3242, 3244 may be flexible and formed of a suitable flexible polymer. The bases 3242, 3244 of retention mounts 3202, 3204 may be attached to body 111, such as in a flexible manner, and provide flexibility to allow rotation of body 111 relative to electronics module 32, as disclosed herein, while preserving the ability to transmit axial tension forces. In some embodiments, other components of retention mounts 3202, 3204 may be formed of a rigid material.

In some embodiments, body 111 may include an aperture 113, through which optical sensor 3216 may conform and pass light through.

As shown in FIG. 30A, electronics module 32 may have a bow or curved shape, thus forming a space between electronics module 32 and body 111 of wearable device 100 when disposed on wearable device 100, as can be seen, in particular with reference to FIG. 2D.

A curved shape of electronics module 32 may accommodate a variety of curvatures of user's body, such as shapes of user's heads and angular foreheads, and push against bio-signal sensors 20, for example, in a forehead region of wearable device 100, in use, due to force applied by the retention of electronics module 32 against a user's head and the retention of wearable device 100 around a user's body part, such as a user's head.

Bio-signal sensors 20 disposed on fabric, which may have some stretching properties, of wearable device 100 may be extended between the retention mounts 3202 and 3204, and thus fabric fits with contact across a user's body part, such that even if a user has a flat forehead, there may be a good amount of pressure on the skin, to allow for better bio-signal data readings. Fabric between retention mounts 3202 and 3204 may also stretch such that it conforms to the shape of electronics module 32 during use. Certain portions of body 111, for example behind electronics module 32, may also contain foam or other suitable compressible material, allowing compression and adaptability to a user's head, and thus may allow for better contact of bio-signal sensors 20 with a user and thus better quality and reliability of received signals.

Conveniently, as middle electrodes in contact with a user's forehead may be important for signal quality, the shape of electronics module 32 may be suitable for any head shape, while allowing electrodes of bio-signal sensors 20 to have a good contact with a user's body, such as skin of a user's forehead.

Referring to FIG. 30G, in some embodiments, the body of electronics module 32 may attach to retention mounts 3202 and 3204 by way of a snap-fit and magnet pairs 3210A-3210B and 3211A-3211B respectively which may be polarized so as to be magnetically attracted to each other. Magnets 3210A and 3211A can be affixed to retention mounts 3202 and 3204 respectively. Magnets 3210B and 3211B can be affixed to electronics module 32 at ends 33A and 33B respectively.

Magnets 3210A, 3210B, 3211A, and 3211B may also be polarized to ensure that the body of electronics module 32 connects to the retention mounts in the proper orientation.

As shown in FIGS. 30D and 30G, in some embodiments, magnets 3210A, 3210B, 3211A, and 3211B have an inward ridge along an inner third circumference to fit them into the body of electronics module 32, and apertures of retention mounts 3202, 3204 to retain the magnets.

Retention mount 3202 may also include a port 3212, for example, a serial port for serial communication between a printed circuit board (PCB) 3220 of electronics module 32 and a flexible PCB (FPCB such as FPCB 1020 or FPCB 1120) in body 111 of wearable device 100, and for example, connected to bio-signal sensors 20.

PCB 3220 may be formed of a flexible PCB which is embedded into three, and connects said three rigid PCBs together.

Connector 3222, in an example, a serial connector for mating with port 3212, in electronics module 32 may include pushpin connections to connect to port 3212, and may be configured as push pin, spring-loaded pin, or pogo pin connectors to counteract forces of magnet pairs 3210A-3210B and 3211A-3211B but maintain firm connection between electronics module 32 (and thus PCB 3220) and retention mount 3202 (and thus a FPCB in body 111, such as FPCB 1020 or 1120 connected to sensors such as bio-signal sensors 20). Thus, connection is maintained between PCT 3220, FPCB such as FPCB 1020 or 1120, and bio-signal sensors 20 by pogo pins and magnetic attraction.

In some embodiments, magnets 3210A, 3210B, 3211A, and 3211B may need to be calibrated to ensure a good hold against the push pins of connector 3222.

Electronics module 32 may be attachable to body 111 by a magnetic force.

In some embodiments, electronics module 32 includes first magnet 3210B at first end 32A to attach to the first retention mount 3204 by magnetic force and a second magnet 3211B at second end 32B to attach to the second retention mount 3202 by magnetic force.

As illustrated in FIG. 30G, in some embodiments, electronics module 32 includes, within a front housing 3201A and a rear housing 3201B, a PCB 3220 secured to rear housing 3201B, for example, by way of a fastener 3203, which is connected to connector 3222 by way of a microcontroller (MCU) 3226 or analog to digital converter (ADC) that digitizes an incoming analog signal, for example, received from bio-signal sensors 20 by way of a PCB or FPCB (such as FPCB 1020 or FPCB 1120) in wearable device 100, which may be amplified by one or more analog signal amplifiers 3224.

In some embodiments, PCB 3220 may be a flexible-rigid PCB and curved in shape, complementary to a curved shape of electronics module 32, and in particular front housing 3201A and rear housing 3201B. A schematic of PCB 3220 is illustrated in FIG. 30H.

As shown in FIG. 30G, in some embodiments, electronics module 32 further includes a power source for various components of electronics module 32, such as a battery 3214, which may be supported by battery foam 3215 and activated by a power button 3213, an optical sensor 3216, a light emitter 3218, a communications module 3230 and associated antenna 3232, and a memory 3240. In this way wearable device 100 can include electronic components such as an optical sensor 3216, a light emitter 3218, a communications module 3230 and associated antenna 3232, and a memory 3240.

In some embodiments, wearable device 100 includes an optical sensor 3216 such as a pulse oximeter which illuminates the skin and measures changes in light absorption to obtain a photoplethysmogram (PPG), an optically obtained plethysmogram that can be used to detect blood volume changes in the microvascular bed of tissue.

Optical sensor 3216 may be disposed behind a cover 3217, formed, for example, of glass, and positioned to extend through aperture 113 and transmit light through aperture 113.

In some embodiments, optical sensor 3216 is disposed in a region of electronics module 32 near to first retention mount 3202 or second retention mount 3204. Electronics module 32 may have a bow or curved shape and positioning optical sensor 3216 nearer to the first retention mount 3202 or second retention mount 3204 can reduce the distance between optical sensor 3216 and a user's skin or fabric.

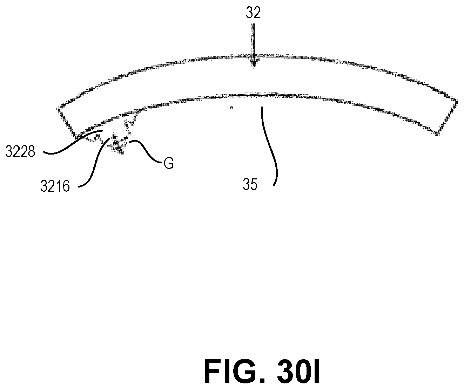

As shown in FIG. 30I, in some embodiments, optical sensor 3216 is mounted on flexible protrusion 3228 on electronics module 32, and flexible protrusion 3228 can compress as electronics module 32 is drawn towards the body of user 10. Flexible protrusion 3228 can enable optical sensor 3216 to accommodate a variety of user curvatures. The flexible protrusion can permit optical sensor 3216 to extend into concave space 35 towards the skin (along line G) of a user for users with little body curvature, but can retract when force is applied by the skin of other users with greater body curvature ensuring that flexible protrusion 3228 accommodates different body curvatures.

Optical sensor 3216 may generate and emit green, red and infrared light wavelengths, which may be used for optical sensing based on detecting a reflection distance of light reflected into the optical sensor. Other suitable wavelengths may also be contemplated. In some embodiments, optical sensor 3216 senses light generated by other light sources, such as LEDs, adjacent to the optical sensor on wearable device 100 or from another suitable light source.

A variety of such wavelength may allow for different signals to be received and different data extrapolated. For example, green light may provide a better quality signal, while red and infrared light may allow for pulse oximeter sensing and analysis. Certain extended wavelengths measure oxygen concentration. In an example, light generated by optical sensor 3216 can reflect off of oxygenated and detection and breathing the light sensing the light reflectance, which can indicate breathing rates and heart rate.

In some embodiments, optical sensor 3216 may sample an optical signal at up to 4 kHz.

Wearable device 100, in certain configurations, may be wearable of a user's clothes, and optical signals may pass through a user's clothing and provide meaningful readings.

In some embodiments, optical sensor 3216 may be used to sense a compression of fabric as a user breathes. In an example, optical sensor 3216, as fabric such as body 111 of wearable device 100 compresses, optical sensor 3216 thus moves close to a user's skin. Thus, it may be possible to detect that a user is breathing based on the light reflected back to optical sensor 3216 from a surface such as user's skin. In an example where wearable device 100 is disposed around a user's chest, as the user's chest expands, wearable device 100 stretches and expands, and the distance between optical sensor 3216 and the user's skin reduces. Therefore, light emitted by optical sensor 3216 does not travel as far before being reflected. Light may thus be used to measure strain or stretch of fabric in wearable device 100. In other embodiments, a strain gauge may be used to determine compression or stretch of a fabric of wearable device 100.

In some embodiments, optical sensor 3216 detects additional bio-signals based at least in part on a detected reflection distance of light reflected into the optical sensor.

In some embodiments, optical sensor 3216 detects additional bio-signals, such as blood flow, based at least in part on a measured colour and intensity of light reflected into the optical sensor.

Light emitter 3218 may include one or more LEDs or other suitable light source(s) such as incandescent or fluorescent bulbs.

In some embodiments, light emitter 3218 is disposed on a bottom of electronics module 32, within a user's line of sight, when wearable device 100 is disposed on the user's head. In some embodiments, light emitter 3218 may be disposed on other parts of electronics module 32, such as a top.

Light emitter 3218 may generate light signals to communicate with a user that wearable device 3218 is on. Light signals generated by light emitter 3218 may include a variety of suitable light colours, frequencies, intensities and settings.

In some embodiments, optical sensor 3216 may be configured to detect light emitted by light emitter 3218. Optical sensor 3216 may measure illumination level on a user's eye lids (in addition to, or as opposed to light level in the environment more generally).

Light emitter 3218 may be configured to emit light used to wake up a user, or as biofeedback. In an example, light cues may be used as a wake-up routine. In an example, light emitter 3218 may flicker a light which to get a user's attention when they are asleep, or in a certain sleep state, for example, to trigger a user to change sleep states or to wake up.

In an example, wearable device 100 may be configured to detect when a user is in a dream state, and send stimulus, for example, light emitted by light emitter 3218, to rouse the user to consciousness. Thus, a user's dream may be disrupted intentionally. Such disruption may be done in a way that is not seriously disrupting the user's sleep, and may improve the user's dream recall.

Other stimuluses may similarly be applied, such as vibration, sound, smell and / or electric shock. For embodiments that are configured to apply a type of stimulus, there exist further embodiments where the device is configured to apply multiple types of stimuluses at once. The combination of several types of stimulus can produce, for some users, a powerful reaction.

Other examples of stimuluses can include user selected and / or generated stimuluses. These can include, for example, positive affirmations. In some embodiments, wearable device 100 can be configured to receive positive affirmations from the user and can use these affirmations as a stimulus triggered by selected events. In some embodiments, the user is able to select which events trigger the positive affirmations.

In some embodiments, electric stimulus can be applied to a user near the ear. Such stimulation may be able to stimulate the auricular region of the head of a user which may in turn stimulate the auricular branch of the vagus nerve. Such nerve stimulation can be highly therapeutic for some users in some applications.

In some embodiments, wearable device 100 can be configured to trigger a scent diffuser in response to certain events. For example, calming scents can be diffused near a user when wearable device 100 detects that the user is in or is about to enter a pre-arousal state while sleeping.

In some embodiments, wearable device 100 can use light, vibration, sound, smell (such as through the misting of essential oils), or other stimulus to intentionally rouse a sleeping user. For example, when wearable device 100 detects that the user is in a dream state wearable device 100 can apply stimulus to the user to make the user aware that they are in a dream state and give the user lucid control over the dream state, allow them to recall their dreams, or serve to alter the dream experience. In the case of lucid dreaming, wearable device 100 rouses the user into a state consciousness that enables lucid dreaming. In these embodiments the stimulus intentionally rouses the user to consciousness without disrupting the dream state through a stimulus that the user learns to recognize within the dream state, such as a sound, a voice stimulus, a pattern of vibration or light flashes that may be visible through closed eyes. Wearable device 100 monitors the dream state and may adaptively control the stimulus to provide the intended function. For example, if the dream state is observed to be disrupted too close to the waking state, the stimulus may stop, lessen, or otherwise change, or if the user is not disturbed in any way, the stimulus intensity may increase or otherwise change.

In some embodiments, wearable device 100 can apply stimulus to a user with a pattern determined by the user's sleep phases. These phase-locked stimuluses can promote restful sleep. Phase locking refers to a function of wearable device 100 that provides a variation in stimulus depending on what phase of sleep the user is in, as well as the pattern observed in the user's sleep phase in the prior duration of their sleep and the historical patterns of sleep over multiple nights. In these embodiments, the stimulus can gently guide the user through their typical sleep cycle or offer a means of shifting their sleep cycle into a pattern that is more effective for rest, performance, or some other goal (such as dream recall).. Further embodiments can train the user to react predictably to certain types of stimulus to more effectively guide the user through a full sleep cycle.

In other embodiments, two users can each wear a wearable device and the devices can communicate with each other. This may allow synchronization between the sleeping patterns of the two users to improve the restfulness of sleep in a shared bed. Another use can be to promote shared dreaming by aligning dream phases. Other embodiments can also allow stimulus to be created within the sleep environment, such as a wakeup alarm that can simultaneously optimize the wakeup alert for both users. The devices can process some information necessary within their electronics modules to reduce latency arising from delays in communication with external devices.

In some embodiments, two or more wearable devices can communicate using light or sound emitted from the wearable devices. The frequency of the light or sound used to communicate between the two or more devices can be selected such that it is outside the range of typical human detection.

In some embodiments, wearable device 100 can be further configured to apply a stimulus (e.g., light, vibration, or sound) to the user and determine the efficacy of that stimulus in arousing the user. The device can be configured to adapt to the user and modulate the stimuluses used (e.g., intensity, timing, type) in order to adaptively alter the user's dream state in a more effective way.

As shown in FIG. 2J, in some embodiments, a light receiver 3256 may receive and detect ambient light or emitted light that is in the proximity of wearable device 100 or the user's eyes, using a suitable light detector.

In some embodiments, light receiver 3256 is disposed on body 111 adjacent an eye of user 10 to detect light adjacent the eye of user 10.

In some embodiments, wearable device 100 is configured to be worn around a user's chest. These embodiments can offer a greater breadth of breath data. For example, devices worn around the chest can offer ECG measurement via the electrodes, and chest expansion using sensors that measure deformation of body 111. For example, a light transmitter / receiver pair may measure compression of body 111 and be appropriately filtered through a low pass filter to measure the chest expansion action of breathing. Devices worn around the chest can be suitable to provide breathing data through chest movement data, cardiac movement and electrical data, and chest expansion data.

In some embodiments, processing as described herein is performed on a local computing device, such as a mobile phone local to a user. A brain model of a user may be maintained.

Using a user's brain model, it may be possible to detect if a user is in a dream state, for example, by detecting changes in brain activity and eye movements through EOG, physical movements and lack of physical movements.

In some embodiments, light emitter 3218 may be used to indicate to others a user's state, for example, that the user is sleeping.

Thus, light emitter 3218 may be used to indicate sleep state, time synchronization, physiological signals, direct feedback, heart rate, state of focus, and the like.

Feedback, such as that from light emitter 3218, may be for a user or others. In an example, emotion can be communicated to someone else. In another example, light emitter 3218 can provide a caregiver with feedback about a patient (the user).

In some embodiments, light emitter 3218 may be used to make a brain measurement. For example, by flashing light and measuring brain response, for example, using one or more bio-signal sensors 20, at concurrently. Thus, a synchronized brain measurement may be obtained by providing the event (flashing light).

Light emitter 3218 may also be configured to achieve real-time communication with a local computing device, for example, with a line of sight to a receiver at a local computing device. Thus, a user's biological state can be communicated, and create biofeedback experiences. Conveniently, reduced latency may be achieved.

In some embodiments, an external camera could read light emitted by light emitter 3218 and synchronize those readings with a user's facial expressions.

In some embodiments, electronics module 32 also includes a communications module 3230 such as a Bluetooth module disposed behind a shielding 3231, which may shield from noise, and connected to an antenna 3232 for communication, for example with a local or remote computing device using an appropriate communications protocol. In other embodiments, data transfer may be wired, Wi-Fi, fibre optic, or other suitable communications protocol, between wearable device 100 and a computing device, such as local computing device or remote computing device, described in further detail below

Communications module 3230 may be disposed away from magnets in electronics module 32 to avoid magnetic interference.

Communications module 3230 may be configured for other communications protocols, selected based on factors such as latency requirements, distance, speed, bandwidth, interference if in noisy environment.

In some embodiments, electronics module 32 includes memory 3240. Memory 3240 may include random-access memory, read-only memory, or persistent storage such as a hard disk, a solid-state drive such as flash memory or the like.

Memory 3240 may be configured to store certain bio-signal data sensed by bio-signal sensors 20, various data sensed or processed by components of wearable device 100, such as an accelerometer, processing done on board wearable device 100, or the like. Thus, memory 3240 may store data such as movement data and EEG signals.

Electronics module 32 may include an external connector 3250 such as a micro-USB connector to charge battery 3214.

In some embodiments, external connector 3250 may be used for data transfer with another device such as a local computing device or a remote computing device, in an example, wired or wireless.

In some embodiments, electronics module 32 also includes an authenticator (not shown), that can be configured for anti-counterfeiting measure to authenticate a source of the electronics module 32.

An authenticator can include a chip on body 111 of wearable device 100 and one on electronics module 32. In an example, a flexible PCB that has chip that has authentication code. Upon connection of electronics module 32 electronics module 32 queries headband to determine authenticity, and receives a reply.

Electronics module 32 can thus authenticate a headband of wearable device 100. Electronics module 32 takes that response and authenticates itself against a remote server. As both electronics module 32 and body 111 headband have serial numbers, they may be authenticated with a private key. And access may be denied for modules that do not successfully authenticate.

Wearable device 100 can also include ambient light receiver 3256, for example, to detect a level of ambient light in an environment or a room.

As shown in FIG. 2J, in an example, wearable device 100 can include an accelerometer 3258. Accelerometer 3258 may be used to track a user's movement. Based on a user's movement, bio-signal data, such as that received from bio-signal sensors 20, during a window of time adjacent the movement at a certain threshold, such as plus or minus 30 seconds, may be discarded because an associated EEG signal may be presumed to be of poor quality due to noise added by the user's movement.

In certain embodiments, it may be desirable to retain bio-signal data received while a user's body is relatively still, for example, as they are sleeping, meditating, or doing light stretching. This may be because when a user moves more, the signals received are less reliable, as a dry electrode contact may not move well with the user.

In some embodiments, accelerometer 3258 may be used to filter out bio-signal data received. For example, if accelerometer 3258 senses movement, which is then attributed to the user, bio-signal data received within a range of time adjacent that movement may be deleted or filtered.

Accelerometer 3258 may also be used to conserve battery life by choosing what data to send (for example, to a local or remote computing device) from wearable device 100. For example, if accelerometer 3258 detects that a user is moving, then data may be deleted or cached (to be sent at a certain time or a later time) instead of being transferred to another device.

In some embodiments, an algorithm onboard wearable device 100 can make a decision on how to process data received from components such as bio-signal sensors and an accelerometer. By way of negotiation between wearable device and an algorithm on a local or remote computing device that wearable device 100 is in communication with can allow certain local decisions.

For example, a neural network such as a multilayer perceptron (MLP) may be implemented on wearable device 100 to determine if bio-signal data, such as EEG, is sufficient and to remove noise, for example. Other suitable neutral network or machine learning techniques may be considered.

Thus, wearable device 100, for example, using electronics module 32, may be configured to filter signals so that they can be transferred to a local or remote computing device without corrupting signals that are being measured, and may provide a signal processing and/or conditioning step.

A decision making process may involve negotiating how and when to save locally and when to transmit to other local or remote computing devices to cope with poor data, different sleep conditions (for example, if a user is sleeping on their head and blocking transmission of data), and the like. Data may be buffered and transmitted when appropriate. In an example, if it is detected that a transmission cannot be verified, data may be saved and buffered, and transmitted when appropriate (for example, with regular checks for the transmission capabilities).

Data on wearable device 100, or electronics module 32, may be time stamped. Thus, bandwidth may be conserved by transmitting only data required for real-time feedback. Lower priority data may be cached or saved, and instead transmitted at a pre-determined point in time, for example, in the morning.

Certain storage or processing capabilities may be available on wearable device 100 or various components such as electronics module 32, and other storage and processing may be offloaded to a local or remote computing device, with which wearable device 100 can communicate, such as by way of Bluetooth or appropriate communication protocol. In some embodiments, processing may be performed on a computing device of wearable device 100, as described in further detail below.

In some embodiments, on-board processing may be performed on wearable device 100. Certain processing can also be done on other local or remote computing devices. In some embodiments, remote processing, for example of bio-signal data or other data received from wearable device 100, may be performed remotely on a cloud.

Processing delegation may be done on the basis of capacity, physical size and price trade-offs.

Certain bio-signal sensor 20 sampling rates may be very high, to ensure useful data is acquired. These signals may be processed on-board on wearable device 100, or a local or remote computing device. Such data may be transferred to the local or remote computing device in real-time, or at certain time intervals.

Processing may also be involved in performing audio detection and audio production capabilities to wearable device 100.

In use, wearable device 100 may be configured for quasi-real-time processing happening between computing on wearable device 100 and local or remote computing devices, and with another level of real-time behaviour on wearable device 100 itself.

For example, a local computing device may be used to do real-time processing, on the level of seconds or minutes, to know what to tune into or detect change in sleep state. It may thus be possible to generate real-time feedback for particular stage of a user's sleep locally, which may be desirable to drive latency as low as possible.

Slow wave feedback may be performed on wearable device 100, however, decision making of computation of when and how to provide such feedback may be performed on another device with more processing power. In an example, sounds or other stimulus to be used for feedback may be then uploaded to wearable device 100.

Thus, it may be possible to create timely feedback locally, while high processing stuff is done at another computing device or offline.

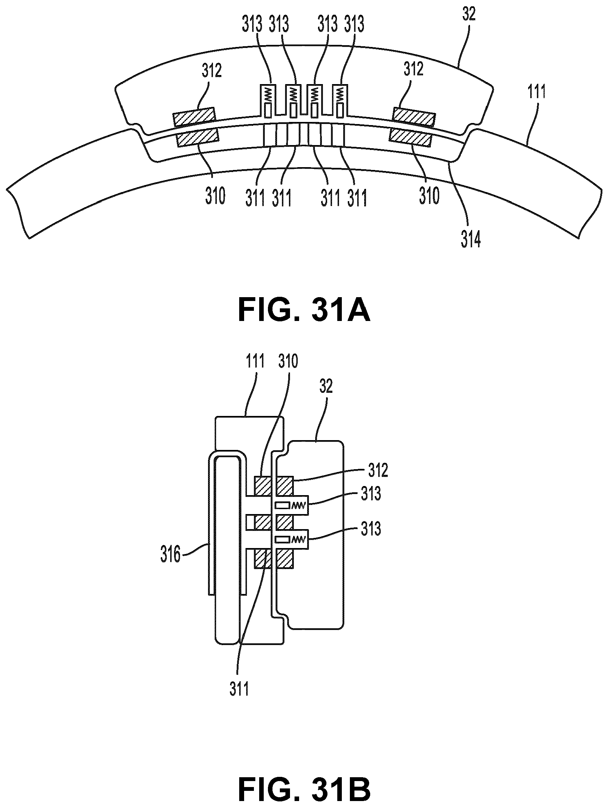

FIG. 31A is a top schematic view of electronics module 32 connected to, in an example, forehead contacting portion 12 of body 111 of wearable device 100. FIG. 31B is a side schematic view of electronics module 32 connected to forehead contacting portion 12 of body 111 of wearable device 100.

As shown in FIGS. 31A, 31B, electronics module 32 may include magnets 312 to cooperate with corresponding magnets 310 in body 111, to retain electronics module 32 against body 111. Electronics module 32 may be thus selectively removable from wearable device 100.

Electronics module 32 may further include spring pins 313 to provide an electrical contact with contacts 320 in body 111. Contacts 311 may further be connected to bio-signal sensors 20, 22 in wearable device 100.

As shown in FIG. 31A, magnets 310 and contacts 311 may be embedded in a substrate 314, made, for example of rubber. Substrate 314 may be generally rigid so as to structurally support electronics module 32.

In some embodiments, contacts 311 may connect to a flexible printed circuit board 316, for example, as shown in FIG. 31B.

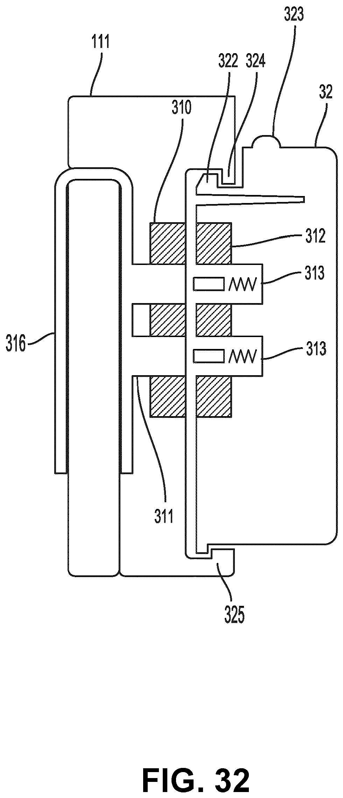

FIG. 32 is a side schematic view of electronics module 32 connected to forehead contacting portion 12 of body 111 of wearable device 100. Electronics module 32 may be configured as shown in FIGS. 31A and 31B and described above, with the addition of a clip 322, to engage with receiving hook 324 to further retain electronics module to wearable device 100. Body 111 may also include a retaining lip 325 to engage with a corresponding lip on electronics module 32 to further secure electronics module 32 to body 111. Protrusion 323 may be pressed to release clip 322 from hook 324 and remove electronics module from body 111.

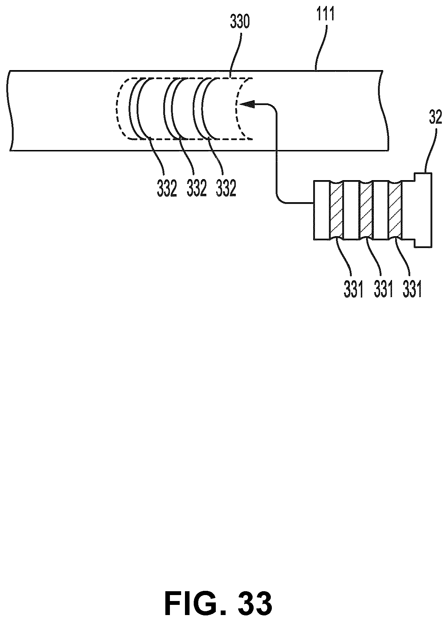

FIG. 33 is a schematic view of a pocket 330 in body 111 for retaining electronics module 32, in a section of body 111 that is elastic, for example, made of elastic fabric. Electrical contacts 331 on electronics module 32 may contact conductive ribs 332 of body 111. Conductive ribs 332 may be integral with body 111, for example, as conductive thread or may be other suitable conductive sensors, and may connect with bio-signal sensors 20, 22.

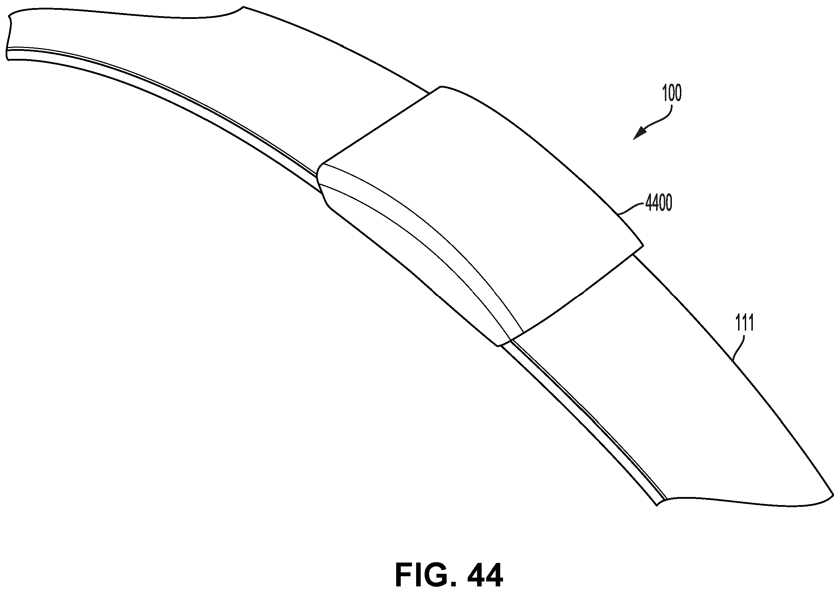

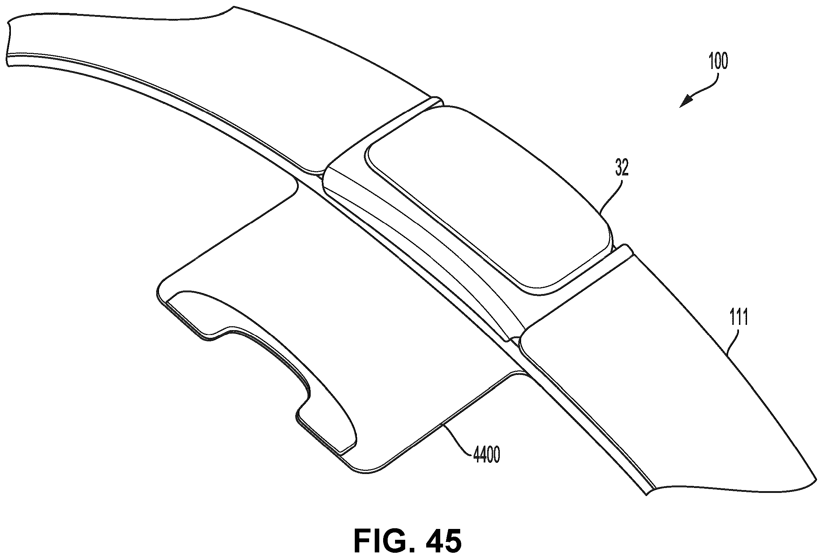

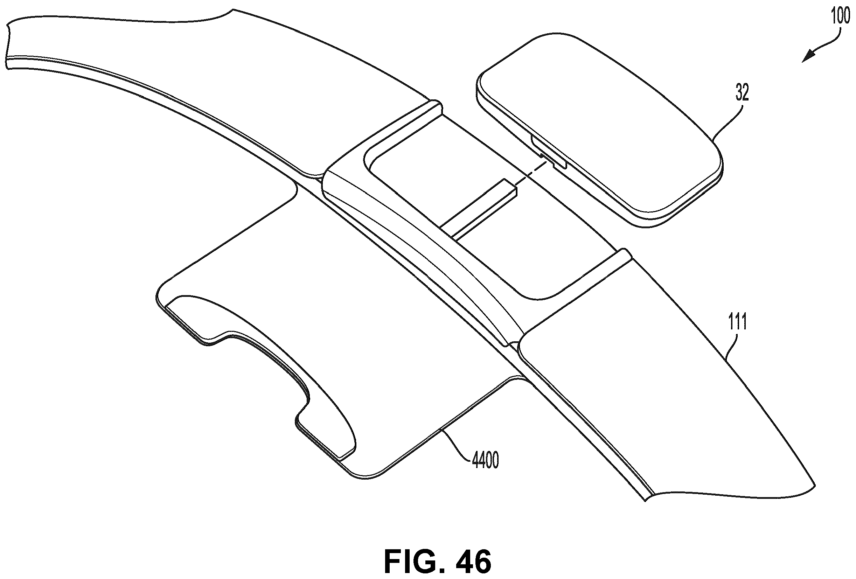

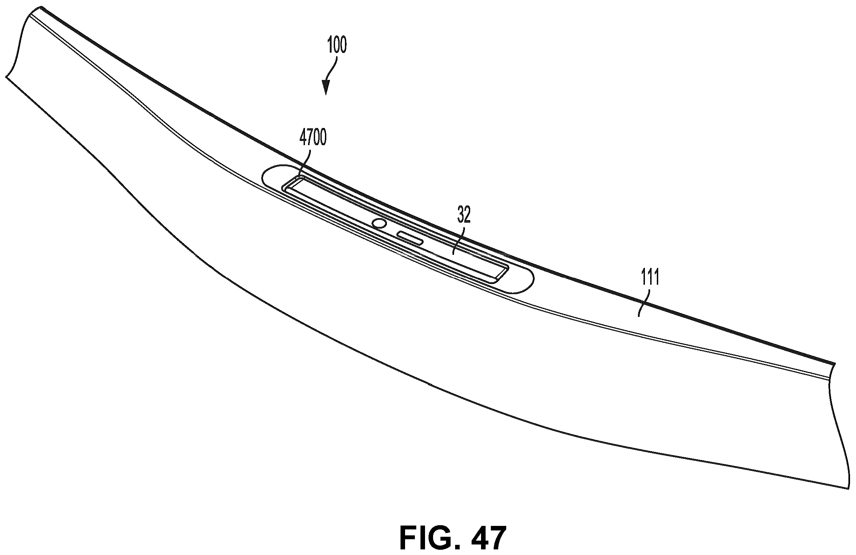

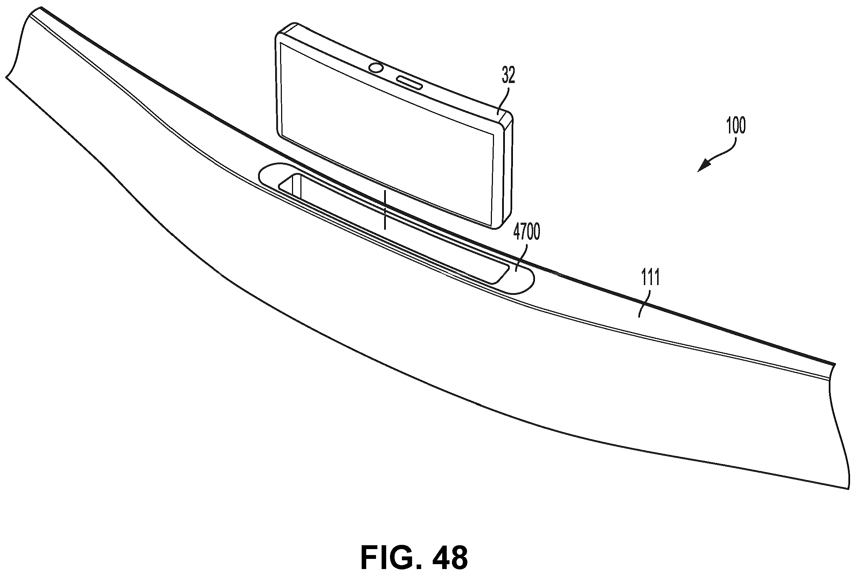

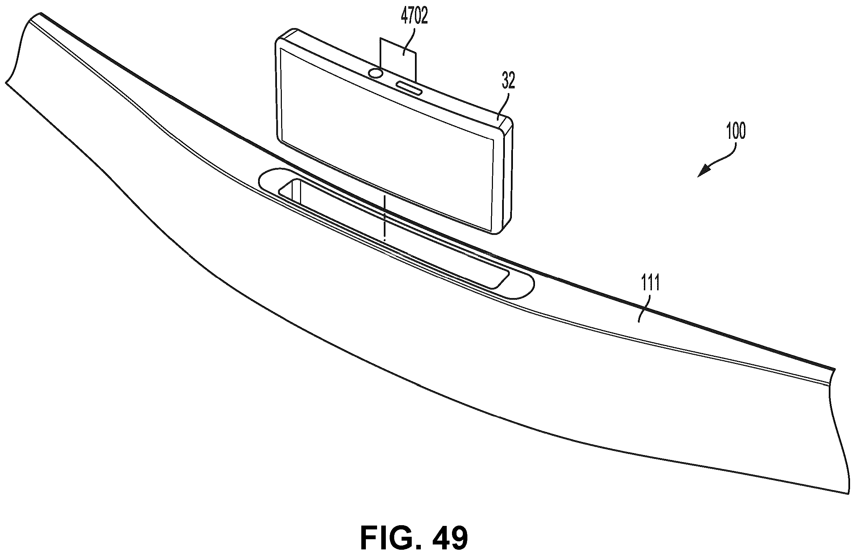

Other embodiments of electronics module 32 incorporate with wearable device are illustrated in FIGS. 44-49. FIG. 44 is a perspective view of wearable device 100 having an electronics module 32 disposed underneath a cover 4400 in a closed position, according to an embodiment. FIG. 45 is a perspective view of wearable device 100 having an electronics module 32 disposed underneath cover 4400 in an open position, according to an embodiment. FIG. 46 is a perspective view of wearable device 100 having an electronics module 32 detached from cover 4400 in an open position, according to an embodiment. FIG. 47 is a perspective view of wearable device 100 having an electronics module 32 disposed in a pocket 4700, according to an embodiment. FIG. 48 is a perspective view of wearable device 100 having an electronics module 32 detached from pocket 4700, according to an embodiment. FIG. 49 is a perspective view of wearable device 100 having an electronics module 32 detached from pocket 4700 and having a fabric flap 4702, according to an embodiment.

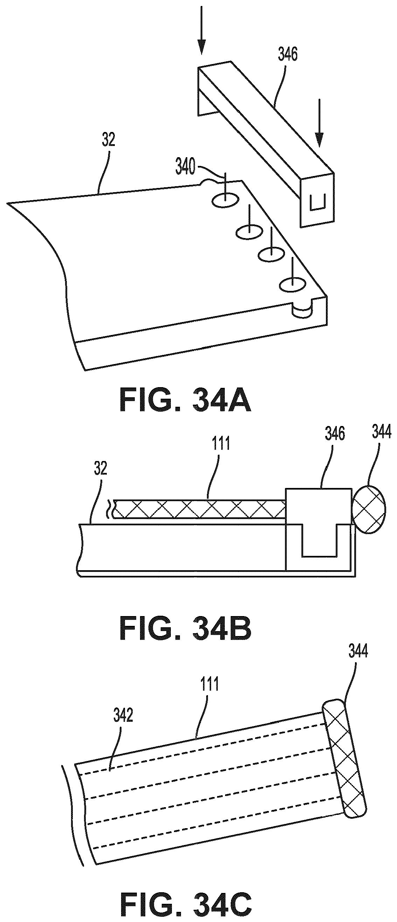

As shown in FIGS. 34A to 34C, in some embodiments electronics module 32 may have conductive pins 340 extruding from a surface, for contact with conductive threads 342, for example, on a section of body 111, and having a molded stop 344. A clip 346 may retain conductive threads 342 against conductive pins 340.

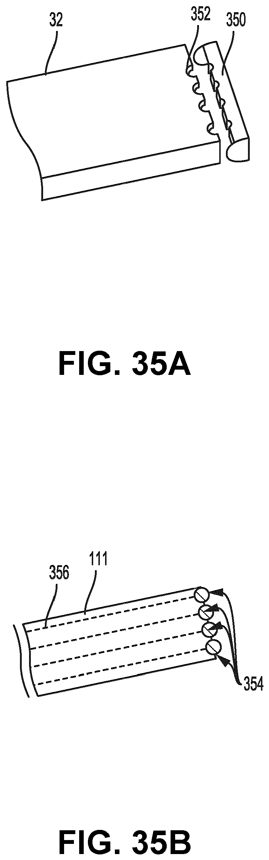

In another embodiment, as shown in FIGS. 35A and 35B, electronics module 32 may include recesses 352 to receive molded contacts 354 connected to conductive threads 356 in body 111. Clip 350 may retain molded contacts 354 in recesses 352 of electronics module 32.

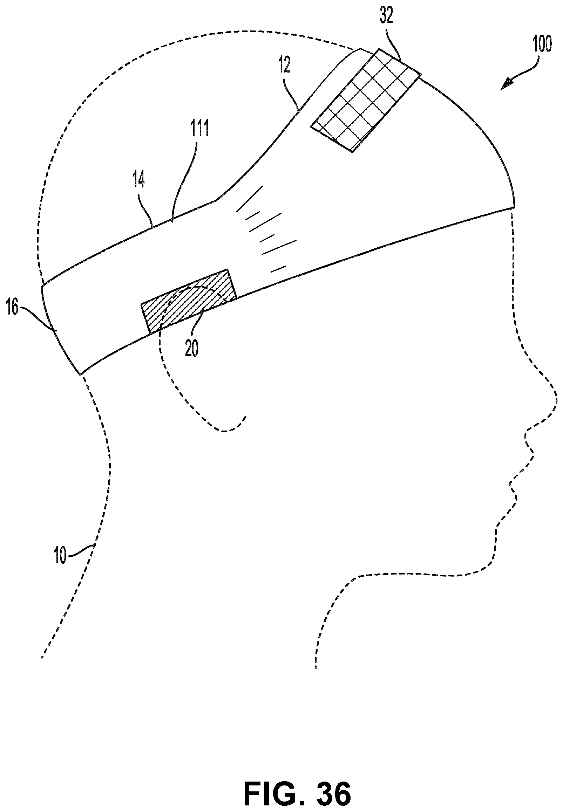

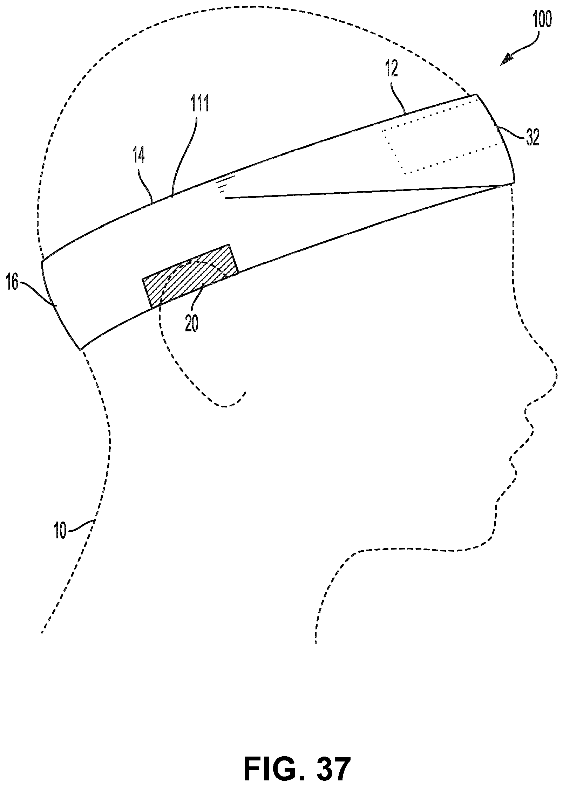

FIGS. 36 and 37 illustrate a side schematic view of an embodiment of the wearable device 100 having an extendable, stretchable forehead contacting portion 12 of body 111 where the electronics module 32 is mountable.

In this embodiment, wearable device 100 may be worn as shown in FIG. 36, with the module high on the head to allow for other wearable technology, such as a heads-up display or VR headset to be worn on the forehead. Alternatively, portion 12, along with electronics module 32, may be folded down as shown in FIG. 37 to hide the electronics module 32.

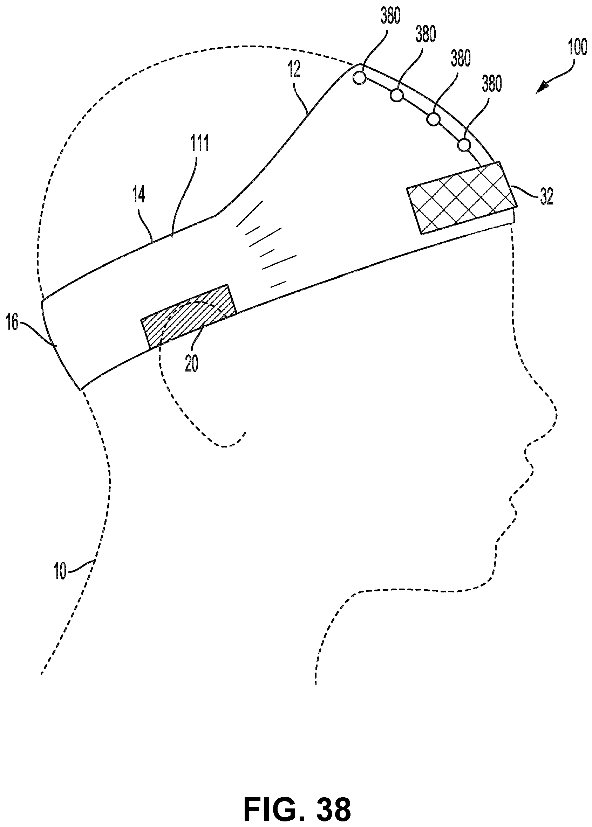

FIG. 38 illustrates a side view of an embodiment of wearable device 100 having an extendable, stretchable portion 12 which has placement or attachment locations 380 for auxiliary electrodes to attach to body 111 for contact with user 10. Attachment locations 380 may provide an opening in which auxiliary electrodes or sensors may be disposed, and provide a contact surface that is conductive and prewired to the electronics module 32, for a connection between auxiliary electrodes or sensors and electronics module 32.

Auxiliary electrodes may be any type of through-hair sensor, and could be attached to the wearable device via snaps, clamps, etc. The extendable, stretchable portion can be pulled back over the hair, offering a large array of potential auxiliary electrode locations (for auxiliary or additional sensors, as described in further detail below).

In some embodiments, the device includes additional auxiliary sensors. Auxiliary sensors may be integrated with electronics module 32, or otherwise integrated with device 100. In some embodiments, an auxiliary sensor is selected from an optical heart rate sensor, a pulse oximeter sensor, a gyroscope, an accelerometer, a magnetometer, a sweat sensor, a light sensor, an audio sensor, a nasal cannula flow sensor or any combination thereof. In some embodiments, the device includes an optical heart rate sensor and/or a pulse oximetry sensor. In some embodiments, the optical heart rate sensor and/or the pulse oximetry sensor are located on the forehead contacting portion such that they contact the forehead or the temple region of the user's head. In some embodiments, signal data from the gyroscope, accelerometer, magnetometer, or combination thereof may be used to determine an attitude and heading reference system (AHRS) to determine the orientation of the head. Such data could be used, for example, to provide additional information when analyzing brain patterns of sleep, activity, etc. For example, analysis of a user's sleep may analyze tossing and turning in conjunction with brainwave signals.

In some embodiments, body 111 may include openings or mounting points for mounting auxiliary sensors and/or auxiliary electrodes, for example, for research purposes. In an example, openings may be defined adjacent or along the mid-line of the head of user 10.

In some embodiments, wearable device 100 may comprise a USB port for attachment of auxiliary components. Auxiliary components can include, for example, auxiliary sensors, auxiliary electrodes, and other auxiliary components that provide additional functionality.

In some embodiments, wearable device 100 can be configured to operate in conjunction with a Continuous Positive Airway Pressure (CPAP) machine. In such embodiments, a nasal cannula flow sensor can provide real time feedback to wearable device 100 which can then transmit the feedback to the CPAP machine or instruct the CPAP machine to modulate its operation. A CPAP machine can be configured to respond dynamically to nasal cannula flow rate data. Such embodiments can make use of other feedback from user 10 to inform the dynamic CPAP machine response.

In some embodiments, the device includes an audio emitter. In some embodiments, the audio emitter is selected from a speaker, a bone conduction transducer, a piezoelectric transducer, or combination thereof.

In various implementations, the wearable device 100 may include a tracker or other sensors, input devices, and output devices. In some embodiments, for example, the tracker is an inertial sensor for measuring movement of the device 100. It detects the 3-dimensional coordinates of the wearable device 100 and accordingly its user's location, orientation or movement. The tracker, for example, comprises one or more accelerometers and/or gyroscopes. The wearable device 100 may comprise a touch sensor for receiving touch input from the user and tactile device for providing vibrational and force feedback to the user.

As shown in FIG. 2J, wearable device 100 may also, in some embodiments, include a vibrational transducer 3254. Waveforms used to generate vibration can be computed locally, for example, in electronics module 32, and modulated remotely, for example, at a local or remote computing device, as describe herein. Thus, conveniently, it may be possible to achieve local rendering with external modulation and control.

In various implementations, the wearable device 100 may include stimulus or feedback components (such as user effectors) to vibrate or provide some audio or visual feedback to user 10. For example, a speaker such as a waveguide speaker may be integrated into body 111 of wearable device 100. A vibro-tactile feedback source may also be integrated into body 111. In some embodiments, a bone conductor transducer may be implemented into body 111.

Stimulus components can also include effectors for any of the senses, including sound, taste, smell, touch and sight, and provide feedback to a user.

In some embodiments, vibrational transducer 3254 is a speaker.

In some embodiments, vibrational transducer 3254 generates physical vibrations.

In some embodiments, vibrational transducer 3254 is a microphone.

In some embodiments, vibrational transducer 3254 is disposed on the body to be adjacent an ear of the user.

In some embodiments, the vibrational transducer is disposed on the body to be adjacent a front of a head of the user. In some embodiments, the vibrational transducer is disposed on the body to be adjacent a bone of the user, which conveniently may pick up more sound transmitted through body as compared to sound transmitted through air.

Wearable device 100 may, in some embodiments, include multiple vibrational transducers for beamforming. In some embodiments, the multiple vibrational transducers are an array of microphones to localize sound from a direction.

As shown in FIG. 2J, in some embodiments, wearable device 100 can include sensors 3260 that can detect a temperature such as a user's temperature. Some embodiments can be configured to detect a relative temperature change. Temperature detection may be carried out using infrared temperature sensors or therm istors.

In some embodiments, a thermistor, which may be flexible, may be integrated as a flexible PCB in body 111 or wearable device 100.

Such a thermistor can be used for detecting relative temperature or changes in temperature. As such, it may be possible to detect changes over time for a user. In some embodiments, it may be possible to calibrate to detect changes in the absolute body heat of an individual.

A thermistor may also be used to detect an ambient temperature in an environment.

Temperature sensing performed by a thermistor may provide useful insight into a user's sleep quality, for example, by correlating temperature with sleep quality, for example, if a user is too hot or too cold to sleep comfortably.

In some embodiments, wearable device 100 may include vibrational transducer 3254 (e.g., a microphone or other audio detection device) that can be used to measure a user's snoring or to detect sleep apnea and timestamp. In some embodiments, bone conduction microphones can be used to detect user's snoring. In some embodiments, microphones mounted on wearable device 100 are configured to focus on sounds originating from user 10.

In some embodiments, wearable device 100 may be configured to detect (e.g., via onboard microphones or other means) when a user is snoring, and send stimulus (e.g., light, sound, electric shock, or vibration) to rouse the user to consciousness. Thus, a user's snoring may be disrupted intentionally. Such disruption may be done in a way that does not seriously disrupt the user's sleep.

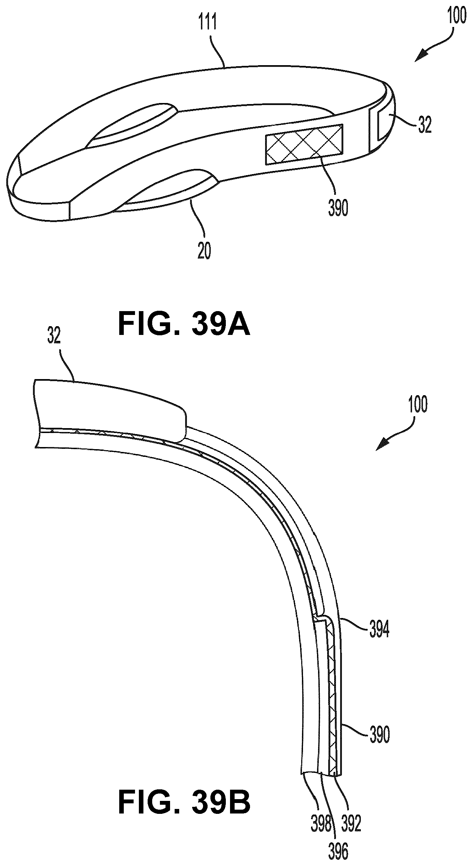

As shown in FIGS. 39A and 39B, in some embodiments, wearable device 100 may include a touchpad location 390. Touchpad location 390 may include a touchpad sensor 392 disposed between a fabric layer 394 and two foam layers 396, 398 and connected to electronics module 32. Touchpad sensor 392 may be used to control various settings of wearable device 100, for example, by way of electronics module 32, such as volume of a sound generating component.

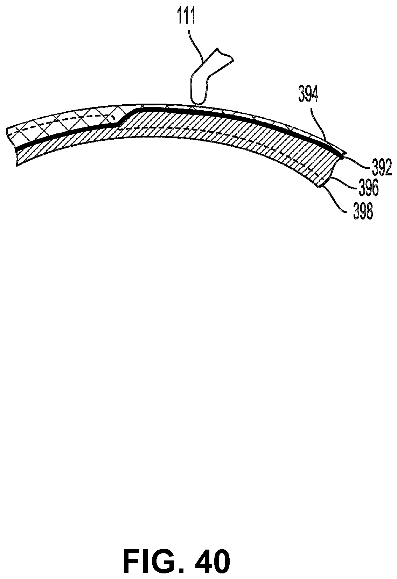

FIG. 40 illustrates a cross-sectional side view of wearable device 100 with touchpad location 390 of FIGS. 39A and 39B, for use, for example, by a finger 400 of user 10. As shown, touchpad sensor 392 may flex between foam layers.

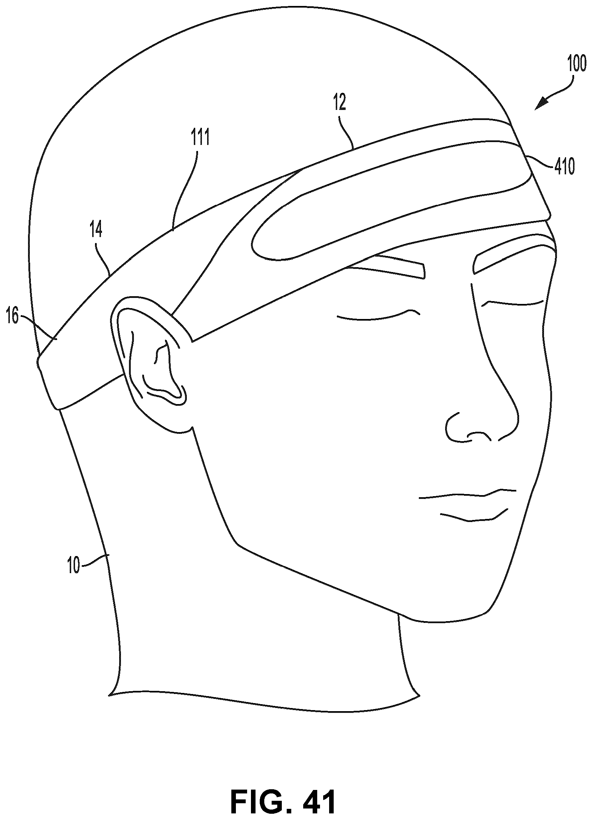

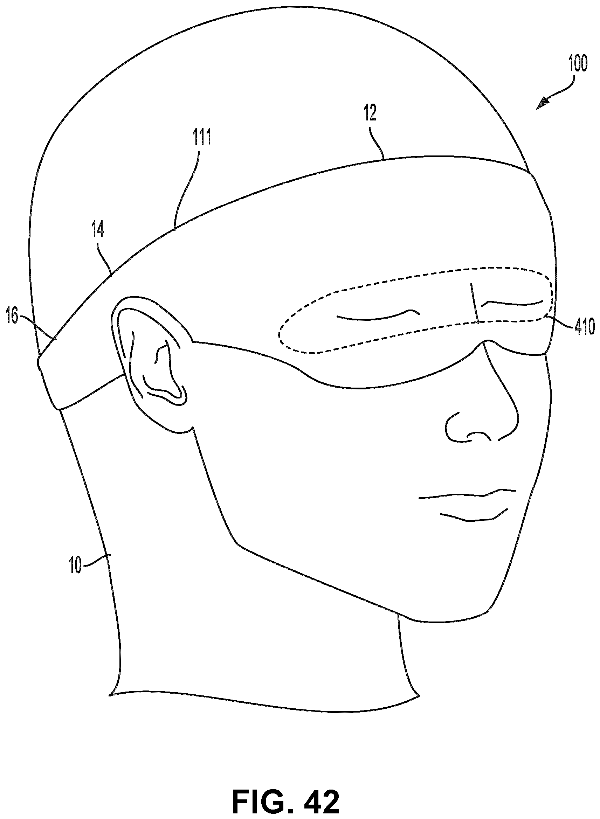

FIG. 41 is a schematic perspective view of wearable device 100 with an extendable, stretchable forehead contacting portion 12 of body 111 in which an organic light-emitting diode (OLED) flexible array 410 may be disposed. As shown in FIG. 42, portion 12, along with OLED flexible array 410, may be folded down for viewing by user 10. OLED flexible array 410 may be integrated into fabric of body 111.

OLED flexible array 410 may provide luminescence to user 10. The luminescence can be used by wearable device 100 to apply light stimulus to user 10. The light stimulus can be applied by single LEDs, a set of LEDs that provide a coloured stimulus, an OLED flexible array 410, or a light emitter that forms an image on the retina of user 10. The light stimulus can be applied in a dynamic manner wherein user 10 has differential luminescence applied across their visual field. In some embodiments this can enable scenes or visual information to be presented to the user. In some embodiments, this can be used to convey complex information to the user. In some embodiments, wearable device 100 is equipped with an eye-tracking sensor and user 10 can engage with a menu presented through OLED flexible array 410 with their eye movements (e.g., user 10 can look at an option and blink to select it).

The dynamic light stimulus offered by OLED flexible array 410 may be used to rouse user 10. For example, OLED flexible array 410 can simulate a sunrise to rouse user 10 to wakefulness in a gentle manner. In other embodiments, if wearable device 100 detects user snoring, then it can apply dynamic light stimulation to rouse the user and stop their snoring without disrupting their sleep. In some embodiments, OLED flexible array 410 can present scenes to train user 10 to fall asleep. In some embodiments, OLED flexible array 410 can present dynamic light stimulus to rouse user 10 while in a dream state to initiate a lucid dreaming session. Dynamic light stimulus can be applied to user 10 and modulated according to an algorithmically adjusted protocol based in part on the bio-signal feedback from user 10, such as a cyclic pattern of light stimulus that has a frequency that is adjusted in relation to the user's neural oscillatory patterns.

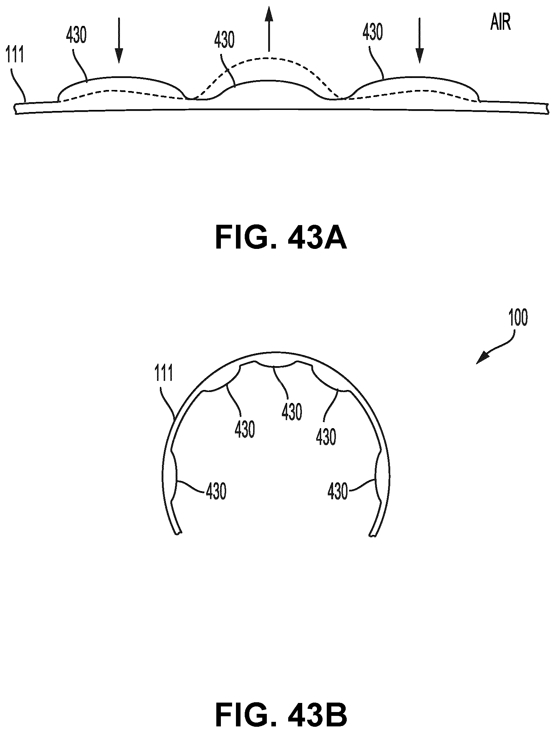

FIG. 43A illustrates a top view of bladders 430 that may be integrated into body 111 of wearable device 100, as shown in FIG. 43B. Bladders 430 may retain gas or fluid, such as air. Bladders 430 may be used to conform wearable device 100 to different areas of the head of user 10. Adding air to certain areas may allow for better contact of electrodes or conductive sensors, such as bio-signal sensors 20, 22, on user 10. Bladders 430 may be controlled by a configuration of valves, and actuated by pressure on the bladder by user 10.

In some embodiments, expanding air in one bladder 430 may reduce air in another section or bladder 430.

In some embodiments, bladders 430 could pulsate to provide a massaging effect on user 10 as wearable device 100 is on the head of user 10.

In some embodiments, wearable device 100 may be configured for synchronization between sensors.

For example, wearable device 100, or associated remote or local computing device(s), can synchronize data capture with light entering a user's eyes, such as illumination changes, or synchronized with sound a user may be hearing.

In another example, a microphone on a local computing device such as a user's mobile phone, maybe used to capture ambient sound and time-stamped. In some embodiments, conventional microphones can be used to capture ambient sound.

In an example, a sample of optical signal received from optical sensor 3216 can be up to 4 kHz and be used to synchronize visual or audio stimulus.

In some embodiments, synchronization can be timed with local events - such as sound intensity and light intensity.

In an example use case, wearable device 100 can be used to generate a feedback loop whereby stimulus is applied to a user, and the user's response is detected using various sensors such as bio-signal sensors 20.

In one example, a vibrating sensor can be used to train user behaviour, such as vibrating when a user is sleeping on their back, or apply electrical stimulus.

In another use case, wearable device 100 can be configured to control a user's environment. For example, wearable device 100 can be used to detect a temperature using thermistor or suitable component and report temperature back to a device to control temperature in room, or detect and/or control a pillow or bed temperature to modulate temperature based on what phase of sleep a user is in.

In an example, using a microphone on a local computing device such as a user's mobile phone, the microphone may detect a user snoring, and cause interruption to try to change that habit. In another example, the environment may be modulated to make a bed hot so user becomes uncomfortable and stops snoring.

Other changes to a user's environment can include changing balance of user so they turn over, connecting to an atomizer to change scent in room (for example, scents used to induce/enhance slow wave), connecting to audio stimulus, or connecting to other devices to control the environment.

Thus, based on bio-signals, wearable device 100 may be used to control an ambient environment.

The physical form factor of wearable device 100 may make it suitable to use while sleeping, and may allow for studying the way people move and their bio-signal data during sleep.

In an aspect, the wearable device may be used to obtain bio-signal data during sleep. For example, a baseline may be established for what is considered an "ideal" sleep. The user's bio-signals may be compared to the baseline to establish a sleep score based on the deviation of the signals from the baseline, such as a deviation of a signal amplitude or a time in which the signal amplitude meets a baseline threshold. In some embodiments, the bio-signal data is timestamped. In some embodiments, the bio-signal acquired during sleep may be used to improve the sleep of the user, for example, by providing a smart wakeup function, arousing the user when they are in a light sleep, or by training the user to sleep better (such as suggesting when a user should sleep based on drowsiness, focus, etc.).

Analysis of such bio-signal and other data may be used to determine a sleep state or score for one or more users.

In some embodiments, a sleep model may be developed for and individual. Such a sleep model may allow for an in-the-moment estimation of what is happening, and how that relates back to the user's model of how the user sleeps, and how model compares to population.

It may thus be possible to make an estimate in a moment on state transition probabilities, for example, between various sleep and awake states, based on a particular user and based on a population of users. A decision on how to intervene may be made based on those inputs.

A direction taken for intervention may be informed by established principles of what is statistically related to a better sleep, and methodologies that have worked in the past.

Such interventions, such as sleep intervention protocols, can be completely data driven (pattern in data) or informed by expert, but can test the diff protocols in ecology of system and use of wearable device 100.

In some embodiments, an up-voting and down-voting system may be implemented to curate the content (for example, protocols) by a community of users.

State transitions may be determined, based on data such as bio-signal data received from bio-signal sensors 20, and used to generate a sleep brain model. Such data may also be compared against a population to generate one or more sleep brain models.

Such sleep brain models can also be applied to a meditative state. For example, a user may be awake or asleep, but could also be awake in a meditative state.

In one embodiment a sleep brain model can be implement as a Markov model.

In some embodiments, wearable device 100 may operate to regularize a user's sleeping patterns. For example, with access to a polysonogram it may be possible to determine what stage of sleep a user is in.

A user may have different ways of sleeping, and to regularize sleeping patterns, it may be necessary to stimulate the user to extend a state of sleep, prompt a user to switch a sleep state, or drop back into a stage of sleep. Certain patterns of sleep are known to be more restful and may improve performance. This may be customized to a particular user or based on a population of users.

Depending on where a user is in journey, intervention will not necessarily be the same, and may be based in part on the user, user's history, other similar users, and used to regularize a sleep pattern based on a stimulus that has been shown to work on other users.

In an example, a state transition probability may be determined of a user's transition between states, for example, from awake to n1, n2. These sleep transitions may be used as a baseline fingerprint for how a user sleeps and to develop a sleep transition model, a sleep model or sleep brain model. Such a model may be compared to a state transition model with other people, and provide an indication of when to intervene.

In some embodiments, wearable device 100 may be used to develop a sleep algorithmic protocol and track a user's sleep properties, such as rapid eye movement.

In an example, wearable device 100 may be used as a sleep monitor or aid to train a user to a rhythm as a sleep aid. It may be possible to entrain brainwaves in the 13-15 Hz range, allowing the user to fall asleep faster at night and maintain some declarative memory improvement.

In some embodiments, wearable device 100 may be used for entrainment with a biological feedback system. This may not be limited to only brain state, but may examine other bio-signals (e.g., heart rate) as described herein.

In an example, a phase lock loop (measuring phase alignment) may be used to influence a user's state, and a phase lock loop built with a user in the loop.

In some embodiments, a user can be "entrained" to sleep, or audio or other stimulus can be used to help "train" a user to sleep (for e.g., by using a mantra, which may be repeating).

Such entrainment may be based in part on a sensed body or head position or orientation of a user.

Measurements may be taken of many signals (brain, heart) and then control/stimulus for one or more of the bio-signals (e.g. joint space of heart/brain entrainment). Sensor measurements may be taken across multiple bio-signals and bio-signal types, and is not just limited to brain, and can be breath, heart, mouth, and the like.

In an example, a user's breath may impact heart/brain. Thus, if entraining one bio-signal, it may have an impact on another bio-signal, for example, entrain heartrate, and brain state ends up being affected as well - e.g., use breath to influence heart, which affects brain.

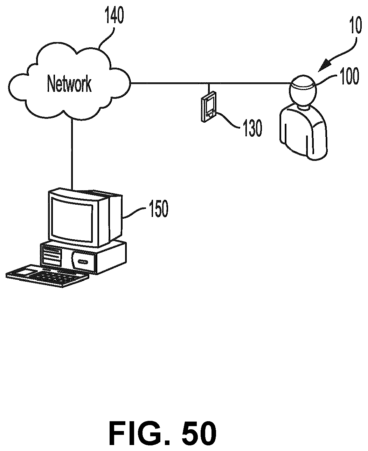

As illustrated in FIG. 50, wearable device 100 or sensors, such as one or more of bio-signal sensors 20 of wearable device 100 can be in communication with a local computing device 130, for example by way of communication protocols such as Bluetooth, Wi-Fi, LTE or 5G networks or other suitable communication protocol. Local device 130 is in communication with a remote computing device 150, by way of a network 140.

Network 140 may, for example, be a packet-switched network, in the form of a LAN, a WAN, the public Internet, a Virtual Private Network (VPN) or the like.

Local computing device 130, may be, for example, a mobile device. Example mobile devices include without limitation, cellular phones, cellular smartphones, wireless organizers, pagers, personal digital assistants, computers, laptops, handheld wireless communication devices, wirelessly enabled notebook computers, portable gaming devices, tablet computers, or any other portable electronic device with processing and communication capabilities. In at least some embodiments, mobile devices as referred to herein can also include without limitation, peripheral devices such as displays, printers, touchscreens, projectors, digital watches, cameras, digital scanners and other types of auxiliary devices that may communicate with another computing device.

In some embodiments, wearable device 100 comprises communicator 3230 to transmit data to a computing device. In some embodiments the computing device is local computing device 130. In some embodiments, the computing device is remote computing device 150.

In some embodiments, communicator 3230 communicates with the computing device on a Bluetooth communications protocol.

In some embodiments, communicator 3230 communicates with the computing device on a Wi-Fi communications protocol.

In one example, local computing device 130 may be a smartphone. In another example, local computing device 130 may be a touchscreen enabled device and the other a type of communication device (e.g., a router) for connecting to other devices. As will be apparent, other types of computing devices can be envisaged that benefit from interconnection and interoperability.

Remote computing device 150 may be a computing device, for example, a cloud computing device, connected by way of network 140 to wearable device 100 and/or local computing device 130 to perform any of the functionality described herein.

In some embodiments, wearable device 100 can be configured to receive user inputs through local computing device 130. In these embodiments the user can customize the functionality afforded by wearable device 100 by manipulating local computing device 130. For example, a user can select the types of stimulus that wearable device 100 will apply and to which events wearable device 100 will trigger the stimulus. In some embodiments, the user can select or deselect certain functionalities (e.g., snore mitigation, lucid dreaming assistance, and sleep assistance) that can be offered by wearable device 100. In other embodiments, the user can record words or phrases on local computing device 130 to be used as stimulus by wearable device 100.

In some embodiments, wearable device 100 can be configured to act as an interface for local computing device 130. Local computing device 130 can be configured to respond to voluntary user actions (e.g., eye movements) and involuntary user actions (e.g., detecting when user 10 needs to focus and, for example, lowering a volume on a device). In some embodiments, all modes of interfacing with wearable device 100 can be used to interface with local computing device 130.

In some embodiments, wearable device 100 can monitor a cognitive load of user 10. In some applications, such as in sports, piloting an aircraft, or performing surgery, brief lapses in focus can be catastrophic. In some embodiments, wearable device 100 can be configured to detect when a user is engaging in an activity that demands a high cognitive load and dynamically adjust the environment to diminish distraction (e.g., temporarily suspending notifications to local computing device 130). In embodiments involving alternate reality or virtual reality experiences (e.g., through wearable device 100 or local computing device 130), wearable device 100 can diminish distractions by modulating the environment (e.g., diminishing the prominence of distracting visual stimuli). In some embodiments, wearable device 100 can mask distracting auditory stimuli by applying noise cancelling techniques or by shrouding the sound (e.g., shrouding a distracting conversation in ambient conversational noise).

In some embodiments, multiple wearable devices 100 can be configured to communicate with each other through network 140. In such embodiments, wearable devices 100 can be configured to synchronize users not proximate to each other. Synchronization can be achieved by applying similar stimulus to the disparate users or applying a similar stimulus protocols that can be implemented to achieve a synchronous target state in the disparate users. Such embodiments can be used by long distance couples to simulate the experience of being together. Such embodiments can optionally permit the disparate users to communicate with one another visually and / or verbally.

In some embodiments, multiple wearable devices 100 can be configured to provide feedback on a group of users. These embodiments can be used to monitor the productivity or creativity of a group of users. In some embodiments, the system of wearable devices can signal to the group (e.g., through a light or audio cue, or through an external device) that the group needs to take a break.

In some embodiments, multiple wearable devices 100 can communicate with a local computing device 130 that is providing entertainment to a group of users. Local computing device 130 can modulate the content presented based on the aggregate feedback from the users. For example, a television can modulate the plot of a show based on the bio-signal feedback from the users wearing wearable devices 100.

In some embodiments, wearable devices 100 can communicate a current state of a user to others (e.g., bored, aroused, or distressed). In some embodiments, this information can be communicated to the group (e.g., through light indications on wearable device 100). In other embodiments where a particular member of a group is orchestrating a group experience (e.g., a DJ, a host, or a virtual shaman) then this information can be communicated to that particular person only (e.g., through remote computing device 150).

In some embodiments, wearable device 100 can be used to detect a user state and predictively group a user with other individuals (e.g., open a channel of communication between the user and other individual or place the user in a virtual setting with the other individual). The predictive placement could be based in part on the individual's current state and the state to which the individual is striving.

In some embodiments, wearable device 100 can be used to predict student-teacher groupings that will result in effective education for the student. Such groupings can be predicted based in part on the user states of the student and teacher and their historic data based on past observation.

In some embodiments, wearable devices 100 can aggregate information from disparate users through network 140 and provide information about demographic bio-signal feedback data. Such feedback can be provided through, for example, local computing device 130. For example, such a system can communicate that, on average, everyone in a city is happier today than yesterday (e.g., "NYC is happy today").

In some embodiments, wearable device 100 can be implemented in a clinical setting. Wearable device 100 can provide a means of remotely monitoring a patient. Such remote monitoring can provide the caretaker or practitioner with detailed feedback (e.g., through a remote computing device) or simple indications of the patient's state (e.g., an external device that provides audio or visual cues to indicate state). In these embodiments, wearable device 100 can be used in the diagnosis, treatment, and monitoring of disorders (e.g., multiple sclerosis, depression, anxiety, attention deficit hyperactive disorder, sleep disorders, neurotoxicity, stoke, traumatic brain injury, epilepsy). Wearable device 100 can also be used in monitoring ongoing conditions or situations (e.g., medication management, pregnancy, addiction management).

In some embodiments, wearable device 100 can be configured to provide fast and reliable emergency diagnoses for emergency medical responders. Such embodiments can be configured to operate and provide feedback using only wearable device 100 or can be integrated into the medical infrastructure of a hospital or ambulance system.

Some embodiments of wearable device 100 are configured for use with infants or young children for child monitoring systems. The system can further comprise an external device that can indicate the present state of the child user to a parent or caregiver. Such external devices can provide audio or visual indications of sleep or wellbeing state (e.g., a light that gets brighter when the child user is sleeping well). In some embodiments, wearable device 100 can detect the sleep state of the child user and communicate to the parent when the child user is experiencing emotional events that may precede sleep disturbances. This can inform the parent or caregiver that the child user requires comfort or silence to avoid a sleep disturbance.

Such systems can implement infant or child specific protocols in state analysis. Such systems may be effective in detecting infant or child specific disorders (e.g., sudden infant death syndrome).

In some embodiments, wearable device 100 can be used to monitor a user with epilepsy. Wearable device 100 can be configured to predict and warn the user when a seizure is incoming. In some embodiments, wearable device 100 can apply stimulus to user 10 in an attempt to prevent a seizure. In some embodiments, wearable device 100 can be configured to warn external devices of an impending seizure (e.g., a caregiver monitoring device or a vehicle the user is then operating).

In some embodiments, wearable device 100 can be implemented in modular treatment delivery. In these embodiments, wearable device 100 can monitor feedback from user 10. In some embodiments, wearable device 100 can detect when glucose levels are down for users with artificial pancreases and modulate the users' glucose levels accordingly. Such modulations can help ensure restful sleep.

In the clinical setting, wearable device 100 can be in communication with remote computing device 150 forming a clinical system. The clinical system can further comprise external medical devices that can send and receive information to and from wearable device 100 and remote computing device 150. For example, wearable device 100 can detect and transmit feedback from user 10 to external medical devices that can act on this feedback. Alternatively, external medical devices can detect feedback from user 10 and transmit this information to wearable device 100 which can apply stimulus to user 10 (e.g., applying light stimulus to rouse a user from a sleep state).

In embodiments, wearable device 100 can be configured to provide feedback to an external caregiver. Such feedback can be provided through remote computing device 150. Alternatively feedback can be provided to a caregiver through light or sound emissions by wearable device 100. In some embodiments, wearable device 100 can trigger an alarm through an alert system when user 10 is in crisis or exhibiting feedback patterns indicative of imminent crisis. In some embodiments, wearable device 100 can warn a caregiver when user 10 is experiencing distress or exhibiting feedback indicating that they might need caregiver assistance.

Wearable device 100 can be equipped with an emergency call button. An emergency call button can alert a caregiver or practitioner to user-experienced threats. Wearable device 100 can be configured to open a line of communication between the user and a caregiver or practitioner. The emergency call button can be configured to alert emergency services to an emergency.

In some embodiments, wearable device 100 can be implemented in a system configured to annotate feedback data for ease of review. Such systems can further comprise remote computing device 150. In these embodiments, the system can receive bio-signal feedback from user 10 and annotate the feedback in real time. Such annotations can be driven by static protocols or by dynamically adaptive protocols that respond to user bio-feedback feedback. The system can further comprise a method of manual annotation to indicate when an event took place (e.g., administration of treatment) or when a subjective state was experienced (e.g., pain or effective treatment). In some embodiments using dynamically adaptive protocols, the protocol can accept and adapt to manual annotations and, in some embodiments, learn to make such annotations automatically.

Manual annotation can be used to make subjective user experience objective. For example, users can annotate feedback with subjective pain experiences. A system implementing wearable device 100 can detect and monitor bio-signal feedback from the user. The system can process and evaluate the bio-signal feedback to analyze biomarkers suitable for objective proxies of the subjective user experience. The determination of such biomarkers can be useful for research and / or treatment of a variety of conditions.

Uses of such system can be effective in the treatment and monitoring of disorders that can be characterized in part by sleep disturbances (such as multiple sclerosis, depression, anxiety, attention deficit hyperactive disorder, sleep disorders, neurotoxicity, stoke, or traumatic brain injury). Such systems can also be useful for monitoring patients with chronic pain, hypertension, diabetes, obesity, chronic obstructive pulmonary disease, or those undergoing chemotherapy. Such systems may be useful in treatment and monitoring in the fields of obstetrics, urology, endocrinology, and others.

The system can undergo machine learning to diagnose various conditions based on characteristic user bio-signal feedback. The system can be used in treatment by dynamically learning user responses to certain stimuluses and applying said stimuluses to guide a user through healthy experiences (e.g., applying stimulus during sleep to interrupt disordered sleeping behaviors). The system can be used in monitoring conditions by comparing a user state based on the user's bio-signal feedback to a typical state pattern exhibited by others with similar conditions and alert caregivers or practitioners of significantly deviant states. Such systems can dynamically adapt to and learn from a user as wearable device 100 observes the user.

Such systems can be useful in pain mitigation treatments wherein they can give the user and / or practitioner objective metrics to judge the subjective distress the user is experiencing. Such insights can help a user determine if and when a user will self-administer pain medication. Such systems can also connect the user with other users in a support group capacity. Similar systems can be used addiction management.

In some embodiments, the system can guide a user (or a group of users) through a therapeutic altered consciousness experience (e.g., a psychedelic experience, meditation, or a mystical experience). In these embodiments, the system can allow a user to conduct emotional processing in a safe and effective manner. In some embodiments, the system can monitor the user's current state based on their bio-signal feedback and provide the feedback to a human guide. In some embodiments, the guide can monitor user 10 through remote computing device 150. In some embodiments, user 10 can undertake the experience in their home.

In some embodiments, wearable device 100 can calibrate to a base state of user 10 (e.g., based on a meditative state of the user). Wearable device 100 can set this base state as the target state if wearable device 100 detects that user 10 is experiencing emotional distress above an allowable threshold.

In some embodiments, wearable device 100 can be programmed with a target protocol experience in mind that can define a typical user experience when engaging with a therapeutic altered consciousness experience. The target protocol can include target user states that change with time (the protocol arch). This protocol can further adapt and modulate based on bio-signal feedback from the user while the user is engaging with the therapeutic altered consciousness experience. The adaptive protocol can define what physical and / or emotional responses from user 10 are typical, atypical, or cause for concern. The protocol can determine what emotional experiences are too distressing for user 10 and can, in some embodiments, alert an external guide or, in some embodiments, apply stimuluses to user 10 to bring them to a base state. In some embodiments, a representation of the protocol arch can be communicated to the user along with an indication of where along the arch said user is.

In embodiments where the therapeutic altered consciousness experience is facilitated in part by a psychoactive therapeutic, wearable device 100 can detect if and when a user requires a higher dose of the therapeutic based in part on the bio-signal feedback of the user. In some embodiments, wearable device 100 can instruct an external or integrated dosing device to increase the dose administered to a user.

In some embodiments, wearable device 100 can record the bio-signal feedback data of a therapeutic altered consciousness experience of user 10. This recorded data can be selected by user 10 to replicate by wearable device 100. For example, if user 10 experienced a Nirvana state, then user 10 could use the recording of that session to generate a target protocol to guide a future therapeutic altered consciousness experience back to the Nirvana state.

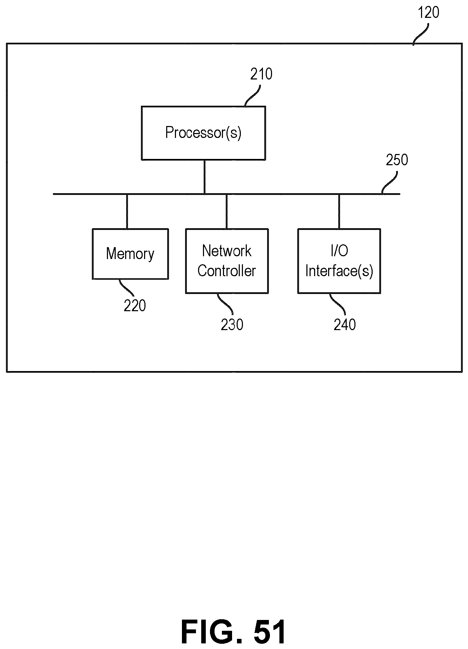

One or more of wearable device 100, local device 130, or remote device 150 can implement a computing device, for example, a computing device 120 as illustrated in FIG. 51.

Systems and methods described herein may be implemented as software and/or hardware, for example, performed by one or more computing device 120 on one or more of wearable device 100, local computing device 130, or remote computing device 150.

As illustrated, computing device 120 includes one or more processor(s) 210, memory 220, a network controller 230, and one or more I/O interfaces 240 in communication over bus 250.

Processor(s) 210 may be one or more Intel x86, Intel x64, AMD x86-64, PowerPC, ARM processors or the like.

Memory 220 may include random-access memory, read-only memory, or persistent storage such as a hard disk, a solid-state drive or the like. Read-only memory or persistent storage is a computer-readable medium. A computer-readable medium may be organized using a file system, controlled and administered by an operating system governing overall operation of the computing device.

Network controller 230 serves as a communication device to interconnect the computing device with one or more computer networks such as, for example, a local area network (LAN) or the Internet.

One or more I/O interfaces 240 may serve to interconnect the computing device with peripheral devices, such as for example, keyboards, mice, video displays, and the like. Such peripheral devices may include a display of device 120. Optionally, network controller 230 may be accessed via the one or more I/O interfaces.

Software instructions are executed by processor(s) 210 from a computer-readable medium. For example, software may be loaded into random-access memory from persistent storage of memory 220 or from one or more devices via I/O interfaces 240 for execution by one or more processors 210. As another example, software may be loaded and executed by one or more processors 210 directly from read-only memory.

Example software components and data stored within memory 220 of computing device 120 may include software to apply bio-signal analysis, as described herein, and operating system (OS) software allowing for basic communication and application operations related to computing device 120.

It will be appreciated that any module or component exemplified herein that executes instructions may include or otherwise have access to computer readable media such as storage media, computer storage media, or data storage devices (removable and/or non-removable) such as, for example, magnetic disks, optical disks, tape, and other forms of computer readable media. Computer storage media may include volatile and non-volatile, removable and non-removable media implemented in any method or technology for storage of information, such as computer readable instructions, data structures, program modules, or other data. Examples of computer storage media include RAM, ROM, EEPROM, flash memory or other memory technology, CD-ROM, digital versatile disks (DVD), blue-ray disks, or other optical storage, magnetic cassettes, magnetic tape, magnetic disk storage or other magnetic storage devices, or any other medium which can be used to store the desired information and which can be accessed by an application, module, or both. Any such computer storage media may be part of the mobile device, tracking module, object tracking application, etc., or accessible or connectable thereto. Any application or module herein described may be implemented using computer readable/executable instructions that may be stored or otherwise held by such computer readable media.

Thus, alterations, modifications and variations can be effected to the particular embodiments by those of skill in the art without departing from the scope of this disclosure, which is defined solely by the claims appended hereto.

In further aspects, the disclosure provides systems, devices, methods, and computer programming products, including non-transient machine-readable instruction sets, for use in implementing such methods and enabling the functionality described previously.

Although the disclosure has been described and illustrated in exemplary forms with a certain degree of particularity, it is noted that the description and illustrations have been made by way of example only. Numerous changes in the details of construction and combination and arrangement of parts and steps may be made. Accordingly, such changes are intended to be included herein, the scope of which is defined by the claims.

Except to the extent explicitly stated or inherent within the processes described, including any optional steps or components thereof, no required order, sequence, or combination is intended or implied. As will be understood by those skilled in the relevant arts, with respect to both processes and any systems, devices, etc., described herein, a wide range of variations is possible, and even advantageous, in various circumstances, without departing from the scope of the disclosure, which is to be limited only by the claims.

Of course, the above described embodiments are intended to be illustrative only and in no way limiting. The described embodiments are susceptible to many modifications of form, arrangement of parts, details and order of operation. The disclosure is intended to encompass all such modification within its scope, as defined by the claims.

## Claims

1. A wearable device comprising:
a flexible and extendable body configured to encircle a portion of a user;
an electronics module having a surface defining a concave space between a first end and a second end opposite the first end,
the first end attachable to the flexible and extendable body at a first connection point with a first flexible retention mount that provides flexibility to allow rotation of the flexible and extendable body relative to the electronics module about a first axis,
the second end attachable to the flexible and extendable body at a second connection point with a second flexible retention mount that provides flexibility to allow rotation of the flexible and extendable body relative to the electronics about a second axis,
wherein the first connection point and second connection point are positioned at a distance apart on the flexible and extendable body such that when the electronics module is received by the flexible and extendable body, the first connection point and second connection point generate tension in a portion of the flexible and extendable body between the first connection point and the second connection point; and
a bio-signal sensor disposed on the portion of the flexible and extendable body between the first connection point and the second connection point to contact at least a part of the user and to receive bio-signals from the user,
when worn by the user with the electronics module attached, the portion of the flexible and extendable body between the first connection point and the second connection point is configured to rotate towards the concave space, and the tension is configured to urge the bio-signal sensor on the portion of the flexible and extendable body against the at least a part of the user; and
the electronics module comprising a processor for receiving the bio-signals from the bio-signal sensor.

2. The wearable device of claim 1, wherein the bio-signal sensor is an electroencephalography (EEG) sensor configured to measure or generate electrical potential; and wherein the bio-signal sensor is configured to contact at least part of a frontal region of a head of the user.

3. The wearable device of any one of claims 1 to 2, further comprising an additional bio-signal sensor to contact at least part of an auricular region of a head of the user; wherein the additional bio-signal sensor is an electroencephalography (EEG) sensor.

4. The wearable device of any one of claims 1 to 3, further comprising an electrical connection between the electronics module and at least one of the flexible and extendable body and the bio-signal sensor.

5. The wearable device of any one of claims 1 to 4, wherein the electronics module is curved to generally correspond to a head of the user.

6. The wearable device of any one of claims 1 to 5, wherein the electronics module is attachable to the flexible and extendable body by a magnetic force.

7. The wearable device of any one of claims 1 to 6, wherein the electronics module includes a first magnet at the first end to attach to the first flexible retention mount by magnetic force and a second magnet at the second end to attach to the second flexible retention mount by magnetic force.

8. The wearable device of any one of claims 1 to 7, further comprising optical sensor disposed on the electronics module; wherein the optical sensor detects at least one of:
a compression of the body based at least in part on a detected reflection distance of light reflected into the optical sensor; and
additional bio-signals based at least in part on at least one of a detected reflection distance of light reflected into the optical sensor and a measured colour and intensity of light reflected into the optical sensor.

9. The wearable device of any one of claims 1 to 8, wherein the flexible and extendable body comprises a compressible section adjacent to the bio-signal sensor to compress to conform the at least one bio-signal sensor to the portion of the user; wherein the compressible section is shaped to conform to the at least part of the portion of the user; and wherein the compressible section comprises foam having variable density.

10. The wearable device of any one of claims 1 to 9, further comprising at least one of a light emitter and a light receiver disposed on the flexible and extendable body adjacent an eye of the user to detect light adjacent the eye of the user.

11. The wearable device of any one of claims 1 to 10, further comprising a vibrational transducer comprising at least one of a speaker, a microphone, and a physical vibration generator;
wherein the vibrational transducer is disposed on the flexible and extendable body to be adjacent to at least one of an ear of the user, a front of a head of the user, and a bone of the user.

12. The wearable device of any one of claims 1 to 11, further comprising multiple vibrational transducers for beamforming; wherein the multiple vibrational transducers are an array of microphones to localize sound from a direction.

13. The wearable device of any one of claims 1 to 12, further comprising at least one of an accelerometer to detect movement of the user and a thermistor to detect a relative temperature change.

14. The wearable device of any one of claims 1 to 13, further comprising a communicator to transmit data to a computing device; wherein the communicator communicates with the computing device using at least one of a Bluetooth communications protocol and a Wi-Fi communications protocol.

## Patentansprüche

1. Tragbare Vorrichtung, umfassend:
einen flexiblen und erweiterbaren Körper, der dazu konfiguriert ist, einen Teil eines Benutzers zu umgeben;
ein Elektronikmodul mit einer Oberfläche, die einen konkaven Raum zwischen einem ersten Ende und einem zweiten Ende gegenüber dem ersten Ende definiert,
wobei das erste Ende an einem ersten Verbindungspunkt mit einer ersten flexiblen Haltebefestigung, die Flexibilität bereitstellt, um eine Drehung des flexiblen und erweiterbaren Körpers relativ zu dem Elektronikmodul um eine erste Achse zu ermöglichen, an den flexiblen und erweiterbaren Körper anbringbar ist,
wobei das zweite Ende an einem zweiten Verbindungspunkt mit einer zweiten flexiblen Haltebefestigung, die Flexibilität bereitstellt, um eine Drehung des flexiblen und erweiterbaren Körpers relativ zu der Elektronik um eine zweite Achse zu ermöglichen, an den flexiblen und erweiterbaren Körper anbringbar ist,
wobei der erste Verbindungspunkt und der zweite Verbindungspunkt auf dem flexiblen und erweiterbaren Körper beabstandet positioniert sind, sodass, wenn das Elektronikmodul vom flexiblen und erweiterbaren Körper aufgenommen wird, der erste Verbindungspunkt und der zweite Verbindungspunkt Spannung in einem Teil des flexiblen und erweiterbaren Körpers zwischen dem ersten Verbindungspunkt und dem zweiten Verbindungspunkt erzeugen; und
einen Biosignalsensor, der auf dem Teil des flexiblen und erweiterbaren Körpers zwischen dem ersten Verbindungspunkt und dem zweiten Verbindungspunkt angeordnet ist, um zumindest einen Teil des Benutzers zu kontaktieren und Biosignale vom Benutzer zu empfangen, wobei, wenn vom Benutzer mit angebrachtem Elektronikmodul getragen, der Teil des flexiblen und erweiterbaren Körpers zwischen dem ersten Verbindungspunkt und dem zweiten Verbindungspunkt konfiguriert ist, sich zu dem konkaven Raum zu drehen, und die Spannung konfiguriert ist, den Biosignalsensor auf dem Teil des flexiblen und erweiterbaren Körpers gegen den zumindest einen Teil des Benutzers zu drängen; und
wobei das Elektronikmodul einen Prozessor zum Empfangen der Biosignale vom Biosignalsensor umfasst.

2. Tragbare Vorrichtung nach Anspruch 1, wobei der Biosignalsensor ein Elektroenzephalographie(EEG)-Sensor ist, der konfiguriert ist, elektrisches Potenzial zu messen oder zu erzeugen; und wobei der Biosignalsensor konfiguriert ist, zumindest einen Teil einer Stirnregion des Kopfes des Benutzers zu kontaktieren.

3. Tragbare Vorrichtung nach einem der Ansprüche 1 bis 2, ferner umfassend einen zusätzlichen Biosignalsensor zum Kontaktieren zumindest eines Teils einer Ohrenregion des Kopfes des Benutzers; wobei der zusätzliche Biosignalsensor ein Elektroenzephalographie(EEG)-Sensor ist.

4. Tragbare Vorrichtung nach einem der Ansprüche 1 bis 3, ferner umfassend eine elektrische Verbindung zwischen dem Elektronikmodul und dem flexiblen und erweiterbaren Körper und/oder dem Biosignalsensor.

5. Tragbare Vorrichtung nach einem der Ansprüche 1 bis 4, wobei das Elektronikmodul gekrümmt ist, um allgemein dem Kopf des Benutzers zu entsprechen.

6. Tragbare Vorrichtung nach einem der Ansprüche 1 bis 5, wobei das Elektronikmodul an dem flexiblen und erweiterbaren Körper mittels Magnetkraft anbringbar ist.

7. Tragbare Vorrichtung nach einem der Ansprüche 1 bis 6, wobei das Elektronikmodul einen ersten Magneten am ersten Ende zum Anbringen der ersten flexiblen Haltebefestigung mittels Magnetkraft und einen zweiten Magneten am zweiten Ende zum Anbringen der zweiten flexiblen Haltebefestigung mittels Magnetkraft beinhaltet.

8. Tragbare Vorrichtung nach einem der Ansprüche 1 bis 7, ferner umfassend einen optischen Sensor, der auf dem Elektronikmodul angeordnet ist; wobei der optische Sensor mindestens eines von Folgendem detektiert:
ein Zusammendrücken des Körpers basierend zumindest teilweise auf einer detektierten Reflexionsdistanz von Licht, das im optischen Sensor reflektiert wird; und
zusätzliche Biosignale basierend zumindest teilweise auf einer detektierten Reflexionsdistanz von Licht, das im optischen Sensor reflektiert wird, und/oder einer gemessenen Farbe und Intensität von Licht, das im optischen Sensor reflektiert wird.

9. Tragbare Vorrichtung nach einem der Ansprüche 1 bis 8, wobei der flexible und erweiterbare Körper einen zusammendrückbaren Abschnitt benachbart zu dem Biosignalsensor umfasst, zum Zusammendrücken, um den mindestens einen Biosignalsensor an den Teil des Benutzers anzupassen; wobei der zusammendrückbare Abschnitt so geformt ist, dass er dem zumindest Teil des Teils des Benutzers entspricht; und wobei der zusammendrückbare Abschnitt Schaumstoff mit variabler Dichte umfasst.

10. Tragbare Vorrichtung nach einem der Ansprüche 1 bis 9, ferner umfassend einen Lichtemitter und/oder einen Lichtempfänger, die auf dem flexiblen und erweiterbaren Körper neben einem Auge des Benutzers angeordnet sind, um Licht neben dem Auge des Benutzers zu detektieren.

11. Tragbare Vorrichtung nach einem der Ansprüche 1 bis 10, ferner umfassend einen Schwingungswandler, der einen Lautsprecher und/oder ein Mikrofon und/oder einen physikalischen Schwingungsgenerator umfasst;
wobei der Schwingungswandler auf dem flexiblen und erweiterbaren Körper angeordnet ist, um sich neben einem Ohr des Benutzers und/oder einer Vorderseite des Kopfes des Benutzers und/oder einem Knochen des Benutzers zu befinden.

12. Tragbare Vorrichtung nach einem der Ansprüche 1 bis 11, ferner umfassend mehrere Schwingungswandler zur Strahlformung; wobei es sich bei den mehreren Schwingungswandlern um ein Array von Mikrofonen handelt, um Ton aus einer Richtung zu lokalisieren.

13. Tragbare Vorrichtung nach einem der Ansprüche 1 bis 12, ferner umfassend einen Beschleunigungsmesser zum Detektieren einer Bewegung des Benutzers und/oder einen Thermistor zum Detektieren einer relativen Temperaturänderung.

14. Tragbare Vorrichtung nach einem der Ansprüche 1 bis 13, ferner umfassend einen Kommunikator zum Übertragen von Daten an eine Rechenvorrichtung; wobei der Kommunikator mit der Rechenvorrichtung unter Verwendung eines Bluetooth-Kommunikationsprotokolls und/oder eines WiFi-Kommunikationsprotokolls kommuniziert.

## Revendications

1. Dispositif portable comprenant :
un corps flexible et extensible configuré pour entourer une partie d'un utilisateur ;
un module électronique ayant une surface définissant un espace concave entre une première extrémité et une deuxième extrémité opposée à la première extrémité,
la première extrémité pouvant être fixée au corps flexible et extensible en un premier point de connexion à l'aide d'un premier support de retenue flexible qui offre une flexibilité permettant la rotation du corps flexible et extensible par rapport au module électronique autour d'un premier axe,
la deuxième extrémité pouvant être fixée au corps flexible et extensible au niveau d'un deuxième point de connexion à l'aide d'un deuxième support de retenue flexible qui offre une flexibilité permettant la rotation du corps flexible et extensible par rapport au module électronique autour d'un deuxième axe,
où le premier point de connexion et le deuxième point de connexion sont positionnés à une certaine distance l'un de l'autre sur le corps flexible et extensible de telle sorte que lorsque le module électronique est reçu par le corps flexible et extensible, le premier point de connexion et le deuxième point de connexion génèrent une tension dans une partie du corps flexible et extensible entre le premier point de connexion et le deuxième point de connexion ; et
un capteur de signaux biologiques disposé sur la partie du corps flexible et extensible entre le premier point de connexion et le deuxième point de connexion pour entrer en contact avec au moins une partie de l'utilisateur et recevoir des signaux biologiques de l'utilisateur,
lorsqu'elle est portée par l'utilisateur avec le module électronique fixé, la partie du corps flexible et extensible entre le premier point de connexion et le deuxième point de connexion est configurée pour tourner vers l'espace concave, et la tension est configurée pour pousser le capteur de signaux biologiques sur la partie du corps flexible et extensible contre au moins une partie de l'utilisateur ; et
le module électronique comprenant un processeur pour recevoir les signaux biologiques provenant du capteur de signaux biologiques.

2. Dispositif portable selon la revendication 1, dans lequel le capteur de signaux biologiques est un capteur d'électroencéphalographie (EEG) configuré pour mesurer ou générer un potentiel électrique ; et dans lequel le capteur de signaux biologiques est configuré pour entrer en contact avec au moins une partie d'une région frontale de la tête de l'utilisateur.

3. Dispositif portable selon l'une quelconque des revendications 1 et 2, comprenant en outre un capteur de signaux biologiques supplémentaire destiné à entrer en contact avec au moins une partie d'une région auriculaire de la tête de l'utilisateur ; où le capteur de signaux biologiques supplémentaire est un capteur d'électroencéphalographie (EEG).

4. Dispositif portable selon l'une quelconque des revendications 1 à 3, comprenant en outre une connexion électrique entre le module électronique et au moins l'un parmi le corps flexible et extensible et le capteur de signaux biologiques.

5. Dispositif portable selon l'une quelconque des revendications 1 à 4, dans lequel le module électronique est courbé pour correspondre globalement à la tête de l'utilisateur.

6. Dispositif portable selon l'une quelconque des revendications 1 à 5, dans lequel le module électronique peut être fixé au corps flexible et extensible par une force magnétique.

7. Dispositif portable selon l'une quelconque des revendications 1 à 6, dans lequel le module électronique comprend un premier aimant à la première extrémité pour se fixer au premier support de retenue flexible par une force magnétique et un deuxième aimant à la deuxième extrémité pour se fixer au deuxième support de retenue flexible par une force magnétique.

8. Dispositif portable selon l'une quelconque des revendications 1 à 7, comprenant en outre un capteur optique disposé sur le module électronique, où le capteur optique détecte au moins l'un des éléments suivants :
une compression du corps basée au moins en partie sur une distance de réflexion détectée de la lumière réfléchie dans le capteur optique ; et
des signaux biologiques supplémentaires basés au moins en partie sur au moins l'une parmi une distance de réflexion détectée de la lumière réfléchie dans le capteur optique et une couleur et une intensité mesurées de la lumière réfléchie dans le capteur optique.

9. Dispositif portable selon l'une quelconque des revendications 1 à 8, dans lequel le corps flexible et extensible comprend une section compressible adjacente au capteur de signaux biologiques afin de se comprimer pour conformer l'au moins un capteur de signaux biologiques à la partie de l'utilisateur ; où la section compressible est mise en forme pour se conformer à l'au moins une partie de la partie de l'utilisateur ; et où la section compressible comprend une mousse ayant une densité variable.

10. Dispositif portable selon l'une quelconque des revendications 1 à 9, comprenant en outre au moins l'un parmi un émetteur de lumière et un récepteur de lumière disposés sur le corps flexible et extensible, adjacents à un œil de l'utilisateur, afin de détecter la lumière adjacente à l'œil de l'utilisateur.

11. Dispositif portable selon l'une quelconque des revendications 1 à 10, comprenant en outre un transducteur vibratoire comprenant au moins l'un d'un haut-parleur, d'un microphone et d'un générateur de vibrations physiques ;
où le transducteur vibratoire est disposé sur le corps flexible et extensible de manière à être adjacent à au moins une partie parmi une oreille de l'utilisateur, le front de la tête de l'utilisateur et un os de l'utilisateur.

12. Dispositif portable selon l'une quelconque des revendications 1 à 11, comprenant en outre plusieurs transducteurs vibratoires pour la formation de faisceaux, où les multiples transducteurs vibratoires sont un réseau de microphones permettant de localiser le son provenant d'une direction.

13. Dispositif portable selon l'une quelconque des revendications 1 à 12, comprenant en outre au moins un accéléromètre pour détecter le mouvement de l'utilisateur et une thermistance pour détecter un changement de température relatif.

14. Dispositif portable selon l'une quelconque des revendications 1 à 13, comprenant en outre un communicateur pour transmettre des données à un dispositif informatique, où le communicateur communique avec le dispositif informatique en utilisant au moins l'un d'un protocole de communication Bluetooth et d'un protocole de communication Wi-Fi.
